# EUROPEAN PATENT SPECIFICATION

(11) **EP 3 385 377 B1**
(45) Date of publication and mention of the grant of the patent: **27.10.2021**
(21) Application number: 17164880.1
(22) Date of filing: 05.04.2017
(51) Int. Cl.: C12N 9/24, C11D 3/386, A23K 20/189, C09K 8/88

(54) **BACTERIAL MANNANASES**
BAKTERIELLE MANNANASEN
MANNANASES BACTÉRIENNES

(43) Date of publication of application: 10.10.2018
(73) Proprietor: AB Enzymes Oy, 05200 Rajamäki (FI)
(72) Inventor: Leinonen, Taija, 80469 München (DE); Valtakari, Leena, 05200 Rajamäki (FI); Seefried, Michael, 63762 Großostheim (DE); Juntunen, Kari, 02680 Espoo (FI); Järvinen, Kristiina, 02940 Espoo (FI); Dollak, Daniela, 64589 Stockstadt a. Rhein (DE); Lorenz, Patrick, 64653 Lorsch (DE); Vehmaanperä, Jari, 00980 Helsinki (FI); Ojapalo, Pentti, 04360 Tuusula (FI); Puranen, Terhi, 05200 Rajamäki (FI); Herbst, Daniela, 40237 Düsseldorf (DE); Wieland, Susanne, 41541 Zons/Dormagen (DE); Mussmann, Nina, 47877 Willich (DE)
(74) Representative: Espatent Oy

(56) References cited:
- WO-A1-2014/088940
- Anonymous: "JCM9152_3753 - Mannanase - Bacillus hemicellulosilyticus JCM 9152 - JCM9152_3753 gene & protein", , 19 March 2014 (2014-03-19), XP055405714, Retrieved from the Internet: URL:http://www.uniprot.org/uniprot/W4QLJ5 [retrieved on 2017-09-12]
- SRIVASTAVA PRAVEEN KUMAR ET AL: "Production, properties, and applications ofendo-[beta]-mannanases", BIOTECHNOLOGY ADVANCES, ELSEVIER PUBLISHING, BARKING, GB, vol. 35, no. 1, 9 November 2016 (2016-11-09), pages 1-19, XP029880385, ISSN: 0734-9750, DOI: 10.1016/J.BIOTECHADV.2016.11.001
- MONTAROP YAMABHAI ET AL: "Mannan biotechnology: from biofuels to health", CRC CRITICAL REVIEWS IN BIOTECHNOLOGY, vol. 36, no. 1, 15 July 2014 (2014-07-15), pages 32-42, XP055405738, US ISSN: 0738-8551, DOI: 10.3109/07388551.2014.923372
- PRAKRAM SINGH CHAUHAN ET AL: "Mannanases: microbial sources, production, properties and potential biotechnological applications", APPLIED MICROBIOLOGY AND BIOTECHNOLOGY, SPRINGER, BERLIN, DE, vol. 93, no. 5, 8 February 2012 (2012-02-08), pages 1817-1830, XP035019260, ISSN: 1432-0614, DOI: 10.1007/S00253-012-3887-5

### SEQUENCE LISTING

<110> AB Enzymes
<120> Bacterial mannanases
<130> 31477EP-lf
<160> 48
<170> PatentIn version 3.5
<210> 1
   <211> 39
   <212> DNA
   <213> Artificial Sequence
<220>
   <223> primer
<400> 1
   caaccgcctc tgcagcttat gcacaaaacg gatttcacg 39
<210> 2
   <211> 39
   <212> DNA
   <213> Artificial Sequence
<220>
   <223> primer
<400> 2
   cggtatatct ctgtcttaat cactcttaag cccattttc 39
<210> 3
   <211> 37
   <212> DNA
   <213> Artificial Sequence
<220>
   <223> primer
<400> 3
   caaccgcctc tgcagcttct gatggtcata gccaaac 37
<210> 4
   <211> 36
   <212> DNA
   <213> Artificial Sequence
<220>
   <223> primer
<400> 4
   cggtatatct ctgtcttatt ggattgttac atgatc 36
<210> 5
   <211> 40
   <212> DNA
   <213> Artificial Sequence
<220>
   <223> primer
<400> 5
   caaccgcctc tgcagctgca agcgggtttt atgtaaacgg 40
<210> 6
   <211> 39
   <212> DNA
   <213> Artificial Sequence
<220>
   <223> primer
<400> 6
   cggtatatct ctgtcttatt taatggtaac gttatcaac 39
<210> 7
   <211> 17
   <212> DNA
   <213> Artificial Sequence
<220>
   <223> primer
<400> 7
   agctgcagag gcggttg 17
<210> 8
   <211> 21
   <212> DNA
   <213> Artificial Sequence
<220>
   <223> primer
<400> 8
   gacagagata taccgacagt g 21
<210> 9
   <211> 975
   <212> DNA
   <213> Bacillus clausii
<400> 9
   atgaagaggg aggacatgga tcaaatgaaa agaaagcggt tacaattgtt tggaacacta      60
   gtggtattgg ttttgttcgt gtacggtagc ggttcggcat atgcacaaaa cggatttcac     120
   gtatccggta cagagttgtt ggacaaaaat ggcgatcctt atgttatgcg tggcgtcaac     180
   catggacact cttggtttaa gcaagatctg gaggaagcaa tccctgccat agcagaaaca     240
   ggggcgaaca cggtgagaat ggtcttatcc aatggacagc aatgggaaaa agatgatgcc     300
   tctgagcttg cccgtgtgct ggctgccaca gaaacatatg gattgacaac tgtgctggaa     360
   gtccatgacg ctacaggaag tgacgatcct gctgatttag agaaagcagt cgattattgg     420
   atcgaaatgg ctgatgttct caaggggaca gaagaccgag taatcattaa cgttgccaat     480
   gaatggtatg ggtcgtggag gagcgacgtt tgggcagaag catacgcaca agcgatcccg     540
   cgcttgcgca gcgctggcct ctcccataca ttaatggttg atgcggcagg ttggggccag     600
   taccctgcct ccatccacga gcggggagcc gatgtgtttg cgtccgatcc attaaaaaac     660
   acgatgtttt cgatccatat gtacgaatat gcaggagctg atagggcgac aattgcctat     720
   aacattgatc gtgtgcttgc tgaaaatctt gctgtggtga tcggtgaatt tggccatagg     780
   catcatgatg gcgatgtcga tgaagatgcg attttggcct atacagcaga gcggcaagtg     840
   ggctggctgg cctggtcatg gtatggcaac agcgggggtg ttgaatactt ggatttagct     900
   gaaggcccat caggcccatt gacgagttgg ggcaaacgaa ttgtttatgg tgaaaatggg     960
   cttaagagtg attaa                                                      975
<210> 10
   <211> 870
   <212> DNA
   <213> Bacillus clausii
<400> 10
   cagaacggct tccacgtctc cggcacggag ctcctggaca agaacggcga cccttacgtc      60
   atgcgcggcg tcaaccacgg ccacagctgg ttcaagcagg acctcgagga agccatccct     120
   gctatcgctg agacgggcgc taacacggtc cgcatggtcc tgagcaacgg ccagcagtgg     180
   gagaaggacg acgctagcga gctggctcgc gtcctcgctg ctacggagac gtacggcctc     240
   accacggtcc tggaggtcca cgacgctacg ggctcggacg accccgccga cctcgagaag     300
   gccgtcgact actggatcga gatggctgac gtcctgaagg gcaccgagga ccgcgtcatc     360
   atcaacgtcg ccaacgagtg gtacggctcc tggcgcagcg acgtctgggc cgaggcctac     420
   gctcaggcta tccctcgcct ccgctcggcc ggcctctccc acacgctcat ggtcgacgct     480
   gccggctggg gccagtaccc tgcttccatc cacgagcgcg gcgctgacgt ctttgcttcg     540
   gaccccctga agaacaccat gttctccatc cacatgtacg agtacgctgg cgctgaccgc     600
   gctaccatcg cctacaacat cgaccgcgtc ctggctgaga acctggctgt cgtcatcggc     660
   gagtttggcc accgccacca cgacggcgac gtcgacgagg acgctatcct ggcttacacc     720
   gccgagcgcc aggtcggctg gctggcttgg tcgtggtacg gcaactcggg cggcgtcgag     780
   tacctggacc tggctgaggg cccttcgggc cctctcacga gctggggcaa gcgcatcgtc     840
   tacggcgaga acggcctgaa gagcgactaa                                      870
<210> 11
   <211> 324
   <212> **PRT**
   <213> Bacillus clausii
<400> 11
   Met Lys Arg Glu Asp Met Asp Gln Met Lys Arg Lys Arg Leu Gln Leu
   1               5                   10                  15
   Phe Gly Thr Leu Val Val Leu Val Leu Phe Val Tyr Gly Ser Gly Ser
               20                  25                  30
   Ala Tyr Ala Gln Asn Gly Phe His Val Ser Gly Thr Glu Leu Leu Asp
           35                  40                  45
   Lys Asn Gly Asp Pro Tyr Val Met Arg Gly Val Asn His Gly His Ser
       50                  55                  60
   Trp Phe Lys Gln Asp Leu Glu Glu Ala Ile Pro Ala Ile Ala Glu Thr
   65                  70                  75                  80
   Gly Ala Asn Thr Val Arg Met Val Leu Ser Asn Gly Gln Gln Trp Glu
                   85                  90                  95
   Lys Asp Asp Ala Ser Glu Leu Ala Arg Val Leu Ala Ala Thr Glu Thr
               100                 105                 110
   Tyr Gly Leu Thr Thr Val Leu Glu Val His Asp Ala Thr Gly Ser Asp
           115                 120                 125
   Asp Pro Ala Asp Leu Glu Lys Ala Val Asp Tyr Trp Ile Glu Met Ala
       130                 135                 140
   Asp Val Leu Lys Gly Thr Glu Asp Arg Val Ile Ile Asn Val Ala Asn
   145                 150                 155                 160
   Glu Trp Tyr Gly Ser Trp Arg Ser Asp Val Trp Ala Glu Ala Tyr Ala
                   165                 170                 175
   Gln Ala Ile Pro Arg Leu Arg Ser Ala Gly Leu Ser His Thr Leu Met
               180                 185                 190
   Val Asp Ala Ala Gly Trp Gly Gln Tyr Pro Ala Ser Ile His Glu Arg
           195                 200                 205
   Gly Ala Asp Val Phe Ala Ser Asp Pro Leu Lys Asn Thr Met Phe Ser
       210                 215                 220
   Ile His Met Tyr Glu Tyr Ala Gly Ala Asp Arg Ala Thr Ile Ala Tyr
   225                 230                 235                 240
   Asn Ile Asp Arg Val Leu Ala Glu Asn Leu Ala Val Val Ile Gly Glu
   245 250 255
   Phe Gly His Arg His His Asp Gly Asp Val Asp Glu Asp Ala Ile Leu
   260 265 270
   Ala Tyr Thr Ala Glu Arg Gln Val Gly Trp Leu Ala Trp Ser Trp Tyr
   275 280 285
   Gly Asn Ser Gly Gly Val Glu Tyr Leu Asp Leu Ala Glu Gly Pro Ser
   290 295 300
   Gly Pro Leu Thr Ser Trp Gly Lys Arg Ile Val Tyr Gly Glu Asn Gly
   305 310 315 320
   Leu Lys Ser Asp
<210> 12
   <211> 289
   <212> PRT
   <213> Bacillus clausii
<400> 12
   Gln Asn Gly Phe His Val Ser Gly Thr Glu Leu Leu Asp Lys Asn Gly
   1 5 10 15
   Asp Pro Tyr Val Met Arg Gly Val Asn His Gly His Ser Trp Phe Lys
   20 25 30
   Gln Asp Leu Glu Glu Ala Ile Pro Ala Ile Ala Glu Thr Gly Ala Asn
   35 40 45
   Thr Val Arg Met Val Leu Ser Asn Gly Gln Gln Trp Glu Lys Asp Asp
   50 55 60
   Ala Ser Glu Leu Ala Arg Val Leu Ala Ala Thr Glu Thr Tyr Gly Leu
   65 70 75 80
   Thr Thr Val Leu Glu Val His Asp Ala Thr Gly Ser Asp Asp Pro Ala
   85 90 95
   Asp Leu Glu Lys Ala Val Asp Tyr Trp Ile Glu Met Ala Asp Val Leu
   100 105 110
   Lys Gly Thr Glu Asp Arg Val Ile Ile Asn Val Ala Asn Glu Trp Tyr
   115 120 125
   Gly Ser Trp Arg Ser Asp Val Trp Ala Glu Ala Tyr Ala Gln Ala Ile
       130                 135                 140
   Pro Arg Leu Arg Ser Ala Gly Leu Ser His Thr Leu Met Val Asp Ala
   145                 150                 155                 160
   Ala Gly Trp Gly Gln Tyr Pro Ala Ser Ile His Glu Arg Gly Ala Asp
                   165                 170                 175
   Val Phe Ala Ser Asp Pro Leu Lys Asn Thr Met Phe Ser Ile His Met
               180                 185                 190
   Tyr Glu Tyr Ala Gly Ala Asp Arg Ala Thr Ile Ala Tyr Asn Ile Asp
           195                 200                 205
   Arg Val Leu Ala Glu Asn Leu Ala Val Val Ile Gly Glu Phe Gly His
       210                 215                 220
   Arg His His Asp Gly Asp Val Asp Glu Asp Ala Ile Leu Ala Tyr Thr
   225                 230                 235                 240
   Ala Glu Arg Gln Val Gly Trp Leu Ala Trp Ser Trp Tyr Gly Asn Ser
                   245                 250                 255
   Gly Gly Val Glu Tyr Leu Asp Leu Ala Glu Gly Pro Ser Gly Pro Leu
               260                 265                 270
   Thr Ser Trp Gly Lys Arg Ile Val Tyr Gly Glu Asn Gly Leu Lys Ser
           275                 280                 285
   Asp
<210> 13
   <211> 1473
   <212> **DNA**
   <213> Bacillus hemicellulosilyticus
<400> 13
   atgagaaatt tcggtaagtt aattgtcagt tcttgtcttc tattcagttt ttttcttttt 60
   gcctctgatg gtcatagcca aacacattct ggtttttata tcgaaggttc aaccctttat 120
   gacgccaacg gagagccctt tgtaatgaga ggtatcaatc atggacatgc ctggtataaa 180
   catgattcta acgtcgctat accagctatt gctaatcaag gagcaaatac aattcgtatt 240
   gttctgtcag atggtggtca atgggcaaaa gatgatataa acacattaaa tcaagtgctc 300
   gatttagcag aggaacatga gatgattgct gttgttgagg ttcacgatgc aacaggatct 360
   aattctatgg ctgacttaaa tcgtgctgtc gattattgga ttgaaatgaa agacgcttta      420
   attggaaaag aagatcgcgt cataattaac attgccaatg aatggtatgg agcatgggac      480
   ggacaaggct gggcaaatgg ctataaggag gttattccac gtttacgaaa tgctggcttc      540
   actcatacat taatggtaga tgcagctggt tggggacaat accctcaatc gattcatgat      600
   tatggtcaag aggtatttaa tgctgatcct ttagcaaata cgatgttttc catccatatg      660
   tatgaatatg ctggcggaaa tgcttcaatg gtacaatcta atatcgatgg tgtcgtcgat      720
   caagggttag ctcttgtaat aggagaattt gggcatatgc atacggacgg agatgttgat      780
   gaagcaacga tattgagcta ctcgcaacaa agaggagtcg gttggctagc ttggtcttgg      840
   aaaggcaatg ggactcaatg ggaatatcta gatttatctt atgattggca aggaacaaac      900
   ttaacttctt ggggaaatac cattgtccac gggcctaatg gattactcga aacatccatt      960
   ccaagctcga ttttccatac cgctccaaac aatggagatc cccctcctca taacggtaat     1020
   gaaacgatct tatatgattt cgaacatggc actcaaggct ggtcaggttc ttcacttctt     1080
   ggaggacctt ggacgacgaa tgaatggagt acaaatggta accattcatt aaaggccgat     1140
   attttcttat cagctaactc caaacatgaa ttagcaaaag ttgaaaatcg aaatttatca     1200
   ggctactcta ctttacaagc cactgtccgc catgcacatt ggggaaatgt tggtaattta     1260
   acggcgagaa tgtatgtaaa aacgggctca aactatagct ggtttaatgg tgatcctatc     1320
   ccagtaaact cagcaaatgg tacgactgtc actttgcctc tttcatctat tccaaaccta     1380
   aatgacgtaa aagaaattgg cgttgaattt attggagctt caaatagcaa tggacaaacc     1440
   gccatttatt tagatcatgt aacaatccaa taa                                  1473
<210> 14
   <211> 1395
   <212> **DNA**
   <213> Bacillus hemicellulosilyticus
<400> **14**
   cagacccact cgggcttcta catcgagggc tcgacgctct acgacgctaa cggcgagcct      60
   tttgtcatgc gcggcatcaa ccacggccac gcctggtaca agcacgactc caacgtcgct     120
   atccctgcta tcgctaacca gggcgctaac accatccgca tcgtcctcag cgacggtggc     180
   cagtgggcca aggacgacat caacacgctg aaccaggtcc tcgacctggc cgaggagcac     240
   gagatgatcg ctgtcgtcga ggtccacgac gctaccggct ccaacagcat ggccgacctc     300
   aaccgcgccg tcgactactg gatcgagatg aaggacgccc tgatcggcaa ggaagaccgc     360
   gtcatcatca acatcgctaa cgagtggtac ggcgcttggg acggccaggg ctgggccaac     420
   ggctacaagg aagtcatccc tcgcctgcgc aacgctggct tcacccacac cctcatggtc     480
   gacgctgccg gctggggcca gtaccctcag agcatccacg actacggcca agaggtcttc     540
   aacgccgacc ctctggccaa caccatgttc tccatccaca tgtacgagta cgctggcggc     600
   aacgcctcca tggtccagag caacatcgac ggcgtcgtcg accagggcct cgctctggtc     660
   atcggcgagt tcggccacat gcacacggac ggcgacgtcg acgaggctac catcctgagc     720
   tactcgcagc agcgcggcgt cggctggctg gcctggtcgt ggaagggcaa cggcacccag     780
   tgggagtacc tcgacctgag ctacgactgg cagggcacca acctcacgtc gtggggcaac     840
   acgatcgtcc acggccctaa cggcctcctg gagacgtcca tcccttccag catctttcac     900
   accgctccta acaacggcga ccctcctccc cacaacggca acgagacgat cctgtacgac     960
   ttcgagcacg gcacgcaggg ctggtcgggc tcgtccctgc tgggcggccc ttggaccacc    1020
   aacgagtggt cgaccaacgg caaccactcc ctcaaggccg acatcttcct gtccgccaac    1080
   agcaagcacg agctcgccaa ggtcgagaac cgcaacctca gcggctactc gacgctgcag    1140
   gctaccgtcc gccacgctca ctggggcaac gtcggcaacc tgacggctcg catgtacgtc    1200
   aagacgggca gcaactactc gtggttcaac ggcgacccca tccctgtcaa ctcggctaac    1260
   ggcaccaccg tcaccctccc tctgagctcg atccccaacc tcaacgacgt caaggagatc    1320
   ggcgtcgagt tcatcggcgc tagcaacagc aacggccaga ccgccatcta cctggaccac    1380
   gtcacgatcc agtaa                                                     1395
<210> 15
   <211> 490
   <212> **PRT**
   <213> Bacillus hemicellulosilyticus
<400> 15
   Met Arg Asn Phe Gly Lys Leu Ile Val Ser Ser Cys Leu Leu Phe Ser
   1               5                   10                  15
   Phe Phe Leu Phe Ala Ser Asp Gly His Ser Gln Thr His Ser Gly Phe
               20                  25                  30
   Tyr Ile Glu Gly Ser Thr Leu Tyr Asp Ala Asn Gly Glu Pro Phe Val
           35                  40                  45
   Met Arg Gly Ile Asn His Gly His Ala Trp Tyr Lys His Asp Ser Asn
       50                  55                  60
   Val Ala Ile Pro Ala Ile Ala Asn Gln Gly Ala Asn Thr Ile Arg Ile
   65                  70                  75                  80
   Val Leu Ser Asp Gly Gly Gln Trp Ala Lys Asp Asp Ile Asn Thr Leu
                   85                  90                  95
   Asn Gln Val Leu Asp Leu Ala Glu Glu His Glu Met Ile Ala Val Val
   100 105 110
   Glu Val His Asp Ala Thr Gly Ser Asn Ser Met Ala Asp Leu Asn Arg
   115 120 125
   Ala Val Asp Tyr Trp Ile Glu Met Lys Asp Ala Leu Ile Gly Lys Glu
   130 135 140
   Asp Arg Val Ile Ile Asn Ile Ala Asn Glu Trp Tyr Gly Ala Trp Asp
   145 150 155 160
   Gly Gln Gly Trp Ala Asn Gly Tyr Lys Glu Val Ile Pro Arg Leu Arg
   165 170 175
   Asn Ala Gly Phe Thr His Thr Leu Met Val Asp Ala Ala Gly Trp Gly
   180 185 190
   Gln Tyr Pro Gln Ser Ile His Asp Tyr Gly Gln Glu Val Phe Asn Ala
   195 200 205
   Asp Pro Leu Ala Asn Thr Met Phe Ser Ile His Met Tyr Glu Tyr Ala
   210 215 220
   Gly Gly Asn Ala Ser Met Val Gln Ser Asn Ile Asp Gly Val Val Asp
   225 230 235 240
   Gln Gly Leu Ala Leu Val Ile Gly Glu Phe Gly His Met His Thr Asp
   245 250 255
   Gly Asp Val Asp Glu Ala Thr Ile Leu Ser Tyr Ser Gln Gln Arg Gly
   260 265 270
   Val Gly Trp Leu Ala Trp Ser Trp Lys Gly Asn Gly Thr Gln Trp Glu
   275 280 285
   Tyr Leu Asp Leu Ser Tyr Asp Trp Gln Gly Thr Asn Leu Thr Ser Trp
   290 295 300
   Gly Asn Thr Ile Val His Gly Pro Asn Gly Leu Leu Glu Thr Ser Ile
   305 310 315 320
   Pro Ser Ser Ile Phe His Thr Ala Pro Asn Asn Gly Asp Pro Pro Pro
   325 330 335
   His Asn Gly Asn Glu Thr Ile Leu Tyr Asp Phe Glu His Gly Thr Gln
   340 345 350
   Gly Trp Ser Gly Ser Ser Leu Leu Gly Gly Pro Trp Thr Thr Asn Glu
   355 360 365
   Trp Ser Thr Asn Gly Asn His Ser Leu Lys Ala Asp Ile Phe Leu Ser
   370 375 380
   Ala Asn Ser Lys His Glu Leu Ala Lys Val Glu Asn Arg Asn Leu Ser
   385 390 395 400
   Gly Tyr Ser Thr Leu Gln Ala Thr Val Arg His Ala His Trp Gly Asn
   405 410 415
   Val Gly Asn Leu Thr Ala Arg Met Tyr Val Lys Thr Gly Ser Asn Tyr
   420 425 430
   Ser Trp Phe Asn Gly Asp Pro Ile Pro Val Asn Ser Ala Asn Gly Thr
   435 440 445
   Thr Val Thr Leu Pro Leu Ser Ser Ile Pro Asn Leu Asn Asp Val Lys
   450 455 460
   Glu Ile Gly Val Glu Phe Ile Gly Ala Ser Asn Ser Asn Gly Gln Thr
   465 470 475 480
   Ala Ile Tyr Leu Asp His Val Thr Ile Gln
   485 490
<210> 16
   <211> **464**
   <212> **PRT**
   <213> Bacillus hemicellulosilyticus
<400> 16
   Gln Thr His Ser Gly Phe Tyr Ile Glu Gly Ser Thr Leu Tyr Asp Ala
   1 5 10 15
   Asn Gly Glu Pro Phe Val Met Arg Gly Ile Asn His Gly His Ala Trp
   20 25 30
   Tyr Lys His Asp Ser Asn Val Ala Ile Pro Ala Ile Ala Asn Gln Gly
   35 40 45
   Ala Asn Thr Ile Arg Ile Val Leu Ser Asp Gly Gly Gln Trp Ala Lys
   50 55 60
   Asp Asp Ile Asn Thr Leu Asn Gln Val Leu Asp Leu Ala Glu Glu His
   65 70 75 80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ala Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ser Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Thr Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Leu Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 17
   <211> **1449**
   <212> **DNA**
   <213> Virgibacillus soli
<400> **17**
   atgttattct ctacttcact gtttacttct acttcaaaag cgaatgcagc aagcgggttt      60
   tatgtaaacg gaaacacact ctatgacgca acaggtaccc cttttgtgat aagaggaatc     120
   aatcatgctc actcttggtt taaagacgac acagcaaccg caatacctgc cattgcagca     180
   actggggcga atactattag aatcgtatta tcggatggca gccaatatag tcgggatgat     240
   attgatggcg tgaggaatct aatatcattg gctgaggaaa ataatctaat tgctatgtta     300
   gaggtccacg atgctactgg aaaagatgat atcagctcat tagatagtgc ggcagattat     360
   tggattagta taaaagaagc acttatcggc aaggaagaca aagtcctaat aaacatcgca     420
   aatgaatggt acggtacttg ggatggggct agttgggcgg atggctacaa acaagtgatt     480
   cccaaattaa gaaatgcagg acttaaccac acactaatag tagactctgc tggctggggg     540
   caatttccgg agtccattca caattacgga aaagaagtat tcaatgctga ccccctacaa     600
   aatacaatgt tctctattca tatgtatgaa tatgctggtg gggacgcttc tactgtcaaa     660
   gcaaatattg acggtgtatt aaatcaaggt ctagccgtaa tcattggaga atttggacat     720
   aggcatacag acggagatgt agatgaagca acaattatga attattccca agagaaaaat     780
   gttggctggc tcgcatggtc gtggaaaggt aatggcatgg aatgggatta tttagactta     840
   tcctatgatt gggccggaaa taacctaacc gactggggaa ataccattgt aaatagtaca     900
   aacggcttaa aagctacatc tgaaataagt ccagtatttg gagatggaga tgacggtgta     960
   ggcgacggtg gtcctgggga ttctaacgga actgaaacta cgctttataa cttcgaaacc    1020
   gggacagaag gatggagcgg cgaaaatata gaaactggac cttggtcagt gaatgagtgg    1080
   gcagcaaaag gtaaccactc tttaaaagct gatgttaatt tgggtgataa ctctgaacat    1140
   tatctatacc taactcaaaa cctaaatttt agcggaaagt cacaactcac agcgactgta    1200
   aagcatgctg attggggaaa cttcggggat gaaataaatg caaagttata tgtaaaaaca    1260
   gaatcagatt ggcaatggtt tgatggagga attgaaaaga tcaattcttc aattggaact    1320
   attataacct tagatttatc atcgctctca aacccaagtg atattaaaga agttggtgtt    1380
   cagtttacgg gttcttcaaa tagttatggc ctaacagctt tatatgttga taacgttacc    1440
   attaaataa                                                            1449
<210> 18
   <211> 1401
   <212> DNA
   <213> Virgibacillus soli
<400> 18
   gcctcgggct tctacgtcaa cggcaacact ctctacgacg ccacgggcac cccatttgtc      60
   atccgcggca tcaaccacgc tcactcgtgg ttcaaggacg acactgccac cgctatccct     120
   gctatcgctg ctacgggcgc caacacgatc cgcatcgtcc tcagcgacgg ctcgcagtac     180
   tcccgcgacg acatcgacgg cgtccgcaac ctcatctccc tggccgagga gaacaacctc     240
   atcgccatgc tggaggtcca cgacgctacc ggcaaggacg acatcagctc gctggacagc     300
   gccgccgact actggatctc gatcaaggaa gccctcatcg gcaaggaaga caaggtcctg     360
   atcaacatcg ccaacgagtg gtacggcacc tgggacggcg ctagctgggc tgacggctac     420
   aagcaggtca tccctaagct ccgcaacgcc ggcctcaacc acacgctcat cgtcgactcg     480
   gctggctggg gccagttccc ggagagcatc cacaactacg gcaaggaagt cttcaacgcc     540
   gaccccctgc agaacacgat gttctcgatc cacatgtacg agtacgccgg cggcgacgct     600
   tccacggtca aggccaacat cgacggcgtc ctcaaccagg gcctggctgt catcatcggc     660
   gagtttggcc accgccacac cgacggcgac gtcgacgagg ccaccatcat gaactacagc     720
   caggagaaga acgtcggctg gctggcttgg agctggaagg gcaacggcat ggagtgggac     780
   tacctcgacc tgagctacga ctgggccggc aacaacctca ccgactgggg caacacgatc     840
   gtcaactcga ccaacggcct gaaggccacc tcggagatca gccctgtctt tggcgacggc     900
   gacgacggcg tcggcgacgg tggccccggc gacagcaacg gcaccgagac gacgctgtac     960
   aactttgaga cgggcaccga gggctggagc ggcgagaaca tcgagacggg cccttggtcg    1020
   gtcaacgagt gggctgccaa gggcaaccac tccctcaagg ccgacgtcaa cctgggcgac    1080
   aacagcgagc actacctcta cctgacgcag aacctcaact tctccggcaa gtcgcagctg    1140
   acggctaccg tcaagcacgc tgactggggc aacttcggcg acgagatcaa cgccaagctc    1200
   tacgtcaaga ccgagagcga ctggcagtgg ttcgacggtg gcatcgagaa gatcaactcc    1260
   agcatcggca ccatcatcac gctcgacctg tcgtccctgt cgaacccgtc cgacatcaag    1320
   gaagtcggcg tccagttcac tggctcgtct aactcttacg gcctcactgc tctttacgtc    1380
   gacaacgtca ctatcaagta g                                             1401
<210> 19
   <211> 482
   <212> PRT
   <213> Virgibacillus soli
<400> 19
   Met Leu Phe Ser Thr Ser Leu Phe Thr Ser Thr Ser Lys Ala Asn Ala
   1               5                   10                  15
   Ala Ser Gly Phe Tyr Val Asn Gly Asn Thr Leu Tyr Asp Ala Thr Gly
               20                  25                  30
   Thr Pro Phe Val Ile Arg Gly Ile Asn His Ala His Ser Trp Phe Lys
           35                  40                  45
   Asp Asp Thr Ala Thr Ala Ile Pro Ala Ile Ala Ala Thr Gly Ala Asn
       50                  55                  60
   Thr Ile Arg Ile Val Leu Ser Asp Gly Ser Gln Tyr Ser Arg Asp Asp
   65                  70                  75                  80
   Ile Asp Gly Val Arg Asn Leu Ile Ser Leu Ala Glu Glu Asn Asn Leu
                   85                  90                  95
   Ile Ala Met Leu Glu Val His Asp Ala Thr Gly Lys Asp Asp Ile Ser
               100                 105                 110
   Ser Leu Asp Ser Ala Ala Asp Tyr Trp Ile Ser Ile Lys Glu Ala Leu
           115                 120                 125
   Ile Gly Lys Glu Asp Lys Val Leu Ile Asn Ile Ala Asn Glu Trp Tyr
       130                 135                 140
   Gly Thr Trp Asp Gly Ala Ser Trp Ala Asp Gly Tyr Lys Gln Val Ile
   145                 150                 155                 160
   Pro Lys Leu Arg Asn Ala Gly Leu Asn His Thr Leu Ile Val Asp Ser
                   165                 170                 175
   Ala Gly Trp Gly Gln Phe Pro Glu Ser Ile His Asn Tyr Gly Lys Glu
               180                 185                 190
   Val Phe Asn Ala Asp Pro Leu Gln Asn Thr Met Phe Ser Ile His Met
           195                 200                 205
   Tyr Glu Tyr Ala Gly Gly Asp Ala Ser Thr Val Lys Ala Asn Ile Asp
       210                 215                 220
   Gly Val Leu Asn Gln Gly Leu Ala Val Ile Ile Gly Glu Phe Gly His
   225                 230                 235                 240
   Arg His Thr Asp Gly Asp Val Asp Glu Ala Thr Ile Met Asn Tyr Ser
                   245                 250                 255
   Gln Glu Lys Asn Val Gly Trp Leu Ala Trp Ser Trp Lys Gly Asn Gly
               260                 265                 270
   Met Glu Trp Asp Tyr Leu Asp Leu Ser Tyr Asp Trp Ala Gly Asn Asn
           275                 280                 285
   Leu Thr Asp Trp Gly Asn Thr Ile Val Asn Ser Thr Asn Gly Leu Lys
       290                 295                 300
   Ala Thr Ser Glu Ile Ser Pro Val Phe Gly Asp Gly Asp Asp Gly Val
   305                 310                 315                 320
   Gly Asp Gly Gly Pro Gly Asp Ser Asn Gly Thr Glu Thr Thr Leu Tyr
                   325                 330                 335
   Asn Phe Glu Thr Gly Thr Glu Gly Trp Ser Gly Glu Asn Ile Glu Thr
               340                 345                 350
   Gly Pro Trp Ser Val Asn Glu Trp Ala Ala Lys Gly Asn His Ser Leu
           355                 360                 365
   Lys Ala Asp Val Asn Leu Gly Asp Asn Ser Glu His Tyr Leu Tyr Leu
       370                 375                 380
   Thr Gln Asn Leu Asn Phe Ser Gly Lys Ser Gln Leu Thr Ala Thr Val
   385                 390                 395                 400
   Lys His Ala Asp Trp Gly Asn Phe Gly Asp Glu Ile Asn Ala Lys Leu
                   405                 410                 415
   Tyr Val Lys Thr Glu Ser Asp Trp Gln Trp Phe Asp Gly Gly Ile Glu
               420                 425                 430
   Lys Ile Asn Ser Ser Ile Gly Thr Ile Ile Thr Leu Asp Leu Ser Ser
           435                 440                 445
   Leu Ser Asn Pro Ser Asp Ile Lys Glu Val Gly Val Gln Phe Thr Gly
       450                 455                 460
   Ser Ser Asn Ser Tyr Gly Leu Thr Ala Leu Tyr Val Asp Asn Val Thr
   465                 470                 475                 480
   Ile Lys
<210> 20
   <211> 466
   <212> PRT
   <213> Virgibacillus soli
<400> 20
   Ala Ser Gly Phe Tyr Val Asn Gly Asn Thr Leu Tyr Asp Ala Thr Gly
   1               5                   10                  15
   Thr Pro Phe Val Ile Arg Gly Ile Asn His Ala His Ser Trp Phe Lys
               20                  25                  30
   Asp Asp Thr Ala Thr Ala Ile Pro Ala Ile Ala Ala Thr Gly Ala Asn
           35                  40                  45
   Thr Ile Arg Ile Val Leu Ser Asp Gly Ser Gln Tyr Ser Arg Asp Asp
       50                  55                  60
   Ile Asp Gly Val Arg Asn Leu Ile Ser Leu Ala Glu Glu Asn Asn Leu
   65                  70                  75                  80
   Ile Ala Met Leu Glu Val His Asp Ala Thr Gly Lys Asp Asp Ile Ser
                   85                  90                  95
   Ser Leu Asp Ser Ala Ala Asp Tyr Trp Ile Ser Ile Lys Glu Ala Leu
               100                 105                 110
   Ile Gly Lys Glu Asp Lys Val Leu Ile Asn Ile Ala Asn Glu Trp Tyr
           115                 120                 125
   Gly Thr Trp Asp Gly Ala Ser Trp Ala Asp Gly Tyr Lys Gln Val Ile
       130                 135                 140
   Pro Lys Leu Arg Asn Ala Gly Leu Asn His Thr Leu Ile Val Asp Ser
   145                 150                 155                 160
   Ala Gly Trp Gly Gln Phe Pro Glu Ser Ile His Asn Tyr Gly Lys Glu
                   165                 170                 175
   Val Phe Asn Ala Asp Pro Leu Gln Asn Thr Met Phe Ser Ile His Met
               180                 185                 190
   Tyr Glu Tyr Ala Gly Gly Asp Ala Ser Thr Val Lys Ala Asn Ile Asp
           195                 200                 205
   Gly Val Leu Asn Gln Gly Leu Ala Val Ile Ile Gly Glu Phe Gly His
       210                 215                 220
   Arg His Thr Asp Gly Asp Val Asp Glu Ala Thr Ile Met Asn Tyr Ser
   225                 230                 235                 240
   Gln Glu Lys Asn Val Gly Trp Leu Ala Trp Ser Trp Lys Gly Asn Gly
                   245                 250                 255
   Met Glu Trp Asp Tyr Leu Asp Leu Ser Tyr Asp Trp Ala Gly Asn Asn
               260                 265                 270
   Leu Thr Asp Trp Gly Asn Thr Ile Val Asn Ser Thr Asn Gly Leu Lys
           275                 280                 285
   Ala Thr Ser Glu Ile Ser Pro Val Phe Gly Asp Gly Asp Asp Gly Val
       290                 295                 300
   Gly Asp Gly Gly Pro Gly Asp Ser Asn Gly Thr Glu Thr Thr Leu Tyr
   305                 310                 315                 320
   Asn Phe Glu Thr Gly Thr Glu Gly Trp Ser Gly Glu Asn Ile Glu Thr
                   325                 330                 335
   Gly Pro Trp Ser Val Asn Glu Trp Ala Ala Lys Gly Asn His Ser Leu
               340                 345                 350
   Lys Ala Asp Val Asn Leu Gly Asp Asn Ser Glu His Tyr Leu Tyr Leu
           355                 360                 365
   Thr Gln Asn Leu Asn Phe Ser Gly Lys Ser Gln Leu Thr Ala Thr Val
       370                 375                 380
   Lys His Ala Asp Trp Gly Asn Phe Gly Asp Glu Ile Asn Ala Lys Leu
   385                 390                 395                 400
   Tyr Val Lys Thr Glu Ser Asp Trp Gln Trp Phe Asp Gly Gly Ile Glu
                   405                 410                 415
   Lys Ile Asn Ser Ser Ile Gly Thr Ile Ile Thr Leu Asp Leu Ser Ser
               420                 425                 430
   Leu Ser Asn Pro Ser Asp Ile Lys Glu Val Gly Val Gln Phe Thr Gly
           435                 440                 445
   Ser Ser Asn Ser Tyr Gly Leu Thr Ala Leu Tyr Val Asp Asn Val Thr
       450                 455                 460
   Ile Lys
   465
<210> 21
   <211> 60
   <212> DNA
   <213> Artificial Sequence
<220>
   <223> primer
<400> 21
   agtcaatcgc gacaagcgcc agacccactc gggcttctac atcgagggct cgacgctcta 60
<210> 22
   <211> 46
   <212> DNA
   <213> Artificial Sequence
<220>
   <223> primer
<400> 22
   cgcgccggat ccttactgga tcgtgacgtg gtccaggtag atggcg 46
<210> 23
   <211> 60
   <212> DNA
   <213> Artificial Sequence
<220>
   <223> primer
<400> 23
   agtcaatcgc gacaagcgcc agaacggctt ccacgtctcc ggcacggagc tcctggacaa 60
<210> 24
   <211> 51
   <212> DNA
   <213> Artificial Sequence
<220>
   <223> primer
<400> 24
   cgcgccggat ccttagtcgc tcttcaggcc gttctcgccg tagacgatgc g 51
<210> 25
   <211> 1846
   <212> DNA
   <213> Bacillus pumilus
<400> 25
   atgaaaaaat gggttcaacg ggtggcttgt tttatgctgc tgatcacttt atgggcgggt 60
   tggttcactc tgaccgtaaa ggcctcctcc tatgtgcaaa catctggtac acattttgta 120
   ttgaacaacc acccatttta ctttgctggc acaaataatt attatttcca ttacaaatca 180
   aaaaagatgg tagatgctgt ttttgacgat atgaaggcaa tggatttaaa ggttattcgt 240
   atttggggat ttcacgatgg tacccctcaa gaaaactcag tcttacaatc tcgtccaggt 300
   gtttatgaag aatccggttt tcaaaaacta gactatgcga tttataaagc agggcaggaa 360
   ggaatcaagc tggtcatacc gctcgtgaac aattgggatg actttggcgg gatgaatcaa 420
   tatgtgaagt ggtttcaggc aggatcacat gatcactttt atacagattc tcggattaaa 480
   acagcttaca aaaactatgt gcgctatgta ttagagagaa ccaatacgta ctcaggtgtt 540
   caatataaag atgaccctgc tattatgaca tgggagctcg ccaatgagcc gcgcgctcag 600
   tcagaccctt cgggagatat actagtaaac tgggcagatg aaatgagtgc atggatcaaa 660
   tcaattgact cgaatcatct tgttgctgta ggagacgaag ggttctttcg catgacaggt 720
   catgatgatt ggttttacag tggaggagaa ggtgttgatt gggatcgttt gactgctctc 780
   cctcatattg attatggaac ctatcattta tacccggatc actggaatca gtctgctgca 840
   tggggagtga aatggatcaa agatcatatc acccgaggaa acgcaatcgg aaaacctgtt 900
   gtattagaag agtttggcta tcaaaatcaa gcagcccgtc ctgatgtata tgatagctgg 960
   ctgaagacaa ttgaacagct cggaggcgca ggtagccaat tttggatttt aacaagcatt 1020
   caagacgatg attccctcta cccggattat gatggttttc gagttttaaa ggagagccgg 1080
   gaggcaggaa ttattcgtga acacgccaaa agaatgaatg aaaagaactg atgaagaatg 1140
   cctgtttata aggaacttca tttgcataaa aaaattggat atggtatagt ttttatggaa 1200
   atgctaacga ttaccgagac aagagtgggg aaacccgctc ttttgtattg aacaggcaat 1260
   ttttgtctcg acattattca tccgttttct gctccccctg ctcacaataa agcagggttt 1320
   ttatgcagaa tgattgataa gagcgtttat cgaaagcaca aggaggaaga gaatgagcaa 1380
   aaaagtagtg gatatcgtaa gcgacatggt gcagccaatt ttagatggct tacagcttga 1440
   actcgttgat gttgaatttg tcaaagaggg tcaaaactgg ttccttcgcg tatttattga 1500
   ctctgataaa ggcgtcgata tcgaggagtg tgccaaagtg agcgaagcct tgagcgaaaa 1560
   gcttgatgag gcagatccaa ttagccaaaa ctactttctt gaagtgtcct ctcctggagc 1620
   ggagcgccca ttaaagaaaa aagctgattt tgaaaaagca cttggaaaaa atgttttcat 1680
   gaaaacatac gaaccaattg atggtgaaaa ggcatttgaa ggtgagctta caagctttga 1740
   tggtgagatt gcaacagtga cagtgaagat caagacaaga aagaaagaga tcaatattcc 1800
   atacgaaaaa attgctaacg caagattagc agtttcgttc aattaa 1846
<210> 26
   <211> 376
   <212> PRT
   <213> Bacillus pumilus
<400> 26
   Met Lys Lys Trp Val Gln Arg Val Ala Cys Phe Met Leu Leu Ile Thr
   1               5                   10                  15
   Leu Trp Ala Gly Trp Phe Thr Leu Thr Val Lys Ala Ser Ser Tyr Val
               20                  25                  30
   Gln Thr Ser Gly Thr His Phe Val Leu Asn Asn His Pro Phe Tyr Phe
           35                  40                  45
   Ala Gly Thr Asn Asn Tyr Tyr Phe His Tyr Lys Ser Lys Lys Met Val
       50                  55                  60
   Asp Ala Val Phe Asp Asp Met Lys Ala Met Asp Leu Lys Val Ile Arg
   65                  70                  75                  80
   Ile Trp Gly Phe His Asp Gly Thr Pro Gln Glu Asn Ser Val Leu Gln
                   85                  90                  95
   Ser Arg Pro Gly Val Tyr Glu Glu Ser Gly Phe Gln Lys Leu Asp Tyr
               100                 105                 110
   Ala Ile Tyr Lys Ala Gly Gln Glu Gly Ile Lys Leu Val Ile Pro Leu
           115                 120                 125
   Val Asn Asn Trp Asp Asp Phe Gly Gly Met Asn Gln Tyr Val Lys Trp
       130                 135                 140
   Phe Gln Ala Gly Ser His Asp His Phe Tyr Thr Asp Ser Arg Ile Lys
   145                 150                 155                 160
   Thr Ala Tyr Lys Asn Tyr Val Arg Tyr Val Leu Glu Arg Thr Asn Thr
                   165                 170                 175
   Tyr Ser Gly Val Gln Tyr Lys Asp Asp Pro Ala Ile Met Thr Trp Glu
               180                 185                 190
   Leu Ala Asn Glu Pro Arg Ala Gln Ser Asp Pro Ser Gly Asp Ile Leu
           195                 200                 205
   Val Asn Trp Ala Asp Glu Met Ser Ala Trp Ile Lys Ser Ile Asp Ser
       210                 215                 220
   Asn His Leu Val Ala Val Gly Asp Glu Gly Phe Phe Arg Met Thr Gly
   225                 230                 235                 240
   His Asp Asp Trp Phe Tyr Ser Gly Gly Glu Gly Val Asp Trp Asp Arg
                   245                 250                 255
   Leu Thr Ala Leu Pro His Ile Asp Tyr Gly Thr Tyr His Leu Tyr Pro
               260                 265                 270
   Asp His Trp Asn Gln Ser Ala Ala Trp Gly Val Lys Trp Ile Lys Asp
           275                 280                 285
   His Ile Thr Arg Gly Asn Ala Ile Gly Lys Pro Val Val Leu Glu Glu
       290                 295                 300
   Phe Gly Tyr Gln Asn Gln Ala Ala Arg Pro Asp Val Tyr Asp Ser Trp
   305                 310                 315                 320
   Leu Lys Thr Ile Glu Gln Leu Gly Gly Ala Gly Ser Gln Phe Trp Ile
                   325                 330                 335
   Leu Thr Ser Ile Gln Asp Asp Asp Ser Leu Tyr Pro Asp Tyr Asp Gly
               340                 345                 350
   Phe Arg Val Leu Lys Glu Ser Arg Glu Ala Gly Ile Ile Arg Glu His
           355                 360                 365
   Ala Lys Arg Met Asn Glu Lys Asn
       370                 375
<210> 27
   <211> 1083
   <212> DNA
   <213> Bacillus amyloliquefaciens
<400> 27
   atgctcaaaa agttcgcagt ctgtctgtct atcattttat tactcatctc agccgcccgt        60
   ccgatatcgg ctcacaccgt ttaccctgtc aatcccaatg cccagcagac gacaaaagac       120
   gtcatgaact ggctggcgca tttgcccaac cgttcagaaa acagggtcat gtccggtgca       180
   ttcggcgggt acagcgatgt caccttttca atgacggagg aaaaccgctt gaaaaacgcg        240
   acgggacagt ctcccgccat ctacggctgt gattatggga gagggtggct ggaaacatcg       300
   gatatcaccg attctatcga ctacagctgc aacagcagcc tcatttcgta ctggaaaagc       360
   ggcggcctcc ctcaggtcag cctgcatctc gcaaatccgg cctttccatc aggacactat       420
   aaaacggcca tttcaaacag ccagtataaa aatatcctga acccttcaac tgttgaagga       480
   cggcggcttg aggccttgct cagcaaaatc gccgacggcc ttactcagct gaaaaatcaa       540
   ggcgtcaccg ttctgttcag gccgctgcat gagatgaacg gtgaatggtt ctggtggggg       600
   ctgacaggct acaaccaaaa agacactgag agaatctcgc tgtacaaaga gctttacaag       660
   aagatatacc gctatatgac agagacaaga ggattggata atcttttgtg ggtgtattcg       720
   cctgatgcca acagagactt caaaacagac ttctacccag gctcatctta tgtggatatt       780
   accggactgg atgcttactt caccgacccg tatgcgatat caggctatga tgaaatgctg       840
   tctctgaaaa aaccgtttgc ctttgccgag accggtccgt ccggtaatat cggaagcttt       900
   gattacgctg tttttatcaa tgcgatcagg caaaagtatc ccgagacaac ctactttttg       960
   acatgggatg aacaattaag cccggcagcc aatcaaggcg cgcaaagcct ttatcaaaac      1020
   agctggacgc tgaacaaggg cgaaatgtgg aatggcggaa ccttgacgcc gatcgcggaa      1080
   taa                                                                    1083
<210> 28
   <211> 360
   <212> PRT
   <213> Bacillus amyloliquefaciens
<400> 28
   Met Leu Lys Lys Phe Ala Val Cys Leu Ser Ile Ile Leu Leu Leu Ile
   1               5                   10                  15
   Ser Ala Ala Arg Pro Ile Ser Ala His Thr Val Tyr Pro Val Asn Pro
               20                  25                  30
   Asn Ala Gln Gln Thr Thr Lys Asp Val Met Asn Trp Leu Ala His Leu
           35                  40                  45
   Pro Asn Arg Ser Glu Asn Arg Val Met Ser Gly Ala Phe Gly Gly Tyr
       50                  55                  60
   Ser Asp Val Thr Phe Ser Met Thr Glu Glu Asn Arg Leu Lys Asn Ala
   65                  70                  75                  80
   Thr Gly Gln Ser Pro Ala Ile Tyr Gly Cys Asp Tyr Gly Arg Gly Trp
                   85                  90                  95
   Leu Glu Thr Ser Asp Ile Thr Asp Ser Ile Asp Tyr Ser Cys Asn Ser
               100                 105                 110
   Ser Leu Ile Ser Tyr Trp Lys Ser Gly Gly Leu Pro Gln Val Ser Leu
           115                 120                 125
   His Leu Ala Asn Pro Ala Phe Pro Ser Gly His Tyr Lys Thr Ala Ile
       130                 135                 140
   Ser Asn Ser Gln Tyr Lys Asn Ile Leu Asn Pro Ser Thr Val Glu Gly
   145                 150                 155                 160
   Arg Arg Leu Glu Ala Leu Leu Ser Lys Ile Ala Asp Gly Leu Thr Gln
                   165                 170                 175
   Leu Lys Asn Gln Gly Val Thr Val Leu Phe Arg Pro Leu His Glu Met
               180                 185                 190
   Asn Gly Glu Trp Phe Trp Trp Gly Leu Thr Gly Tyr Asn Gln Lys Asp
           195                 200                 205
   Thr Glu Arg Ile Ser Leu Tyr Lys Glu Leu Tyr Lys Lys Ile Tyr Arg
       210                 215                 220
   Tyr Met Thr Glu Thr Arg Gly Leu Asp Asn Leu Leu Trp Val Tyr Ser
   225                 230                 235                 240
   Pro Asp Ala Asn Arg Asp Phe Lys Thr Asp Phe Tyr Pro Gly Ser Ser
                   245                 250                 255
   Tyr Val Asp Ile Thr Gly Leu Asp Ala Tyr Phe Thr Asp Pro Tyr Ala
               260                 265                 270
   Ile Ser Gly Tyr Asp Glu Met Leu Ser Leu Lys Lys Pro Phe Ala Phe
           275                 280                 285
   Ala Glu Thr Gly Pro Ser Gly Asn Ile Gly Ser Phe Asp Tyr Ala Val
       290                 295                 300
   Phe Ile Asn Ala Ile Arg Gln Lys Tyr Pro Glu Thr Thr Tyr Phe Leu
   305                 310                 315                 320
   Thr Trp Asp Glu Gln Leu Ser Pro Ala Ala Asn Gln Gly Ala Gln Ser
   325 330 335
   Leu Tyr Gln Asn Ser Trp Thr Leu Asn Lys Gly Glu Met Trp Asn Gly
   340 345 350
   Gly Thr Leu Thr Pro Ile Ala Glu
   355 360
<210> 29
   <211> 1494
   <212> DNA
   <213> Amphibacillus xylanus
<400> 29
   gtgaagttaa ctaaactaaa actattgagt agtgtatttt ttgttgtatt aactgtgtta 60
   atgttgtttg tccctgggaa tattgtgaat gtaaaagctg ctaacggctt ttatgtaagc 120
   gattccaatc tgtatgatgc aaatggaaat caatttgtta tgcgtggggt taatcatgcc 180
   cattcatggt ataaggacac gtataccgag gcaattcctg caattgcggc tacaggagcg 240
   aatactatcc gaattgtatt atctgatgga gggcaatacc aaaaagatga tataaacata 300
   gtcagaaatt tgattgaaac cgcagaagcc aataatttag tcgctgtact tgaggttcat 360
   gatgctactg ggtcggattc attatcggat ttgaaccggg ctgtagatta ttggattgaa 420
   attaaagatg cgttaattgg taaagaagat acggtgatca taaacattgt caatgaatgg 480
   tatggcactt gggatggtcg tctctgggca gatggttata aacaggcgat accgagatta 540
   agagatgctg gattaacaca tacgttgatg attgatgcag caggttgggg gcaatttcct 600
   agctcgatcc atcaatatgg tagagaagta tttaatgcag atcgtttagg gaatacaatg 660
   ttttcgattc atatgtatga atatgctggc ggtgatgatc aaatggttag agataatatt 720
   aacggtgtga tcaatcaaga cttagctcta gtgattggtg aatttggtca ttatcacaca 780
   gatggcgatg ttgatgaaga tacgattttg agttacgcgg agcagacagg tgttggttgg 840
   ttagcatggt catggaaagg caatggaact gagtgggagt atcttgatct atcaaatgat 900
   tggggaggaa attatttaac atcttggggt gacaggattg taaatggagc aaatggatta 960
   agagaaacga gtcaaattgc ttctgttttt tcaggaaaca atggcgggac tcctggaaat 1020
   ggtgaggaag agactcctgg tgatgtaagt catttcgcaa acttcgagaa tggtactgaa 1080
   ggttgggaag caagcaatgt atctggtgga ccttgggcaa caaatgaatg gagtgctagt 1140
   ggttcatatg ctttaaaagc cgatgcgcaa ttagcatctg gaagagaaca ctatttatat 1200
   cgaatcggtc cctttaattt atctgggtca acattaaacg caacggtaag gggtgctaat 1260
   tgggggaatt atggatctgg tatcgacgtg aagctatacg ttaagtacgg agatggctgg 1320
   acgtggagag atagtggtgt acagacaatt agagcgggag aatctattga tctatcacta 1380
   gatttatcaa atgttgatcg ctcaaacatt agagaagttg gtatccagtt tattggtgga 1440
   aatcattcat ctggaaaaac cgctttttat gttgatcatg tttattcaca ttag 1494
<210> 30
   <211> 497
   <212> PRT
   <213> Amphibacillus xylanus
<400> 30
   Val Lys Leu Thr Lys Leu Lys Leu Leu Ser Ser Val Phe Phe Val Val
   1               5                   10                  15
   Leu Thr Val Leu Met Leu Phe Val Pro Gly Asn Ile Val Asn Val Lys
               20                  25                  30
   Ala Ala Asn Gly Phe Tyr Val Ser Asp Ser Asn Leu Tyr Asp Ala Asn
           35                  40                  45
   Gly Asn Gln Phe Val Met Arg Gly Val Asn His Ala His Ser Trp Tyr
       50                  55                  60
   Lys Asp Thr Tyr Thr Glu Ala Ile Pro Ala Ile Ala Ala Thr Gly Ala
   65                  70                  75                  80
   Asn Thr Ile Arg Ile Val Leu Ser Asp Gly Gly Gln Tyr Gln Lys Asp
                   85                  90                  95
   Asp Ile Asn Ile Val Arg Asn Leu Ile Glu Thr Ala Glu Ala Asn Asn
               100                 105                 110
   Leu Val Ala Val Leu Glu Val His Asp Ala Thr Gly Ser Asp Ser Leu
           115                 120                 125
   Ser Asp Leu Asn Arg Ala Val Asp Tyr Trp Ile Glu Ile Lys Asp Ala
       130                 135                 140
   Leu Ile Gly Lys Glu Asp Thr Val Ile Ile Asn Ile Val Asn Glu Trp
   145                 150                 155                 160
   Tyr Gly Thr Trp Asp Gly Arg Leu Trp Ala Asp Gly Tyr Lys Gln Ala
                   165                 170                 175
   Ile Pro Arg Leu Arg Asp Ala Gly Leu Thr His Thr Leu Met Ile Asp
               180                 185                 190
   Ala Ala Gly Trp Gly Gln Phe Pro Ser Ser Ile His Gln Tyr Gly Arg
           195                 200                 205
   Glu Val Phe Asn Ala Asp Arg Leu Gly Asn Thr Met Phe Ser Ile His
       210                 215                 220
   Met Tyr Glu Tyr Ala Gly Gly Asp Asp Gln Met Val Arg Asp Asn Ile
   225                 230                 235                 240
   Asn Gly Val Ile Asn Gln Asp Leu Ala Leu Val Ile Gly Glu Phe Gly
                   245                 250                 255
   His Tyr His Thr Asp Gly Asp Val Asp Glu Asp Thr Ile Leu Ser Tyr
               260                 265                 270
   Ala Glu Gln Thr Gly Val Gly Trp Leu Ala Trp Ser Trp Lys Gly Asn
           275                 280                 285
   Gly Thr Glu Trp Glu Tyr Leu Asp Leu Ser Asn Asp Trp Gly Gly Asn
       290                 295                 300
   Tyr Leu Thr Ser Trp Gly Asp Arg Ile Val Asn Gly Ala Asn Gly Leu
   305                 310                 315                 320
   Arg Glu Thr Ser Gln Ile Ala Ser Val Phe Ser Gly Asn Asn Gly Gly
                   325                 330                 335
   Thr Pro Gly Asn Gly Glu Glu Glu Thr Pro Gly Asp Val Ser His Phe
               340                 345                 350
   Ala Asn Phe Glu Asn Gly Thr Glu Gly Trp Glu Ala Ser Asn Val Ser
           355                 360                 365
   Gly Gly Pro Trp Ala Thr Asn Glu Trp Ser Ala Ser Gly Ser Tyr Ala
       370                 375                 380
   Leu Lys Ala Asp Ala Gln Leu Ala Ser Gly Arg Glu His Tyr Leu Tyr
   385                 390                 395                 400
   Arg Ile Gly Pro Phe Asn Leu Ser Gly Ser Thr Leu Asn Ala Thr Val
                   405                 410                 415
   Arg Gly Ala Asn Trp Gly Asn Tyr Gly Ser Gly Ile Asp Val Lys Leu
               420                 425                 430
   Tyr Val Lys Tyr Gly Asp Gly Trp Thr Trp Arg Asp Ser Gly Val Gln
           435                 440                 445
   Thr Ile Arg Ala Gly Glu Ser Ile Asp Leu Ser Leu Asp Leu Ser Asn
       450                 455                 460
   Val Asp Arg Ser Asn Ile Arg Glu Val Gly Ile Gln Phe Ile Gly Gly
   465 470 475 480
   Asn His Ser Ser Gly Lys Thr Ala Phe Tyr Val Asp His Val Tyr Ser
   485 490 495
   His
<210> 31
   <211> 1767
   <212> DNA
   <213> Paenibacillus polymyxa
<400> 31
   atgaaaaaac tactgtcttg tctcatttcg ctgtcaatgc ttgtgtatat cttaccgaca 60
   atgatagtgt ccgctaacaa tgatggcgta acgaaccttg ctcttgattc aacacctagt 120
   gcgcaaagtg atattatttc tgatgctgtc tacaaaatca cagctcagca ttcaggaaaa 180
   agccttgagg ttgaaggcgg ttctaaagat gacggcgcga atgttcaaca atggacagat 240
   aacgggaaag aacagcagaa atggagagtt gtggacgtcg gtggcggata ttacaagctc 300
   atcagtcaat ctagcggaaa agcactggat gtggcaggtg gtaatacaca tgatggtgcc 360
   aatgtgcaac agtggacgga caacggaaat gctcagcaaa agtggaagat catcgatgta 420
   ggaggaggct attataagtt gatctcacaa agctctggaa aggcactcga cgtcgttggt 480
   ggttatacgc acgacggggc caatgtgcag caatgggcag acaatggatc tgctcaacag 540
   cgctggcgtt tcacacaaat tgatacaacc acggatacga cgccgccaac agcaccaacg 600
   aatttacaat catcatcgaa aacaagtacc tctgtaacat tgacttggac cacaagcatt 660
   gataatgtag gtgtgacagg ctatgtcatt tataatggaa cagatttggt cgggacttct 720
   acaactacat cttatattgt tacaggatta acagcgaaca cttcctataa cttcactgtc 780
   aaagcgaagg atgccgctgg gaatatttca gaaccatcaa atgtcttgaa agtcacaacg 840
   agttcagatt cttctcaaaa cacaggtttt tatgtgaagg gcacaacatt atatgatgga 900
   aacggtaatc catttgtgat gagaggaatc aatcatgcat acacatggta taaagggcaa 960
   gaatcagtag caattcctgc gattgcgaaa acgggtgcaa acaccatccg gattgtctta 1020
   tctgacggac agcagtggac aaaagatgat ttaagcgcgc ttcaaaattt gattacactc 1080
   agtgagcaaa acaaacttgt agtgatttta gaggtgcacg acggtactgg caatgacaat 1140
   gccgcagttt taaataaaat tgctgattat tggattgaaa tgaagtcagc tttaattggg 1200
   aaggaaaata cagttatttt aaacatcgca aatgaatggt ttggtacatg ggatggaaac 1260
   ggctgggcgc agggctacaa atcagtcata ccaaagctgc gaaatgcggg catcaaaaac 1320
   acgattatgg tggatgcggc tggatgggga caatatccaa aatcgatttt tgattacgga     1380
   acgcaagtgt tcgatgcaga tccgctcaag aatacgatgt tttccattca tatgtatgaa     1440
   tacgcaggcg gcaacgcaga aacagtgaaa agtaatatcg acaacgtcct gaataaaaat     1500
   cttgcactca tcattggaga atttggaatt aaacatacaa acggagatgt tgatgaagca     1560
   acgatcatgt catacgcaca gcaaaaaggt gttgggtatc ttggctggtc atggaaagga     1620
   aatggttcag gtcttgaata tttagatatg agtaacgatt gggctggcag cagttataca     1680
   gagcaaggac atgccattat cgaaggacca aatggcattc gtgcaacatc aaaattatca     1740
   accatttaca gcaatgggaa acaataa                                         1767
<210> 32
   <211> 588
   <212> PRT
   <213> Paenibacillus polymyxa
<400> 32
   Met Lys Lys Leu Leu Ser Cys Leu Ile Ser Leu Ser Met Leu Val Tyr
   1               5                   10                  15
   Ile Leu Pro Thr Met Ile Val Ser Ala Asn Asn Asp Gly Val Thr Asn
               20                  25                  30
   Leu Ala Leu Asp Ser Thr Pro Ser Ala Gln Ser Asp Ile Ile Ser Asp
           35                  40                  45
   Ala Val Tyr Lys Ile Thr Ala Gln His Ser Gly Lys Ser Leu Glu Val
       50                  55                  60
   Glu Gly Gly Ser Lys Asp Asp Gly Ala Asn Val Gln Gln Trp Thr Asp
   65                  70                  75                  80
   Asn Gly Lys Glu Gln Gln Lys Trp Arg Val Val Asp Val Gly Gly Gly
                   85                  90                  95
   Tyr Tyr Lys Leu Ile Ser Gln Ser Ser Gly Lys Ala Leu Asp Val Ala
               100                 105                 110
   Gly Gly Asn Thr His Asp Gly Ala Asn Val Gln Gln Trp Thr Asp Asn
           115                 120                 125
   Gly Asn Ala Gln Gln Lys Trp Lys Ile Ile Asp Val Gly Gly Gly Tyr
       130                 135                 140
   Tyr Lys Leu Ile Ser Gln Ser Ser Gly Lys Ala Leu Asp Val Val Gly
   145                 150                 155                 160
   Gly Tyr Thr His Asp Gly Ala Asn Val Gln Gln Trp Ala Asp Asn Gly
                   165                 170                 175
   Ser Ala Gln Gln Arg Trp Arg Phe Thr Gln Ile Asp Thr Thr Thr Asp
               180                 185                 190
   Thr Thr Pro Pro Thr Ala Pro Thr Asn Leu Gln Ser Ser Ser Lys Thr
           195                 200                 205
   Ser Thr Ser Val Thr Leu Thr Trp Thr Thr Ser Ile Asp Asn Val Gly
       210                 215                 220
   Val Thr Gly Tyr Val Ile Tyr Asn Gly Thr Asp Leu Val Gly Thr Ser
   225                 230                 235                 240
   Thr Thr Thr Ser Tyr Ile Val Thr Gly Leu Thr Ala Asn Thr Ser Tyr
                   245                 250                 255
   Asn Phe Thr Val Lys Ala Lys Asp Ala Ala Gly Asn Ile Ser Glu Pro
               260                 265                 270
   Ser Asn Val Leu Lys Val Thr Thr Ser Ser Asp Ser Ser Gln Asn Thr
           275                 280                 285
   Gly Phe Tyr Val Lys Gly Thr Thr Leu Tyr Asp Gly Asn Gly Asn Pro
       290                 295                 300
   Phe Val Met Arg Gly Ile Asn His Ala Tyr Thr Trp Tyr Lys Gly Gln
   305                 310                 315                 320
   Glu Ser Val Ala Ile Pro Ala Ile Ala Lys Thr Gly Ala Asn Thr Ile
                   325                 330                 335
   Arg Ile Val Leu Ser Asp Gly Gln Gln Trp Thr Lys Asp Asp Leu Ser
               340                 345                 350
   Ala Leu Gln Asn Leu Ile Thr Leu Ser Glu Gln Asn Lys Leu Val Val
           355                 360                 365
   Ile Leu Glu Val His Asp Gly Thr Gly Asn Asp Asn Ala Ala Val Leu
       370                 375                 380
   Asn Lys Ile Ala Asp Tyr Trp Ile Glu Met Lys Ser Ala Leu Ile Gly
   385                 390                 395                 400
   Lys Glu Asn Thr Val Ile Leu Asn Ile Ala Asn Glu Trp Phe Gly Thr
   405 410 415
   Trp Asp Gly Asn Gly Trp Ala Gln Gly Tyr Lys Ser Val Ile Pro Lys
   420 425 430
   Leu Arg Asn Ala Gly Ile Lys Asn Thr Ile Met Val Asp Ala Ala Gly
   435 440 445
   Trp Gly Gln Tyr Pro Lys Ser Ile Phe Asp Tyr Gly Thr Gln Val Phe
   450 455 460
   Asp Ala Asp Pro Leu Lys Asn Thr Met Phe Ser Ile His Met Tyr Glu
   465 470 475 480
   Tyr Ala Gly Gly Asn Ala Glu Thr Val Lys Ser Asn Ile Asp Asn Val
   485 490 495
   Leu Asn Lys Asn Leu Ala Leu Ile Ile Gly Glu Phe Gly Ile Lys His
   500 505 510
   Thr Asn Gly Asp Val Asp Glu Ala Thr Ile Met Ser Tyr Ala Gln Gln
   515 520 525
   Lys Gly Val Gly Tyr Leu Gly Trp Ser Trp Lys Gly Asn Gly Ser Gly
   530 535 540
   Leu Glu Tyr Leu Asp Met Ser Asn Asp Trp Ala Gly Ser Ser Tyr Thr
   545 550 555 560
   Glu Gln Gly His Ala Ile Ile Glu Gly Pro Asn Gly Ile Arg Ala Thr
   565 570 575
   Ser Lys Leu Ser Thr Ile Tyr Ser Asn Gly Lys Gln
   580 585
<210> 33
   <211> 1470
   <212> DNA
   <213> Bacillus hemicellulosilyticus
<400> 33
   atggatatat taagaaagtg tgtacttgta ctattggcct tactattgtt gttacctacg      60
   acatcaacgg cattttctga aagcgcttct actaatgaga gagtgctaaa tttatctgat     120
   ccgaatgcga cacgctatac gaaggaattg tttgcgtttc ttcaagacgt gagtggtgag     180
   caagtgttgt tcgggcaaca gcatgcaaca gatgaagggt tgactctgac aggtgaagga     240
   aatcgaattg gttcaactga gtcggaggtg aagaatgcag taggtgatta tccagctgtt     300
   tttgggtggg atacgaacag cttggatggt cgtgaaaagc caggtacaga tgtggaaagt     360
   caagagcaac gaattttaaa tacagcagaa tcgatgaaag tggcacatga attaggaggg     420
   atcatcacat taagtatgca tccggataac tttgttaccg gtcattacta tggcgatacg     480
   gatggtaacg tcgttcaaga aatattgcca ggtggctcca agcacaatga atttaacgct     540
   tggctagata atattgctgc cctagcacat gaattagttg atgataatgg agagcctatt     600
   ccggttatct tccgtccatt ccatgagcaa acaggttcgt ggttttggtg gggtgcgagc     660
   acaacaactc ctgagcaata caaagcgatt tttcgatata cagtcgaata cttaagagat     720
   gcaaagggtg ttcataactt tttatatgga ttctcccctg gtgcgggtcc tgctggcgat     780
   ctagatcgat atttagaaac gtacccaggt gataattatg tcgatatctt aggtattgat     840
   aattatgata gtaagtcaaa tgcggggtca gacgcttggt tatctggaat ggtaaaagat     900
   ttagcgatga tctcgaaatt agcagaggaa agagggaagg tatcagcctt tactgaattt     960
   ggatacagcg ctgaagggat gagtcaaacg ggtgatgcgt tagattggta tacacgtgtg    1020
   ttaaatgcga taaaagcaga tgaagatgcg cgaaacatat cctacatgct aacgtgggct    1080
   aactttgggt ggcctaataa tatatttgtt ccgtatcgtg atgtgaatgg ggatttaggt    1140
   ggagatcatg agttattacc tgactttgta cagttttatg aagatgaata ctcagcattt    1200
   cgtgaagata taaatgaaag tgtttacaat cgtaatgaga gttatattgt tgcggatcat    1260
   gagccattta tgtatgttgt ttcccctacg acaggtacat atataacagg ctcgtctgtt    1320
   gtcttacgag cgaaagtagt taacgatgag gatccgtccg ttacgtatca agtggcgggt    1380
   tctgaagaag tctatgagat gactttagat gaaaatgggt attactctgc tgattatatt    1440
   cctactgctc ctaagaatgg agctctgtag                                     1470
<210> 34
   <211> 489
   <212> PRT
   <213> Bacillus hemicellulosilyticus
<400> 34
   Met Asp Ile Leu Arg Lys Cys Val Leu Val Leu Leu Ala Leu Leu Leu
   1               5                   10                  15
   Leu Leu Pro Thr Thr Ser Thr Ala Phe Ser Glu Ser Ala Ser Thr Asn
               20                  25                  30
   Glu Arg Val Leu Asn Leu Ser Asp Pro Asn Ala Thr Arg Tyr Thr Lys
           35                  40                  45
   Glu Leu Phe Ala Phe Leu Gln Asp Val Ser Gly Glu Gln Val Leu Phe
       50                  55                  60
   Gly Gln Gln His Ala Thr Asp Glu Gly Leu Thr Leu Thr Gly Glu Gly
   65                  70                  75                  80
   Asn Arg Ile Gly Ser Thr Glu Ser Glu Val Lys Asn Ala Val Gly Asp
                   85                  90                  95
   Tyr Pro Ala Val Phe Gly Trp Asp Thr Asn Ser Leu Asp Gly Arg Glu
               100                 105                 110
   Lys Pro Gly Thr Asp Val Glu Ser Gln Glu Gln Arg Ile Leu Asn Thr
           115                 120                 125
   Ala Glu Ser Met Lys Val Ala His Glu Leu Gly Gly Ile Ile Thr Leu
       130                 135                 140
   Ser Met His Pro Asp Asn Phe Val Thr Gly His Tyr Tyr Gly Asp Thr
   145                 150                 155                 160
   Asp Gly Asn Val Val Gln Glu Ile Leu Pro Gly Gly Ser Lys His Asn
                   165                 170                 175
   Glu Phe Asn Ala Trp Leu Asp Asn Ile Ala Ala Leu Ala His Glu Leu
               180                 185                 190
   Val Asp Asp Asn Gly Glu Pro Ile Pro Val Ile Phe Arg Pro Phe His
           195                 200                 205
   Glu Gln Thr Gly Ser Trp Phe Trp Trp Gly Ala Ser Thr Thr Thr Pro
       210                 215                 220
   Glu Gln Tyr Lys Ala Ile Phe Arg Tyr Thr Val Glu Tyr Leu Arg Asp
   225                 230                 235                 240
   Ala Lys Gly Val His Asn Phe Leu Tyr Gly Phe Ser Pro Gly Ala Gly
                   245                 250                 255
   Pro Ala Gly Asp Leu Asp Arg Tyr Leu Glu Thr Tyr Pro Gly Asp Asn
               260                 265                 270
   Tyr Val Asp Ile Leu Gly Ile Asp Asn Tyr Asp Ser Lys Ser Asn Ala
           275                 280                 285
   Gly Ser Asp Ala Trp Leu Ser Gly Met Val Lys Asp Leu Ala Met Ile
       290                 295                 300
   Ser Lys Leu Ala Glu Glu Arg Gly Lys Val Ser Ala Phe Thr Glu Phe
   305                 310                 315                 320
   Gly Tyr Ser Ala Glu Gly Met Ser Gln Thr Gly Asp Ala Leu Asp Trp
                   325                 330                 335
   Tyr Thr Arg Val Leu Asn Ala Ile Lys Ala Asp Glu Asp Ala Arg Asn
               340                 345                 350
   Ile Ser Tyr Met Leu Thr Trp Ala Asn Phe Gly Trp Pro Asn Asn Ile
           355                 360                 365
   Phe Val Pro Tyr Arg Asp Val Asn Gly Asp Leu Gly Gly Asp His Glu
       370                 375                 380
   Leu Leu Pro Asp Phe Val Gln Phe Tyr Glu Asp Glu Tyr Ser Ala Phe
   385                 390                 395                 400
   Arg Glu Asp Ile Asn Glu Ser Val Tyr Asn Arg Asn Glu Ser Tyr Ile
                   405                 410                 415
   Val Ala Asp His Glu Pro Phe Met Tyr Val Val Ser Pro Thr Thr Gly
               420                 425                 430
   Thr Tyr Ile Thr Gly Ser Ser Val Val Leu Arg Ala Lys Val Val Asn
           435                 440                 445
   Asp Glu Asp Pro Ser Val Thr Tyr Gln Val Ala Gly Ser Glu Glu Val
       450                 455                 460
   Tyr Glu Met Thr Leu Asp Glu Asn Gly Tyr Tyr Ser Ala Asp Tyr Ile
   465                 470                 475                 480
   Pro Thr Ala Pro Lys Asn Gly Ala Leu
                   485
<210> 35
   <211> 1110
   <212> DNA
   <213> Bacillus alcalophilus
<400> 35
   atgagaagta tgaagctttt atttgctatg tttattttag ttttttcctc ttttactttt    60
   aacttagtag ttgcgcaagc tagtggacat ggacaaatgc ataaagtacc ttgggcaccc   120
   caagctgaag cacctggaaa aacggctgag aatggagtct gggataaagt tagaaataat   180
   cctggaaaag ccaatcctcc agcaggaaaa gtcaatggtt tttatataga tggaacaacc   240
   ttatatgatg caaatggtaa gccatttgtg atgcgcggaa ttaaccacgc tcattcctgg   300
   tacaagcctc acatagaaac cgcgatggag gcaattgctg atactggagc aaactccatt   360
   cgtgtagttc tctcagatgg acaacagtgg accaaagatg atgttgacga agtagcaaaa   420
   attatatctt tagcagaaaa acattcttta gttgctgttc ttgaggtaca tgatgcactc   480
   ggaacagatg atattgaacc attacttaaa acagtcgatt actggattga gatcaaagat   540
   gctttaatcg gaaaagagga caaagtaatt attaacattt ctaatgaatg gtttggttct   600
   tggagcagtg aaggttgggc agaaggatat aaaaaagcaa ttcctttact aagagaggcg   660
   ggtctaaaac atacacttat ggttgacgca gctgggtggg gacaatttcc tagatctatt   720
   catgaaaaag gattagacgt ttttaactca gacccattaa agaatacaat gttttccatt   780
   catatgtatg aatgggcagc gggtaatcct caacaagtaa aagacaatat tgacggtgtt   840
   cttgaaaaga atttagctgt agtaattggt gagttcggtc atcatcacta cggaagagat   900
   gttgctgttg atacgatctt aagtcattca gagaagtatg atgtaggttg gcttgcctgg   960
   tcttggcacg gaaatagtgg tggtgtagag tatcttgact tagcaacaga tttttcaggg  1020
   acgcaactaa ctgaatgggg agaaagaatt gtgtacggtc cgaatggttt aaaagaaact  1080
   tctgaaatcg ttagtgtata caaaaaataa                                   1110
<210> 36
   <211> 369
   <212> PRT
   <213> Bacillus alcalophilus
<400> 36
   Met Arg Ser Met Lys Leu Leu Phe Ala Met Phe Ile Leu Val Phe Ser
   1               5                   10                  15
   Ser Phe Thr Phe Asn Leu Val Val Ala Gln Ala Ser Gly His Gly Gln
               20                  25                  30
   Met His Lys Val Pro Trp Ala Pro Gln Ala Glu Ala Pro Gly Lys Thr
           35                  40                  45
   Ala Glu Asn Gly Val Trp Asp Lys Val Arg Asn Asn Pro Gly Lys Ala
       50                  55                  60
   Asn Pro Pro Ala Gly Lys Val Asn Gly Phe Tyr Ile Asp Gly Thr Thr
   65                  70                  75                  80
   Leu Tyr Asp Ala Asn Gly Lys Pro Phe Val Met Arg Gly Ile Asn His
                   85                  90                  95
   Ala His Ser Trp Tyr Lys Pro His Ile Glu Thr Ala Met Glu Ala Ile
               100                 105                 110
   Ala Asp Thr Gly Ala Asn Ser Ile Arg Val Val Leu Ser Asp Gly Gln
   115 120 125
   Gln Trp Thr Lys Asp Asp Val Asp Glu Val Ala Lys Ile Ile Ser Leu
   130 135 140
   Ala Glu Lys His Ser Leu Val Ala Val Leu Glu Val His Asp Ala Leu
   145 150 155 160
   Gly Thr Asp Asp Ile Glu Pro Leu Leu Lys Thr Val Asp Tyr Trp Ile
   165 170 175
   Glu Ile Lys Asp Ala Leu Ile Gly Lys Glu Asp Lys Val Ile Ile Asn
   180 185 190
   Ile Ser Asn Glu Trp Phe Gly Ser Trp Ser Ser Glu Gly Trp Ala Glu
   195 200 205
   Gly Tyr Lys Lys Ala Ile Pro Leu Leu Arg Glu Ala Gly Leu Lys His
   210 215 220
   Thr Leu Met Val Asp Ala Ala Gly Trp Gly Gln Phe Pro Arg Ser Ile
   225 230 235 240
   His Glu Lys Gly Leu Asp Val Phe Asn Ser Asp Pro Leu Lys Asn Thr
   245 250 255
   Met Phe Ser Ile His Met Tyr Glu Trp Ala Ala Gly Asn Pro Gln Gln
   260 265 270
   Val Lys Asp Asn Ile Asp Gly Val Leu Glu Lys Asn Leu Ala Val Val
   275 280 285
   Ile Gly Glu Phe Gly His His His Tyr Gly Arg Asp Val Ala Val Asp
   290 295 300
   Thr Ile Leu Ser His Ser Glu Lys Tyr Asp Val Gly Trp Leu Ala Trp
   305 310 315 320
   Ser Trp His Gly Asn Ser Gly Gly Val Glu Tyr Leu Asp Leu Ala Thr
   325 330 335
   Asp Phe Ser Gly Thr Gln Leu Thr Glu Trp Gly Glu Arg Ile Val Tyr
   340 345 350
   Gly Pro Asn Gly Leu Lys Glu Thr Ser Glu Ile Val Ser Val Tyr Lys
   355 360 365
   Lys
<210> 37
   <211> 1482
   <212> DNA
   <213> Bacillus sp.
<400> 37
   atgaaaaaaa agttatcaca gatttatcat ttaattattt gcacacttat aataagtgtg      60
   ggaataatgg ggattacaac gtccccatca gaagcaagtt caggctttta tgttgatggc     120
   aatacgttat atgacgcaaa cgggcaacca tttgtcatga aaggcattaa ccatggacat     180
   gcttggtata aagacaccgc ttcaacagct attcctgcca ttgcagagca aggcgcgaac     240
   acgatacgta ttgttttatc agatggcggt caatgggaaa aagacgacat tgacaccgtt     300
   cgtgaagtta ttgagcttgc ggagcaaaat aaaatggtgg ctgtcgttga agttcatgat     360
   gccacgggcc gtgattcacg cagtgattta gatcgggcag tcgattattg gatagagatg     420
   aaagatgcac ttatcggcaa agaggatact gtcattatta acattgcaaa cgaatggtat     480
   ggcagttggg atggcgccgc ttgggctgat ggctacattg atgtcattcc gaagcttcgc     540
   gatgccggct taacacacac cttaatggtt gatgcagcag gatgggggca atatccgcaa     600
   tctattcatg attacggaca agatgtgttt aatgcagatc cgttaaaaaa tacgatattc     660
   tccatccata tgtatgagta tgctggtggt gatgctaaca ctgttagatc aaatattgat     720
   agagtcatag atcaagacct tgctctcgta ataggtgagt tcggtcatag acacactgat     780
   ggcgatgttg atgaagatac aatccttagt tattctgaag aaactggcac aggatggctc     840
   gcttggtctt ggaaaggcaa cagtgccgaa tgggattatt tagacctttc agaagattgg     900
   gctggtaacc atttaactga ttggggaaat aggattgtcc acggggcaaa tggcttgcag     960
   gaaacctcca aaccatccac cgtatttaca gatgataacg gtggtgcccc tgaaccgcca    1020
   actactacta ccttgtatga ctttgaagga agcacacaag ggtggcatgg aagcaacgtg    1080
   atgggtggcc cttggtccgt aacagaatgg ggtgcgtcag gcaactactc tttaaagggc    1140
   gatgtcaatt taagctcaaa ttcttcacat gaactgtata gtgaacaaag tcgtaatcta    1200
   cacggatact ctcagctaaa tgcaaccgtt cgccatgcca attggggaaa tcccggtaat    1260
   ggcatgaatg caagacttta cgtgaaaacg ggctctgatt atacatggta tagcggtcct    1320
   tttacacgta tcaatagctc caactcaggt acaacgttat cttttgattt aaacaacatc    1380
   gaaaatagtc atcatgttag ggaaataggt gtgcaatttt cagctgcaga taatagcagc    1440
   ggtcaaactg ctctatacgt tgataatgtt actttaagat aa                        1482
<210> 38
   <211> 493
   <212> PRT
   <213> Bacillus sp.
<400> 38
   Met Lys Lys Lys Leu Ser Gln Ile Tyr His Leu Ile Ile Cys Thr Leu
   1               5                   10                  15
   Ile Ile Ser Val Gly Ile Met Gly Ile Thr Thr Ser Pro Ser Glu Ala
               20                  25                  30
   Ser Ser Gly Phe Tyr Val Asp Gly Asn Thr Leu Tyr Asp Ala Asn Gly
           35                  40                  45
   Gln Pro Phe Val Met Lys Gly Ile Asn His Gly His Ala Trp Tyr Lys
       50                  55                  60
   Asp Thr Ala Ser Thr Ala Ile Pro Ala Ile Ala Glu Gln Gly Ala Asn
   65                  70                  75                  80
   Thr Ile Arg Ile Val Leu Ser Asp Gly Gly Gln Trp Glu Lys Asp Asp
                   85                  90                  95
   Ile Asp Thr Val Arg Glu Val Ile Glu Leu Ala Glu Gln Asn Lys Met
               100                 105                 110
   Val Ala Val Val Glu Val His Asp Ala Thr Gly Arg Asp Ser Arg Ser
           115                 120                 125
   Asp Leu Asp Arg Ala Val Asp Tyr Trp Ile Glu Met Lys Asp Ala Leu
       130                 135                 140
   Ile Gly Lys Glu Asp Thr Val Ile Ile Asn Ile Ala Asn Glu Trp Tyr
   145                 150                 155                 160
   Gly Ser Trp Asp Gly Ala Ala Trp Ala Asp Gly Tyr Ile Asp Val Ile
                   165                 170                 175
   Pro Lys Leu Arg Asp Ala Gly Leu Thr His Thr Leu Met Val Asp Ala
               180                 185                 190
   Ala Gly Trp Gly Gln Tyr Pro Gln Ser Ile His Asp Tyr Gly Gln Asp
           195                 200                 205
   Val Phe Asn Ala Asp Pro Leu Lys Asn Thr Ile Phe Ser Ile His Met
       210                 215                 220
   Tyr Glu Tyr Ala Gly Gly Asp Ala Asn Thr Val Arg Ser Asn Ile Asp
   225 230 235 240
   Arg Val Ile Asp Gln Asp Leu Ala Leu Val Ile Gly Glu Phe Gly His
   245 250 255
   Arg His Thr Asp Gly Asp Val Asp Glu Asp Thr Ile Leu Ser Tyr Ser
   260 265 270
   Glu Glu Thr Gly Thr Gly Trp Leu Ala Trp Ser Trp Lys Gly Asn Ser
   275 280 285
   Ala Glu Trp Asp Tyr Leu Asp Leu Ser Glu Asp Trp Ala Gly Asn His
   290 295 300
   Leu Thr Asp Trp Gly Asn Arg Ile Val His Gly Ala Asn Gly Leu Gln
   305 310 315 320
   Glu Thr Ser Lys Pro Ser Thr Val Phe Thr Asp Asp Asn Gly Gly Ala
   325 330 335
   Pro Glu Pro Pro Thr Thr Thr Thr Leu Tyr Asp Phe Glu Gly Ser Thr
   340 345 350
   Gln Gly Trp His Gly Ser Asn Val Met Gly Gly Pro Trp Ser Val Thr
   355 360 365
   Glu Trp Gly Ala Ser Gly Asn Tyr Ser Leu Lys Gly Asp Val Asn Leu
   370 375 380
   Ser Ser Asn Ser Ser His Glu Leu Tyr Ser Glu Gln Ser Arg Asn Leu
   385 390 395 400
   His Gly Tyr Ser Gln Leu Asn Ala Thr Val Arg His Ala Asn Trp Gly
   405 410 415
   Asn Pro Gly Asn Gly Met Asn Ala Arg Leu Tyr Val Lys Thr Gly Ser
   420 425 430
   Asp Tyr Thr Trp Tyr Ser Gly Pro Phe Thr Arg Ile Asn Ser Ser Asn
   435 440 445
   Ser Gly Thr Thr Leu Ser Phe Asp Leu Asn Asn Ile Glu Asn Ser His
   450 455 460
   His Val Arg Glu Ile Gly Val Gln Phe Ser Ala Ala Asp Asn Ser Ser
   465 470 475 480
   Gly Gln Thr Ala Leu Tyr Val Asp Asn Val Thr Leu Arg
                   485                     490
<210> 39
   <211> 1551
   <212> DNA
   <213> Bacillus circulans
<400> 39
   atggggtggt ttttagtgat tttacgcaag tggttgattg cttttgtcgc atttttactg      60
   atgttctcgt ggactggaca acttacgaac aaagcacatg ctgcaagcgg attttatgta     120
   agcggtacca aattattgga tgctacagga caaccatttg tgatgcgagg agtcaatcat     180
   gcgcacacat ggtataaaga tcaactatcc accgcaatac cagccattgc taaaacaggt     240
   gccaacacga tacgtattgt actggcgaat ggacacaaat ggacgcttga tgatgtaaac     300
   accgtcaaca atattctcac cctctgtgaa caaaacaaac taattgccgt tttggaagta     360
   catgacgcta caggaagcga tagtctttcc gatttagaca acgccgttaa ttactggatt     420
   ggtattaaaa gcgcgttgat cggcaaggaa gaccgtgtaa tcattaatat agctaacgag     480
   tggtacggaa catgggatgg agtcgcctgg gctaatggtt ataagcaagc catacccaaa     540
   ctgcgtaatg ctggtctaac tcatacgctg attgttgact ccgctggatg gggacaatat     600
   ccagattcgg tcaaaaatta tgggacagaa gtactgaatg cagacccgtt aaaaaacaca     660
   gtattctcta tccatatgta tgaatatgct gggggcaatg caagtaccgt caaatccaat     720
   attgacggtg tgctgaacaa gaatcttgca ctgattatcg gcgaatttgg tggacaacat     780
   acaaacggtg atgtggatga agccaccatt atgagttatt cccaagagaa gggagtcggc     840
   tggttggctt ggtcctggaa gggaaatagc agtgatttgg cttatctcga tatgacaaat     900
   gattgggctg gtaactccct cacctcgttc ggtaataccg tagtgaatgg cagtaacggc     960
   attaaagcaa cttctgtgtt atccggcatt tttggaggtg ttacgccaac ctcaagccct    1020
   acttctacac ctacatctac gccaacctca actcctactc ctacgccaag tccgaccccg    1080
   agtccaggta ataacgggac gatcttatat gatttcgaaa caggaactca aggctggtcg    1140
   ggaaacaata tttcgggagg cccatgggtc accaatgaat ggaaagcaac gggagcgcaa    1200
   actctcaaag ccgatgtctc cttacaatcc aattccacgc atagtctata tataacctct    1260
   aatcaaaatc tgtctggaaa aagcagtctg aaagcaacgg ttaagcatgc gaactggggc    1320
   aatatcggca acgggattta tgcaaaacta tacgtaaaga ccgggtccgg gtggacatgg    1380
   tacgattccg gagagaatct gattcagtca aacgacggta ccattttgac actatccctc    1440
   agcggcattt cgaatttgtc ctcagtcaaa gaaattgggg tagaattccg cgcctcctca    1500
   aacagtagtg gccaatcagc tatttatgta gatagtgtta gtctgcaata a             1551
<210> 40
   <211> 516
   <212> PRT
   <213> Bacillus circulans
<400> 40
   Met Gly Trp Phe Leu Val Ile Leu Arg Lys Trp Leu Ile Ala Phe Val
   1               5                   10                  15
   Ala Phe Leu Leu Met Phe Ser Trp Thr Gly Gln Leu Thr Asn Lys Ala
               20                  25                  30
   His Ala Ala Ser Gly Phe Tyr Val Ser Gly Thr Lys Leu Leu Asp Ala
           35                  40                  45
   Thr Gly Gln Pro Phe Val Met Arg Gly Val Asn His Ala His Thr Trp
       50                  55                  60
   Tyr Lys Asp Gln Leu Ser Thr Ala Ile Pro Ala Ile Ala Lys Thr Gly
   65                  70                  75                  80
   Ala Asn Thr Ile Arg Ile Val Leu Ala Asn Gly His Lys Trp Thr Leu
                   85                  90                  95
   Asp Asp Val Asn Thr Val Asn Asn Ile Leu Thr Leu Cys Glu Gln Asn
               100                 105                 110
   Lys Leu Ile Ala Val Leu Glu Val His Asp Ala Thr Gly Ser Asp Ser
           115                 120                 125
   Leu Ser Asp Leu Asp Asn Ala Val Asn Tyr Trp Ile Gly Ile Lys Ser
       130                 135                 140
   Ala Leu Ile Gly Lys Glu Asp Arg Val Ile Ile Asn Ile Ala Asn Glu
   145                 150                 155                 160
   Trp Tyr Gly Thr Trp Asp Gly Val Ala Trp Ala Asn Gly Tyr Lys Gln
                   165                 170                 175
   Ala Ile Pro Lys Leu Arg Asn Ala Gly Leu Thr His Thr Leu Ile Val
               180                 185                 190
   Asp Ser Ala Gly Trp Gly Gln Tyr Pro Asp Ser Val Lys Asn Tyr Gly
           195                 200                 205
   Thr Glu Val Leu Asn Ala Asp Pro Leu Lys Asn Thr Val Phe Ser Ile
       210                 215                 220
   His Met Tyr Glu Tyr Ala Gly Gly Asn Ala Ser Thr Val Lys Ser Asn
   225                 230                 235                 240
   Ile Asp Gly Val Leu Asn Lys Asn Leu Ala Leu Ile Ile Gly Glu Phe
                   245                 250                 255
   Gly Gly Gln His Thr Asn Gly Asp Val Asp Glu Ala Thr Ile Met Ser
               260                 265                 270
   Tyr Ser Gln Glu Lys Gly Val Gly Trp Leu Ala Trp Ser Trp Lys Gly
           275                 280                 285
   Asn Ser Ser Asp Leu Ala Tyr Leu Asp Met Thr Asn Asp Trp Ala Gly
       290                 295                 300
   Asn Ser Leu Thr Ser Phe Gly Asn Thr Val Val Asn Gly Ser Asn Gly
   305                 310                 315                 320
   Ile Lys Ala Thr Ser Val Leu Ser Gly Ile Phe Gly Gly Val Thr Pro
                   325                 330                 335
   Thr Ser Ser Pro Thr Ser Thr Pro Thr Ser Thr Pro Thr Ser Thr Pro
               340                 345                 350
   Thr Pro Thr Pro Ser Pro Thr Pro Ser Pro Gly Asn Asn Gly Thr Ile
           355                 360                 365
   Leu Tyr Asp Phe Glu Thr Gly Thr Gln Gly Trp Ser Gly Asn Asn Ile
       370                 375                 380
   Ser Gly Gly Pro Trp Val Thr Asn Glu Trp Lys Ala Thr Gly Ala Gln
   385                 390                 395                 400
   Thr Leu Lys Ala Asp Val Ser Leu Gln Ser Asn Ser Thr His Ser Leu
                   405                 410                 415
   Tyr Ile Thr Ser Asn Gln Asn Leu Ser Gly Lys Ser Ser Leu Lys Ala
               420                 425                 430
   Thr Val Lys His Ala Asn Trp Gly Asn Ile Gly Asn Gly Ile Tyr Ala
           435                 440                 445
   Lys Leu Tyr Val Lys Thr Gly Ser Gly Trp Thr Trp Tyr Asp Ser Gly
       450                 455                 460
   Glu Asn Leu Ile Gln Ser Asn Asp Gly Thr Ile Leu Thr Leu Ser Leu
   465                 470                 475                 480
   Ser Gly Ile Ser Asn Leu Ser Ser Val Lys Glu Ile Gly Val Glu Phe
   485 490 495
   Arg Ala Ser Ser Asn Ser Ser Gly Gln Ser Ala Ile Tyr Val Asp Ser
   500 505 510
   Val Ser Leu Gln
   515
<210> 41
   <211> 984
   <212> DNA
   <213> Paenibacillus sp.
<400> 41
   atgagacaac ttttagcaaa aggtatttta gctgcactgg tcatgatgtt agcgatgtat 60
   ggattgggga atctctcttc taaagcttcg gctgcaacag gtttttatgt aagcggtacc 120
   actctatatg attctactgg taaacctttt gtaatgcgcg gtgtcaatca ttcgcatacc 180
   tggttcaaaa atgatctaaa tgcagccatc cctgctattg ccaaaacagg tgcaaataca 240
   gtacgtatcg ttttatctaa tggtgttcag tatactagag atgatgtaaa ctcagtcaaa 300
   aatattattt ccctggttaa ccaaaacaaa atgattgctg ttcttgaggt gcatgatgct 360
   accggtaaag acgattacgc ttctcttgat gccgctgtaa actactggat cagcatcaaa 420
   gatgccttga ttggcaagga agatcgagtc attgttaata ttgccaatga atggtacggt 480
   acatggaatg gcagtgcttg ggcagatggt tataagcagg ctattcccaa actaagaaat 540
   gcaggcatca aaaacacttt aatcgttgat gccgccggct ggggacaatg tcctcaatcg 600
   atcgttgatt acgggcaaag tgtatttgca gcagattcgc ttaaaaatac aattttctct 660
   attcacatgt atgaatatgc aggcggtaca gatgcgatcg tcaaaagcaa tatggaaaat 720
   gtactgaaca aaggacttcc tttgatcatc ggtgaatttg gcgggcagca tacaaacggc 780
   gatgtagatg aacatgcaat tatgcgttat ggtcagcaaa aaggtgtagg ttggctggca 840
   tggtcgtggt atggcaacaa tagtgaactc agttatctgg atttggctac aggtcccgcc 900
   ggtagtctga caagtatcgg caatacgatt gtaaatgatc catatggtat caaagctacc 960
   tcgaaaaaag cgggtatctt ctaa 984
<210> 42
   <211> 327
   <212> PRT
   <213> Paenibacillus sp.
<400> 42
   Met Arg Gln Leu Leu Ala Lys Gly Ile Leu Ala Ala Leu Val Met Met
   1               5                   10                  15
   Leu Ala Met Tyr Gly Leu Gly Asn Leu Ser Ser Lys Ala Ser Ala Ala
               20                  25                  30
   Thr Gly Phe Tyr Val Ser Gly Thr Thr Leu Tyr Asp Ser Thr Gly Lys
           35                  40                  45
   Pro Phe Val Met Arg Gly Val Asn His Ser His Thr Trp Phe Lys Asn
       50                  55                  60
   Asp Leu Asn Ala Ala Ile Pro Ala Ile Ala Lys Thr Gly Ala Asn Thr
   65                  70                  75                  80
   Val Arg Ile Val Leu Ser Asn Gly Val Gln Tyr Thr Arg Asp Asp Val
                   85                  90                  95
   Asn Ser Val Lys Asn Ile Ile Ser Leu Val Asn Gln Asn Lys Met Ile
               100                 105                 110
   Ala Val Leu Glu Val His Asp Ala Thr Gly Lys Asp Asp Tyr Ala Ser
           115                 120                 125
   Leu Asp Ala Ala Val Asn Tyr Trp Ile Ser Ile Lys Asp Ala Leu Ile
       130                 135                 140
   Gly Lys Glu Asp Arg Val Ile Val Asn Ile Ala Asn Glu Trp Tyr Gly
   145                 150                 155                 160
   Thr Trp Asn Gly Ser Ala Trp Ala Asp Gly Tyr Lys Gln Ala Ile Pro
                   165                 170                 175
   Lys Leu Arg Asn Ala Gly Ile Lys Asn Thr Leu Ile Val Asp Ala Ala
               180                 185                 190
   Gly Trp Gly Gln Cys Pro Gln Ser Ile Val Asp Tyr Gly Gln Ser Val
           195                 200                 205
   Phe Ala Ala Asp Ser Leu Lys Asn Thr Ile Phe Ser Ile His Met Tyr
       210                 215                 220
   Glu Tyr Ala Gly Gly Thr Asp Ala Ile Val Lys Ser Asn Met Glu Asn
   225                 230                 235                 240
   Val Leu Asn Lys Gly Leu Pro Leu Ile Ile Gly Glu Phe Gly Gly Gln
                   245                 250                 255
   His Thr Asn Gly Asp Val Asp Glu His Ala Ile Met Arg Tyr Gly Gln
   260 265 270
   Gln Lys Gly Val Gly Trp Leu Ala Trp Ser Trp Tyr Gly Asn Asn Ser
   275 280 285
   Glu Leu Ser Tyr Leu Asp Leu Ala Thr Gly Pro Ala Gly Ser Leu Thr
   290 295 300
   Ser Ile Gly Asn Thr Ile Val Asn Asp Pro Tyr Gly Ile Lys Ala Thr
   305 310 315 320
   Ser Lys Lys Ala Gly Ile Phe
   325
<210> 43
   <211> 981
   <212> DNA
   <213> Bacillus circulans
<400> 43
   atggccaagt tgcaaaaggg tacaatctta acagtcattg cagcactgat gtttgtcatt 60
   ttggggagcg cggcgcccaa agccgcagca gctacaggtt tttacgtgaa tggaggcaaa 120
   ttgtacgatt ctacgggtaa accattttac atgaggggta tcaatcatgg gcactcctgg 180
   tttaaaaatg atttgaacac ggctatccct gcgatcgcaa aaacgggtgc caatacggta 240
   cgaattgttt tatcaaacgg tacacaatac accaaggatg atctgaattc cgtaaaaaac 300
   atcattaatg tcgtaaatgc aaacaagatg attgctgtgc ttgaagtaca cgatgccact 360
   gggaaagatg acttcaactc gttggatgca gcggtcaact actggataag catcaaagaa 420
   gcactgatcg ggaaggaaga tcgggttatt gtaaacattg caaacgagtg gtacggaaca 480
   tggaacggaa gcgcgtgggc tgacgggtac aaaaaagcta ttccgaaatt aagagatgcg 540
   ggtattaaaa ataccttgat tgtagatgca gcaggctggg gtcagtaccc tcaatcgatc 600
   gtcgattacg gacaaagcgt attcgccgcg gattcacaga aaaatacggc gttttccatt 660
   cacatgtatg agtatgcagg caaggatgcg gccaccgtca aatccaatat ggaaaatgtg 720
   ctgaataagg ggctggcctt aatcattggt gagttcggag gatatcacac caatggagat 780
   gtcgatgaat atgcaatcat gaaatatggt ctggaaaaag gggtaggatg gcttgcatgg 840
   tcttggtacg gtaatagctc tggattaaac tatcttgatt tggcaacagg acctaacggc 900
   agtttgacga gctatggtaa tacggttgtc aatgatactt acggaattaa aaatacgtcc 960
   caaaaagcgg gaatctttta a 981
<210> 44
   <211> 326
   <212> PRT
   <213> Bacillus circulans
<400> 44
   Met Ala Lys Leu Gln Lys Gly Thr Ile Leu Thr Val Ile Ala Ala Leu
   1               5                   10                  15
   Met Phe Val Ile Leu Gly Ser Ala Ala Pro Lys Ala Ala Ala Ala Thr
               20                  25                  30
   Gly Phe Tyr Val Asn Gly Gly Lys Leu Tyr Asp Ser Thr Gly Lys Pro
           35                  40                  45
   Phe Tyr Met Arg Gly Ile Asn His Gly His Ser Trp Phe Lys Asn Asp
       50                  55                  60
   Leu Asn Thr Ala Ile Pro Ala Ile Ala Lys Thr Gly Ala Asn Thr Val
   65                  70                  75                  80
   Arg Ile Val Leu Ser Asn Gly Thr Gln Tyr Thr Lys Asp Asp Leu Asn
                   85                  90                  95
   Ser Val Lys Asn Ile Ile Asn Val Val Asn Ala Asn Lys Met Ile Ala
               100                 105                 110
   Val Leu Glu Val His Asp Ala Thr Gly Lys Asp Asp Phe Asn Ser Leu
           115                 120                 125
   Asp Ala Ala Val Asn Tyr Trp Ile Ser Ile Lys Glu Ala Leu Ile Gly
       130                 135                 140
   Lys Glu Asp Arg Val Ile Val Asn Ile Ala Asn Glu Trp Tyr Gly Thr
   145                 150                 155                 160
   Trp Asn Gly Ser Ala Trp Ala Asp Gly Tyr Lys Lys Ala Ile Pro Lys
                   165                 170                 175
   Leu Arg Asp Ala Gly Ile Lys Asn Thr Leu Ile Val Asp Ala Ala Gly
               180                 185                 190
   Trp Gly Gln Tyr Pro Gln Ser Ile Val Asp Tyr Gly Gln Ser Val Phe
           195                 200                 205
   Ala Ala Asp Ser Gln Lys Asn Thr Ala Phe Ser Ile His Met Tyr Glu
       210                 215                 220
   Tyr Ala Gly Lys Asp Ala Ala Thr Val Lys Ser Asn Met Glu Asn Val
   225                 230                 235                 240
   Leu Asn Lys Gly Leu Ala Leu Ile Ile Gly Glu Phe Gly Gly Tyr His
                   245                 250                 255
   Thr Asn Gly Asp Val Asp Glu Tyr Ala Ile Met Lys Tyr Gly Leu Glu
               260                 265                 270
   Lys Gly Val Gly Trp Leu Ala Trp Ser Trp Tyr Gly Asn Ser Ser Gly
           275                 280                 285
   Leu Asn Tyr Leu Asp Leu Ala Thr Gly Pro Asn Gly Ser Leu Thr Ser
       290                 295                 300
   Tyr Gly Asn Thr Val Val Asn Asp Thr Tyr Gly Ile Lys Asn Thr Ser
   305                 310                 315                 320
   Gln Lys Ala Gly Ile Phe
                   325
<210> 45
   <211> 1110
   <212> DNA
   <213> Bacillus nealsonii
<400> 45
   atggttgtga aaaaattatc aagttttatt ctaattttac tgttagttac ttctgctttg      60
   tttattactg attcaaaagc aagtgctgct tcgggatttt atgtaagcgg taccacttta     120
   tatgatgcaa cgggtaaacc gtttactatg agaggtgtaa atcatgctca ttcttggttt     180
   aaagaagatt cagcagctgc tattccagca atagcagcaa ctggagcaaa cacagtaaga     240
   attgttttat ctgatggtgg acaatacacc aaagatgata ttaatactgt taaaagcctt     300
   ttgtcattgg cagaaaaaat aaacttgcat tctggagtca tgacgcacag aaaagacgat     360
   gtggaatctt taaatcgtgc agtcgattat tggatcagct taaaagacac attgataggc     420
   aaagaagata aagtgataat aaacattgcg aatgaatggt atggtacttg ggatggtgcg     480
   gcatgggcag ctggttataa acaagctatt ccaaagttac ggaatgcagg cttaaatcat     540
   actctaataa ttgattctgc tggatgggga caatacccag cttccattca taattatgga     600
   aaagaggtat ttaatgcgga tccattgaaa aatacaatgt tctccataca tatgtatgag     660
   tacgctggtg gggatgcagc aactgttaag tcaaatattg atggtgtctt aaaccaagga     720
   ttagctttaa taataggaga gtttggacaa aaacatacaa atggagatgt agatgaagca     780
   accatcatga gttattcaca gcaaaaaaat atcggttggc ttgcatggtc ttggaaagga     840
   aatagcacag attggagcta tctggattta agcaacgatt ggtctggtaa cagtttaact     900
   gattggggta atacggttgt taatggggca aatgggttaa aagccacttc aaaactaagc     960
   ggagtattcg gtagctcagc aggaacaaat aatatattgt atgattttga aagcggtaat    1020
   caaaactgga ctggatcaaa tatcgcgggt ggaccttgga acgaattcaa gcttgatatc    1080
   attcaggacg agcctcagac tccagcgtaa                                     1110
<210> 46
   <211> 369
   <212> PRT
   <213> Bacillus nealsonii
<400> 46
   Met Val Val Lys Lys Leu Ser Ser Phe Ile Leu Ile Leu Leu Leu Val
   1               5                   10                  15
   Thr Ser Ala Leu Phe Ile Thr Asp Ser Lys Ala Ser Ala Ala Ser Gly
               20                  25                  30
   Phe Tyr Val Ser Gly Thr Thr Leu Tyr Asp Ala Thr Gly Lys Pro Phe
           35                  40                  45
   Thr Met Arg Gly Val Asn His Ala His Ser Trp Phe Lys Glu Asp Ser
       50                  55                  60
   Ala Ala Ala Ile Pro Ala Ile Ala Ala Thr Gly Ala Asn Thr Val Arg
   65                  70                  75                  80
   Ile Val Leu Ser Asp Gly Gly Gln Tyr Thr Lys Asp Asp Ile Asn Thr
                   85                  90                  95
   Val Lys Ser Leu Leu Ser Leu Ala Glu Lys Ile Asn Leu His Ser Gly
               100                 105                 110
   Val Met Thr His Arg Lys Asp Asp Val Glu Ser Leu Asn Arg Ala Val
           115                 120                 125
   Asp Tyr Trp Ile Ser Leu Lys Asp Thr Leu Ile Gly Lys Glu Asp Lys
       130                 135                 140
   Val Ile Ile Asn Ile Ala Asn Glu Trp Tyr Gly Thr Trp Asp Gly Ala
   145                 150                 155                 160
   Ala Trp Ala Ala Gly Tyr Lys Gln Ala Ile Pro Lys Leu Arg Asn Ala
                   165                 170                 175
   Gly Leu Asn His Thr Leu Ile Ile Asp Ser Ala Gly Trp Gly Gln Tyr
               180                 185                 190
   Pro Ala Ser Ile His Asn Tyr Gly Lys Glu Val Phe Asn Ala Asp Pro
   195 200 205
   Leu Lys Asn Thr Met Phe Ser Ile His Met Tyr Glu Tyr Ala Gly Gly
   210 215 220
   Asp Ala Ala Thr Val Lys Ser Asn Ile Asp Gly Val Leu Asn Gln Gly
   225 230 235 240
   Leu Ala Leu Ile Ile Gly Glu Phe Gly Gln Lys His Thr Asn Gly Asp
   245 250 255
   Val Asp Glu Ala Thr Ile Met Ser Tyr Ser Gln Gln Lys Asn Ile Gly
   260 265 270
   Trp Leu Ala Trp Ser Trp Lys Gly Asn Ser Thr Asp Trp Ser Tyr Leu
   275 280 285
   Asp Leu Ser Asn Asp Trp Ser Gly Asn Ser Leu Thr Asp Trp Gly Asn
   290 295 300
   Thr Val Val Asn Gly Ala Asn Gly Leu Lys Ala Thr Ser Lys Leu Ser
   305 310 315 320
   Gly Val Phe Gly Ser Ser Ala Gly Thr Asn Asn Ile Leu Tyr Asp Phe
   325 330 335
   Glu Ser Gly Asn Gln Asn Trp Thr Gly Ser Asn Ile Ala Gly Gly Pro
   340 345 350
   Trp Asn Glu Phe Lys Leu Asp Ile Ile Gln Asp Glu Pro Gln Thr Pro
   355 360 365
   Ala
<210> 47
   <211> 984
   <212> DNA
   <213> Bacillus circulans
<400> 47
   atgatgttga tatggatgca gggatggaag tctattctag tcgcgatctt ggcgtgtgtg      60
   tcagtaggcg gtgggcttcc tagtccagaa gcagccacag gattttatgt aaacggtacc     120
   aagctgtatg attcaacggg caaggccttt gtgatgaggg gtgtaaatca tccccacacc     180
   tggtacaaga atgatctgaa cgcggctatt ccggctatcg cgcaaacggg agccaatacc     240
   gtacgagtcg tcttgtcgaa cgggtcgcaa tggaccaagg atgacctgaa ctccgtcaac     300
   agtatcatct cgctggtgtc gcagcatcaa atgatagccg ttctggaggt gcatgatgcg     360
   acaggcaaag atgagtatgc ttcccttgaa gcggccgtcg actattggat cagcatcaaa     420
   ggggcattga tcggaaaaga agaccgcgtc atcgtcaata ttgctaatga atggtatgga     480
   aattggaaca gcagcggatg ggccgatggt tataagcagg ccattcccaa attaagaaac     540
   gcgggcatta agaatacgtt gatcgttgat gcagcgggat gggggcaata cccgcaatcc     600
   atcgtggatg agggggccgc ggtatttgct tccgatcaac tgaagaatac ggtattctcc     660
   atccatatgt atgagtatgc cggtaaggat gccgctacgg tgaaaacgaa tatggacgat     720
   gttttaaaca aaggattgcc tttaatcatt ggggagttcg gcggctatca tcaaggtgcc     780
   gatgtcgatg agattgctat tatgaagtac ggacagcaga aggaagtggg ctggctggct     840
   tggtcctggt acggaaacag cccggagctg aacgatttgg atctggctgc agggccaagc     900
   ggaaacctga ccggctgggg aaacacggtg gttcatggaa ccgacgggat tcagcaaacc     960
   tccaagaaag cgggcattta ttaa                                            984
<210> 48
   <211> 327
   <212> PRT
   <213> Bacillus circulans
<400> 48
   Met Met Leu Ile Trp Met Gln Gly Trp Lys Ser Ile Leu Val Ala Ile
   1               5                   10                  15
   Leu Ala Cys Val Ser Val Gly Gly Gly Leu Pro Ser Pro Glu Ala Ala
               20                  25                  30
   Thr Gly Phe Tyr Val Asn Gly Thr Lys Leu Tyr Asp Ser Thr Gly Lys
           35                  40                  45
   Ala Phe Val Met Arg Gly Val Asn His Pro His Thr Trp Tyr Lys Asn
       50                  55                  60
   Asp Leu Asn Ala Ala Ile Pro Ala Ile Ala Gln Thr Gly Ala Asn Thr
   65                  70                  75                  80
   Val Arg Val Val Leu Ser Asn Gly Ser Gln Trp Thr Lys Asp Asp Leu
                   85                  90                  95
   Asn Ser Val Asn Ser Ile Ile Ser Leu Val Ser Gln His Gln Met Ile
               100                 105                 110
   Ala Val Leu Glu Val His Asp Ala Thr Gly Lys Asp Glu Tyr Ala Ser
           115                 120                 125
   Leu Glu Ala Ala Val Asp Tyr Trp Ile Ser Ile Lys Gly Ala Leu Ile
       130                 135                 140
   Gly Lys Glu Asp Arg Val Ile Val Asn Ile Ala Asn Glu Trp Tyr Gly
   145                 150                 155                 160
   Asn Trp Asn Ser Ser Gly Trp Ala Asp Gly Tyr Lys Gln Ala Ile Pro
                   165                 170                 175
   Lys Leu Arg Asn Ala Gly Ile Lys Asn Thr Leu Ile Val Asp Ala Ala
               180                 185                 190
   Gly Trp Gly Gln Tyr Pro Gln Ser Ile Val Asp Glu Gly Ala Ala Val
           195                 200                 205
   Phe Ala Ser Asp Gln Leu Lys Asn Thr Val Phe Ser Ile His Met Tyr
       210                 215                 220
   Glu Tyr Ala Gly Lys Asp Ala Ala Thr Val Lys Thr Asn Met Asp Asp
   225                 230                 235                 240
   Val Leu Asn Lys Gly Leu Pro Leu Ile Ile Gly Glu Phe Gly Gly Tyr
                   245                 250                 255
   His Gln Gly Ala Asp Val Asp Glu Ile Ala Ile Met Lys Tyr Gly Gln
               260                 265                 270
   Gln Lys Glu Val Gly Trp Leu Ala Trp Ser Trp Tyr Gly Asn Ser Pro
           275                 280                 285
   Glu Leu Asn Asp Leu Asp Leu Ala Ala Gly Pro Ser Gly Asn Leu Thr
       290                 295                 300
   Gly Trp Gly Asn Thr Val Val His Gly Thr Asp Gly Ile Gln Gln Thr
   305                 310                 315                 320
   Ser Lys Lys Ala Gly Ile Tyr
                   325

## Description

### FIELD OF THE INVENTION

This invention relates to bacterial mannanase enzymes. The mannanases are useful in industrial applications wherein degradation or modification of mannan is desired, such as in laundry and cleaning applications, in feed, food, pulp and oil industry. The invention also provides useful mannanases enzymes, polynucleotides encoding these enzymes, enzyme compositions and methods for their production and use.

### BACKGROUND

Mannans are mannose containing polysaccharides found in various plants. Mannans are poorly soluble in an aqueous environment and their physicochemical properties give rise to viscous dispersions. Additionally, mannans have high water binding capacity. All of these characteristics cause problems in several industries including brewing, baking, animal nutrition, and laundry and cleaning applications.

In plant-based diets different β-mannans are present and depending on their amounts and properties they can compromise nutrient digestion, microbial colonisation and growth performance. Enzymatic degradation of mannans reduces digesta viscosity of high water soluble mannans and leads to production of manno-oligosaccharides that may form water-insoluble linear mannans present in leguminoseae. Mannanase increases average daily gain, feed efficiency, weight uniformity and livability in all monogastric animals.

For animal feed applications, such as feed for monogastric animals with cereal diets, mannan is a contributing factor to viscosity of gut contents and it thereby adversely affects the feed digestibility and animal growth rate. For ruminants, mannan represents a substantial component of fiber intake and a more complete digestion of mannan would facilitate higher feed conversion efficiencies.

For laundry and cleaning applications enzyme compositions comprising mannanase can be used to degrade mannan. However, providing mannanases that are stable in varying storage and use conditions while still showing good mannan degrading activity is difficult.

It is an object of the present invention to provide novel enzymes exhibiting mannanase activity when applied in different industrial processes, as well as enzyme compositions for mannan degradation or modification.

### SUMMARY

According to the first aspect of the invention there is provided an enzyme composition comprising at least one mannanase enzyme having an amino acid sequence which has at least 80% sequence identity with SEQ ID NO: 16 (Man7).

The present enzyme composition is advantageous in having good stability and mannanase activity in detergents and in formulations. It is also suitable for various industrial applications wherein mannan degradation or modification is desired. The mannanases of the enzyme composition of the invention are suitable for degrading and modifying mannan containing material in various chemical environments.

As evidenced by the Examples, the mannanases comprised in the enzyme composition according to the invention have a structure and properties that allow production in recombinant host cells and make them useful in enzyme compositions for industrial applications. A common structural element shared by Man6, Man7 and Man14 is the GH5 domain. Another common structural element is a sequence identity of 60% between Man6 and Man7, a sequence identity of 57% between Man6 and Man14 and sequence identity of 69% between Man7 and Man14. Another common structural characteristic is the core region. These structural elements are characteristic for the mannanases.

According to the second aspect there is provided a recombinant host cell comprising genetic elements that allow producing at least one recombinant polypeptide having mannanase activity and at least 80% sequence identity with the amino acid sequence of SEQ ID NO: 16, and wherein the host cell is selected from the group consisting of:
fungal cells,
filamentous fungal cells from Division *Ascomycota,* Subdivision *Pezizomycotina;* preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola;*
more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium;*
more preferably from the group consisting of *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,*
bacterial cells, preferably gram positive Bacilli such as B. *subtilis, B. licheniformis, B. megaterium, B. amyloliquefaciens, B. pumilus,* gram negative bacteria such as *Escherichia coli,* actinomycetales such as *Streptomyces* sp., and
yeasts, such as *Saccharomyces cerevisiae, Pichia pastoris, Yarrowia lipolytica,* most preferably *Trichoderma reesei* or *Bacillus.*

The recombinant host cell can be used to produce mannanase and to carry the polynucleotide encoding mannanase. The recombinant host cell is useful also in preparation of mannanases with different properties. For example, a host cell can be selected, which provides post-translational modifications beneficial for stability or activity, or which facilitates post-processing and formulation of mannanase produced in the host cell.

According to the third aspect is provided a recombinant polypeptide having mannanase activity and obtainable by using the host cell of the second aspect.

The recombinant polypeptide may have structural or functional properties that differentiate it from a native polypeptide having the same or similar amino acid sequence. For example, a host cell can be selected which provides the produced recombinant polypeptide with post-translational modifications, a lack thereof, or localization to facilitate production and/or formulation of the recombinant polypeptide.

According to the fourth aspect is provided a method for producing mannanase comprising:
a. cultivating a recombinant host cell of the second aspect, wherein
   i. the genetic elements comprise at least one control sequence which controls the production of the recombinant polypeptide in the recombinant host cell under conditions that allow production of the polypeptide;
   ii. the genetic elements optionally comprise at least one sequence encoding a signal sequence for transporting the polypeptide outside the host cell; and
   iii. cultivating is carried out in conditions allowing production of the polypeptide; and
b. recovering the polypeptide.

The method provides an efficient way to produce mannanase. Because the mannanase is produced in a recombinant host cell, a mannanase production system is provided which can be optimized, tailored, and controlled in a desired manner. The mannanase produced by the method may differ from natural mannanases at a structural level. The mannanase produced by the method can e.g. have a glycosylation pattern, or other post translational modification, which causes differences in the structure and/or function when compared to a natural mannanase, such as a mannanase having similar or the same amino acid sequence, or compared to a mannanase having the same amino acid sequence but produced in another host cell. The mannanase produced by the method can be used as such or formulated into a selected formulation.

According to another aspect is provided an enzyme preparation comprising a recombinant polypeptide having mannanase activity and obtainable by using the host cell of the second aspect.

The enzyme preparation or composition may further comprise other enzyme(s) selected from the group consisting of proteases, amylases, cellulases, lipases, xylanases, mannanases, cutinases, esterases, phytases, DNAses, pectinases, pectinolytic enzymes, xanthanases, xyloglucanases, laccases, peroxidases and oxidases with or without a mediator, as well as suitable additives selected from the group consisting of stabilizers, buffers, surfactants, bleaching agents, mediators, anti-corrosion agents, builders, anti-redeposition agents, optical brighteners, dyes, pigments, perfumes, caustics, abrasives and preservatives.

According to a fifth aspect is provided a method for degrading or modifying mannan containing material comprising treating said mannan containing material with an effective amount of the present enzyme composition or the recombinant polypeptide.

According to a sixth aspect is provided an animal feed comprising the present enzyme composition or the recombinant host cell, and at least one protein source of plant origin or a mannan containing product or by-product, and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

According to a seventh aspect is provided a feed supplement comprising the present enzyme composition or the enzyme obtainable from host cell; and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

The feed and the feed supplement improve nutritional value of feed compared to a feed without mannanase. The present enzyme composition degrades mannan present in the feed and thereby makes it more easily digestible for the animal. In particular for soybean meal containing feeds mannan-oligosaccharides that result from enzymatic digestion have a beneficial effects on the intestinal microbes, and consequently on the performance of the animals. The effect of mannanases can be enhanced by including xylanase to digest arabinoxylans present in corn soybean based diets. Mannanase can also be used to modify rheological properties of wet feeds.

In an embodiment the feed may comprise animal protein, such as meat meal or bone meal.

According to a eighth aspect is provided a use, and a method of using, the animal feed of the sixth aspect or the feed supplement of the seventh aspect in:
a. feeding animals, preferably monogastric animals and ruminants;
b. improving weight gain of animals.

According to an ninth aspect is provided a use of, and a method of using, the present enzyme composition or the enzyme obtainable from the host cell in a detergent.

In one embodiment of the present invention the detergent composition further comprises one or more additional enzymes selected from the group consisting of protease, lipase, cutinase, amylase, carbohydrase, cellulase, pectinase, pectatelyase, pectinolytic enzyme, esterase, mannanase, arabinase, galactanase, xylanase, oxidase, xanthanase, xyloglucanase, laccase, DNAse and/or peroxidase, preferably selected from the group consisting of proteases, amylases, cellulases and lipases.

In a further embodiment of the present invention the detergent composition is in a form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition.

The present invention furthermore relates to the use of the enzyme composition or the detergent composition as herein disclosed for degrading mannan.

In a further embodiment the present invention relates to the use of the enzyme composition or the detergent composition as herein disclosed in a laundry process.

The present invention furthermore relates to a method for removing a stain from a surface, comprising contacting the surface with the enzyme composition or the detergent composition as herein disclosed.

The present invention also relates to a method for degrading mannan comprising applying the enzyme composition or the detergent composition as herein disclosed to mannan, preferably wherein the mannan is on a surface of a textile, or at least partially embedded in a textile.

According to a tenth aspect is provided a use of, and a method of using, the present enzyme composition of the first aspect or the enzyme obtainable from the host cell of the third aspect in oil drilling.

The present enzyme composition is advantageous in modifying rheological properties of oil drilling fluids and to improve oil recovery.

According to an eleventh aspect is provided a use of, and a method of using, the present enzyme composition of the first aspect or the enzyme obtainable from the host cell of the third aspect in processing coffee extract, fruit juice, pineapple juice, or soya milk.

Using the present enzyme composition or the enzyme obtainable from the host cell is advantageous in processing coffee extract because it reduces viscosity of the coffee extract.

Using the present enzyme composition or the enzyme obtainable from the host cell is advantageous in processing and manufacturing fruit juice because it lowers viscosity and improves filtration rate, stability and helps to extract fruit components.

Using the present enzyme composition or the enzyme obtainable from the host cell is advantageous in processing and manufacturing soya milk because it improves yield, colour, protein content and taste of soya milk.

In another aspect the disclosed sequence information herein relating to a polynucleotide sequence encoding a mannanase of the invention can be used as a tool to identify other homologous mannanases. For instance, polymerase chain reaction (PCR) can be used to amplify sequences encoding other homologous mannanases from a variety of biological sources. In addition, genome mining approaches can be used to identify sequences encoding other homologous mannanases from genome databases.

### BRIEF DESCRIPTION OF THE FIGURES

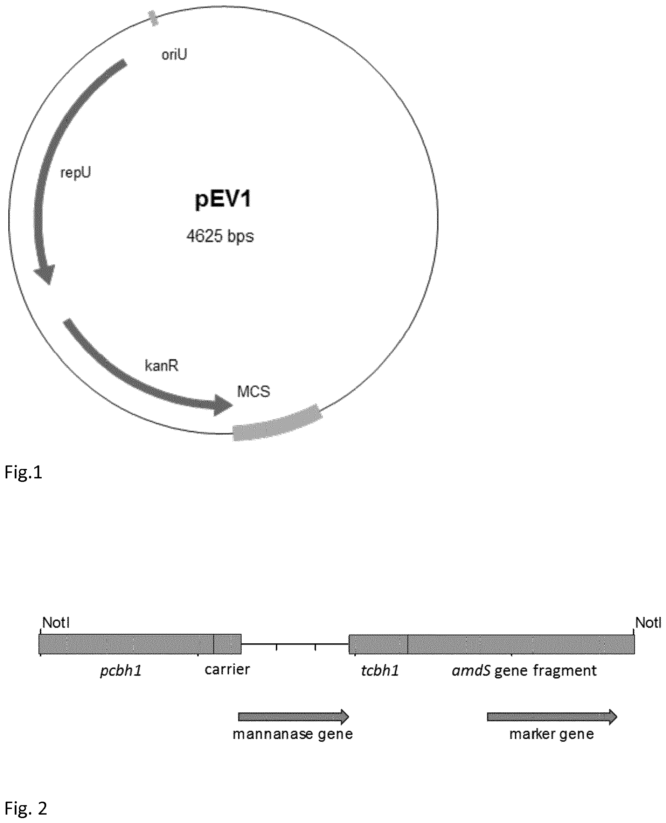

**Fig 1** shows schematic representation of vector pEV1 for replication in *Bacillus.*
**Fig 2** schematically shows the expression cassettes used in the transformation of *Trichoderma reesei* protoplasts for overproducing the recombinant mannanase proteins (Man6, Man7 and Man14). The mannanase genes were under the control of *T. reesei cel7A*/*cbh1* promoter *(pcbh1)* and the termination of the transcription was ensured by using *T. reesei cel7A*/*cbh1* terminator sequence *(tcbh1).* The *amdS* gene was included as a transformation marker.

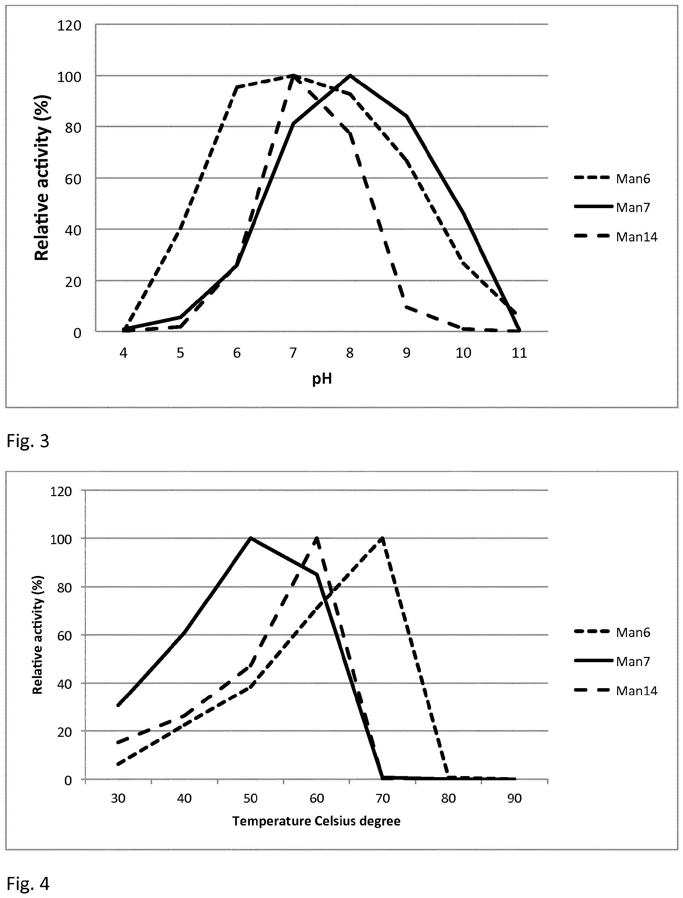

**Fig 3** describes the effect of pH on the activity of recombinant Man6, Man7 and Man14 *(Bacillus* produced) mannanase proteins in 40 mM Britton-Robinson buffer at pH 4 to pH 11. Reaction temperature was 50°C and the reaction time was 10 min. Azurine-crosslinked carob galactomannan was used as a substrate. All measurements were made at least duplicates. The data points are averages of separate measurements.
**Fig 4** shows the temperature profile of recombinant Man6, Man7 and Man14 *(Bacillus* produced) mannanases assayed in 40 mM Britton-Robinson buffer pH 7 using 10 min reaction time, Azurine-crosslinked carob galactomannan was used as a substrate. All measurements were made at least duplicates. The data points are averages of separate measurements.

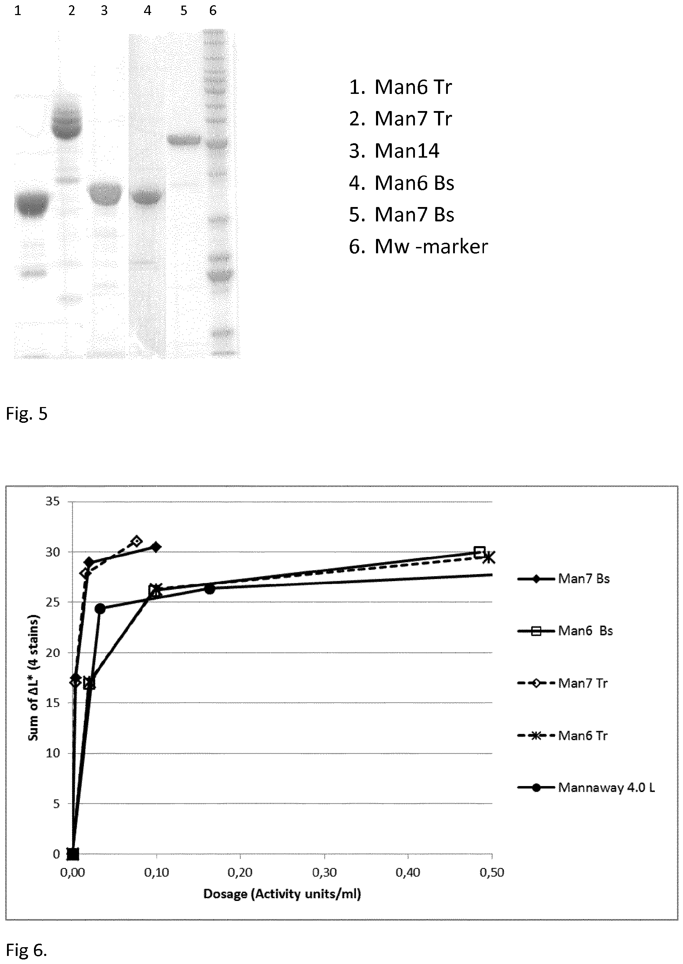

**Fig 5** shows SDS PAGE analysis of bacterial mannanases.
**Fig 6** describes the stain removal performance of Man6 and Man7 (produced in *Bacillus* and *Trichoderma)* as an increase of lightness (sum of ΔL*of 4 stains) in the presence of 4,4 g/l of Commercial heavy duty liquid detergent A at 40°C, 16 °dH, 60 min, pH approx. 8.3 and enzymes dosed as activity units. Commercial preparation Mannaway^{®}4,0 L was used for comparison.

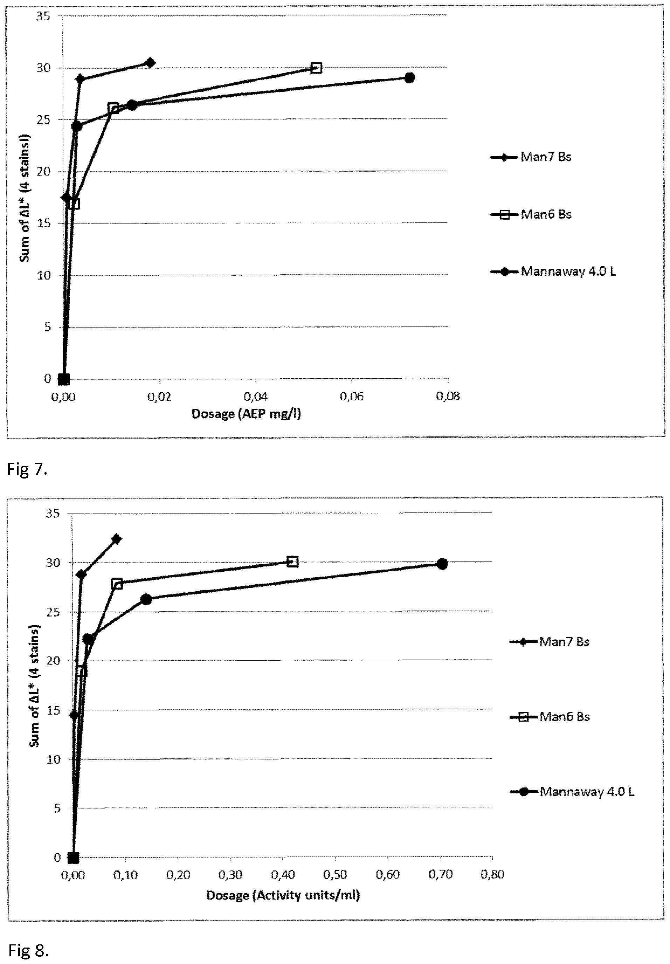

**Fig 7** describes the stain removal performance of Man6 and Man7 (produced in *Bacillus)* as an increase of lightness (sum of ΔL*of 4 stains) in the presence of 4.4 g/l of Commercial heavy duty liquid detergent A at 40°C, 16 °dH, 60 min, pH approx. 8.3 and enzymes dosed as active enzyme protein (AEP). Commercial preparation Mannaway^{®}4,0 L was used for comparison.
**Fig 8** describes the stain removal performance of Man6 and Man7 (produced in *Bacillus)* as an increase of lightness (sum of ΔL*of 4 stains) in the presence of 3.8 g/l of Commercial color detergent powder at 40°C, 16 °dH, 60 min, pH approx. 10 and enzymes dosed as activity units. Commercial preparation Mannaway^{®} 4,0 L was used for comparison.

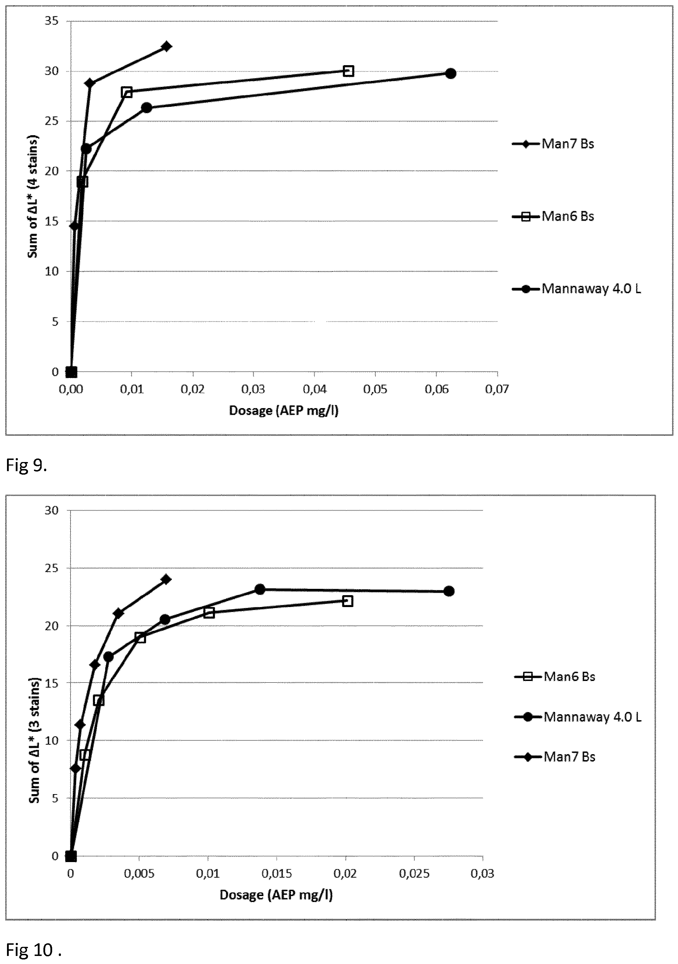

**Fig 9** describes the stain removal performance of Man6 and Man7 (produced in *Bacillus)* as an increase of lightness (sum of ΔL*of 4 stains) in the presence of 3.8 g/l of Commercial color detergent powder at 40°C, 16 °dH, 60 min, pH approx. 10 and enzymes dosed as active enzyme protein. Commercial preparation Mannaway^{®}4,0 L was used for comparison.
**Fig 10** describes the stain removal performance of Man6 and Man7 (produced in *Bacillus)* as an increase of lightness (sum of ΔL* of 3 stains) in the presence of 4,2 g/l of Commercial bleach detergent powder at 40°C, 16 °dH, 60 min, pH approximately 9.5 and enzymes dosed as active enzyme protein. Commercial preparation Mannaway^{®}4,0 L was used for comparison.

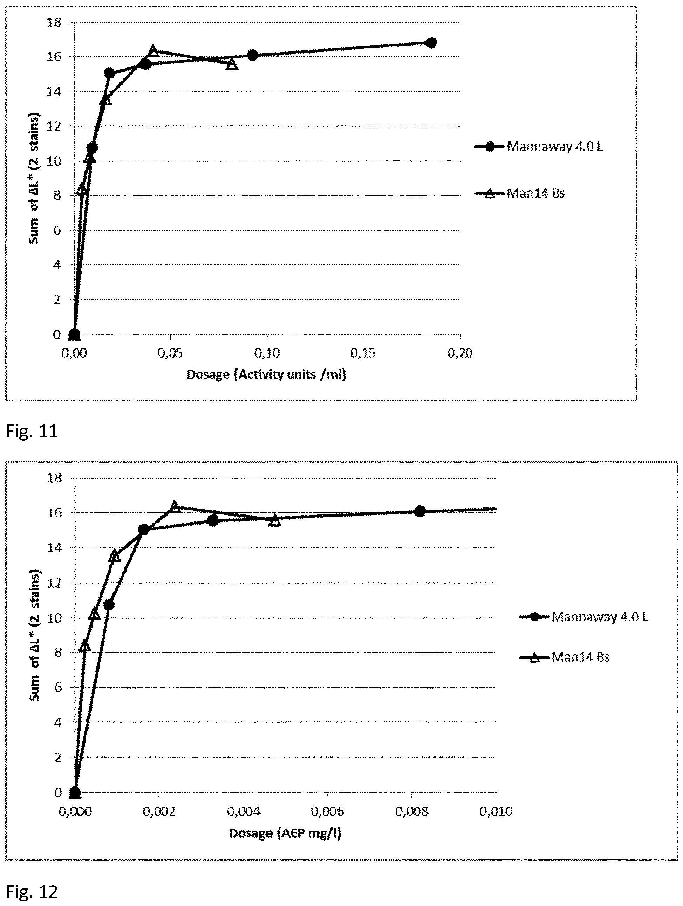

**Fig 11** describes the stain removal performance of Man14 (produced in *Bacillus)* as an increase of lightness (sum of ΔL*of 2 stains) in the presence of 5 g/l of Commercial heavy duty liquid detergent B at 40°C, 16 °dH, 60 min, pH approximately 8.3 and enzymes dosed as activity units. Commercial preparation Mannaway^{®}4,0 L was used for comparison.
**Fig 12** describes the stain removal performance of Man14 (produced in *Bacillus)* as an increase of lightness (sum of ΔL*of 2 stains) in the presence of 5 g/l of Commercial heavy duty liquid detergent B at 40°C, 16 °dH, 60 min, pH approximately 8.3 and enzymes dosed as active enzyme protein. Commercial preparation Mannaway^{®}4,0 L was used for comparison.

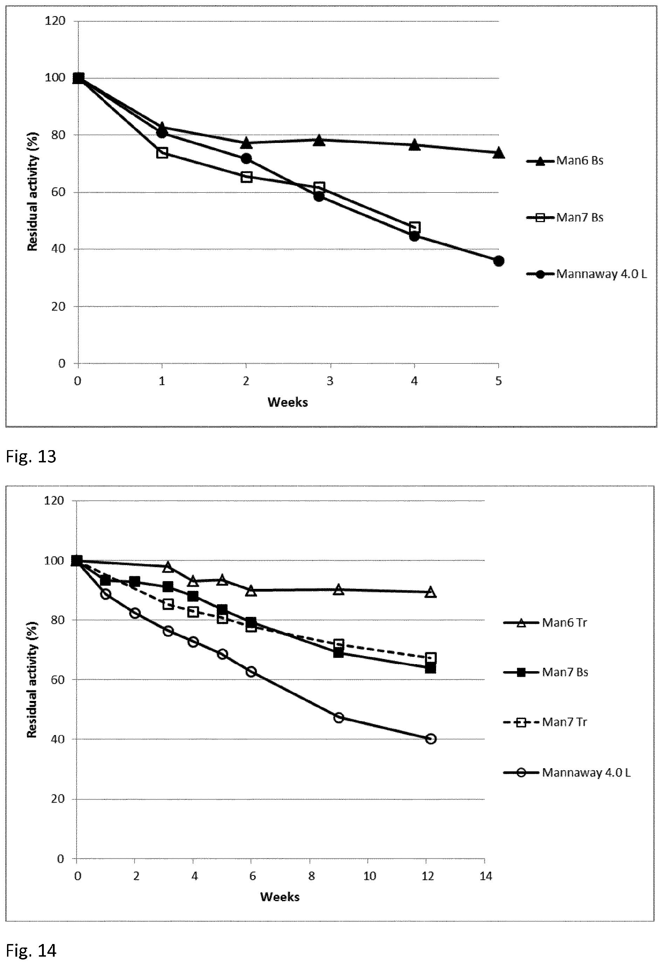

**Fig 13** describes the stability of Man6 and Man7 (produced in *Bacillus)* in liquid detergent (OMO Color) at 37°C. Commercial preparation Mannaway^{®} 4,0 L was used for comparison
**Fig 14** describes the stability of Man7 (produced both in *Bacillus* and *Trichoderma)* and Man6 (produced in Bacillus) in Commercial heavy duty liquid detergent A. Commercial preparation Mannaway^{®} 4,0 L was used for comparison.

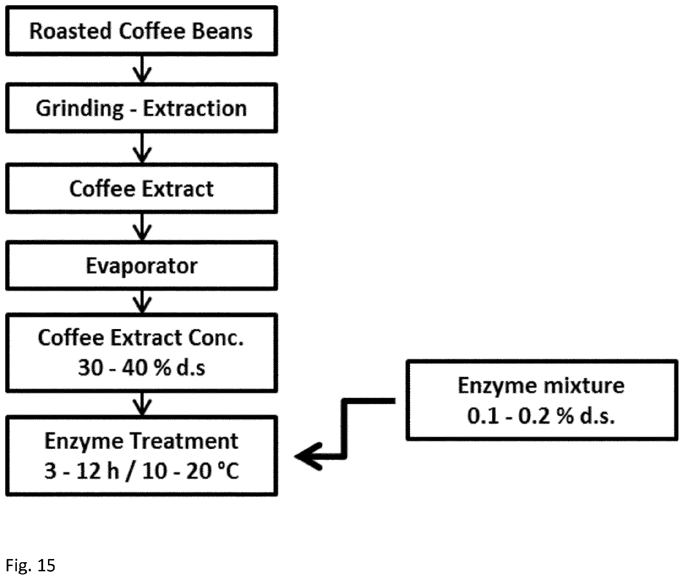

**Fig 15** shows a flow chart of instant coffee production involving use of the mannanase of the invention.

### SEQUENCE LISTINGS

SEQ ID NO: 1 Sequence of the oligonucleotide primer Man6_1
SEQ ID NO: 2 Sequence of the oligonucleotide primer Man6_2
SEQ ID NO: 3 Sequence of the oligonucleotide primer Man7_1
SEQ ID NO: 4 Sequence of the oligonucleotide primer Man7_2
SEQ ID NO: 5 Sequence of the oligonucleotide primer Man14_1
SEQ ID NO: 6 Sequence of the oligonucleotide primer Man14_2
SEQ ID NO: 7 Sequence of the oligonucleotide primer Vec_1
SEQ ID NO: 8 Sequence of the oligonucleotide primer Vec_2
SEQ ID NO: 9 The nucleotide sequence of the *Bacillus clausii man6*
SEQ ID NO: 10 The nucleotide sequence of the *Bacillus clausii man6* without signal peptide encoding sequence and with codon optimization to *Trichoderma reesei*
SEQ ID NO: 11 The deduced amino acid sequence of the *Bacillus clausii* Man6
SEQ ID NO: 12 The deduced amino acid sequence of the *Bacillus clausii* Man6 without signal peptide
SEQ ID NO: 13 The nucleotide sequence of the *Bacillus hemicellulosilyticus man*7
SEQ ID NO: 14 The nucleotide sequence of the *Bacillus hemicellulosilyticus man7* without signal peptide encoding sequence and with codon optimization to *Trichoderma reesei*
SEQ ID NO: 15 The deduced amino acid sequence of the *Bacillus hemicellulosilyticus* Man7
SEQ ID NO: 16 The deduced amino acid sequence of the *Bacillus hemicellulosilyticus* Man7 without signal peptide
SEQ ID NO: 17 The nucleotide sequence of the *Virgibacillus soli man*14
SEQ ID NO: 18 The nucleotide sequence of the *Virgibacillus soli man*14 without signal peptide encoding sequence and with codon optimization to *Trichoderma reesei*
SEQ ID NO: 19 The deduced amino acid sequence of the *Virgibacillus soli* Man14
SEQ ID NO: 20 The deduced amino acid sequence of the *Virgibacillus soli* Man14 without signal peptide
SEQ ID NO: 21 Sequence of the oligonucleotide primer BMAN1
SEQ ID NO: 22 Sequence of the oligonucleotide primer BMAN2
SEQ ID NO: 23 Sequence of the oligonucleotide primer BMAN3
SEQ ID NO: 24 Sequence of the oligonucleotide primer BMAN4
SEQ ID NO: 25 The nucleotide sequence of *Bacillus pumilus man*31
SEQ ID NO: 26 The deduced amino acid sequence of the *Bacillus pumilus* Man31
SEQ ID NO: 27 The nucleotide sequence of the *Bacillus amyloliquefaciens man*32
SEQ ID NO: 28 The deduced amino acid sequence of the *Bacillus amyloliquefaciens* Man32
SEQ ID NO: 29 The nucleotide sequence of the *Amphibacillus xylanus man*33
SEQ ID NO: 30 The deduced amino acid sequence of the *Amphibacillus xylans* Man33
SEQ ID NO: 31 The nucleotide sequence of the *Paenibacillus polymyxa man*34
SEQ ID NO: 32 The deduced amino acid sequence of the *Paenibacillus polymyxa* Man34
SEQ ID NO: 33 The nucleotide sequence of the *Bacillus hemicellulosilyticus man35*
SEQ ID NO: 34 The deduced amino acid sequence of the *Bacillus hemicellulosilyticus* Man35
SEQ ID NO: 35 The nucleotide sequence of the *Bacillus alcalophilus man*36
SEQ ID NO: 36 The deduced amino acid sequence of the *Bacillus alcalophilus* Man36
SEQ ID NO: 37 The nucleotide sequence of the *Bacillus* sp. *man*37
SEQ ID NO: 38 The deduced amino acid sequence of the *Bacillus* sp. Man37
SEQ ID NO: 39 The nucleotide sequence of the *Bacillus circulans man*38
SEQ ID NO: 40 The deduced amino acid sequence of the *Bacillus circulans* Man38
SEQ ID NO: 41 The nucleotide sequence of the *Paenibacillus* sp. *man*39
SEQ ID NO: 42 The deduced amino acid sequence of the *Paenibacillus* sp. Man39
SEQ ID NO: 43 The nucleotide sequence of the *Bacillus circulans man*40
SEQ ID NO: 44 The deduced amino acid sequence of the *Bacillus circulans* Man40
SEQ ID NO: 45 The nucleotide sequence of the *Bacillus nealsonii man*41
SEQ ID NO: 46 The deduced amino acid sequence of the *Bacillus nealsonii* Man41
SEQ ID NO: 47 The nucleotide sequence of the *Bacillus circulans man*42
SEQ ID NO: 48 The nucleotide sequence of the *Bacillus circulans* Man42

### DETAILED DESCRIPTION

Mannan refers to polysaccharides consisting of a mannose backbone linked together by β-1,4-linkages with side-chains of galactose attached to the backbone by α-1,6-linkages. Mannans comprise plant-based material such as guar gum and locust bean gum. Glucomannans are polysaccharides having a backbone of more or less regularly alternating β-1,4 linked mannose and glucose, galactomannans and galactoglucomannans are mannans and glucomannans with alpha-1,6 linked galactose side branches.

As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78.

As used herein, "isolated" means a substance in a form or environment that does not occur in nature. Non-limiting examples of isolated substances include (1) any non-naturally occurring substance, (2) any substance including any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing or decreasing the amount of the substance relative to other components with which it is naturally associated (e.g., recombinant production in a host cell; one or multiple copies of a gene encoding the substance; and use of an alternative promoter to the promoter naturally associated with the gene encoding the substance). In an embodiment a polypeptide, enzyme, polynucleotide, host cell or composition of the invention is isolated.

As used herein, the term "comprising" includes the broader meanings of "including", "containing", and "comprehending", as well as the narrower expressions "consisting of' and "consisting only of".

As used herein, "fragment" means a protein or a polynucleotide having one or more amino acids or nucleotides deleted. In the context of DNA, a fragment includes both single-stranded and double-stranded DNA of any length. A fragment may be an active fragment, which has the biological function, such as enzyme activity or regulatory activity, of the protein or the polynucleotide. A fragment may also be an inactive fragment, i.e. it does not have one or more biological effects of the native protein or polynucleotide.

As used herein, a "peptide" and a "polypeptide" are amino acid sequences including a plurality of consecutive polymerized amino acid residues. For purpose of this invention, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

The term "polynucleotide" denotes a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules.

As used herein, "modification", "modified", and similar terms in the context of polynucleotides refer to modification in a coding or a non-coding region of the polynucleotide, such as a regulatory sequence, 5' untranslated region, 3' untranslated region, up-regulating genetic element, down-regulating genetic element, enhancer, suppressor, promoter, exon, or intron region. The modification may in some embodiments be only structural, having no effect on the biological effect, action or function of the polynucleotide. In other embodiments the modification is a structural modification, which provides a change in the biological effect, action or function of the polynucleotide. Such a modification may enhance, suppress or change the biological function of the polynucleotide.

As used herein, "identity" means the percentage of exact matches of amino acid residues between two aligned sequences over the number of positions where there are residues present in both sequences. When one sequence has a residue with no corresponding residue in the other sequence, the alignment program allows a gap in the alignment, and that position is not counted in the denominator of the identity calculation. Identity is a value determined with the Pairwise Sequence Alignment tool EMBOSS Needle at the EMBL-EBI website (www.ebi.ac.uk/Tools/psa/emboss_needle/).

As used herein, "host cell" means any cell type that is susceptible to transformation, transfection, transduction, mating, crossing or the like with a nucleic acid construct or expression vector comprising a polynucleotide. The term "host cell" encompasses any progeny that is not identical due to mutations that occur during replication. Non-limiting examples of a host cell are fungal cells, filamentous fungal cells from Division *Ascomycota,* Subdivision *Pezizomycotina;* preferably from the group consisting of members of the Class *Sordariomycetes,* Subclass *Hypocreomycetidae,* Orders *Hypocreales* and *Microascales* and *Aspergillus, Chrysosporium, Myceliophthora and Humicola;* more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium;* more preferably from the group consisting of *Trichoderma reesei (Hypocrea jecorina), T. citrinoviridae, T. longibrachiatum, T. virens, T. harzianum, T. asperellum, T. atroviridae, T. parareesei,* , *Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,* most preferably *Trichoderma reesei.* Non-limiting examples of a host cell are bacterial cells, preferably gram positive Bacilli (e.g. *Bacillus subtilis, B. licheniformis, B. megaterium, B. amyloliquefaciens, B. pumilus),* gram-negative bacteria (e.g. *Escherichia coli),* actinomycetales (e.g. *Streptomyces* sp.) and yeasts (e.g. *Saccharomyces cerevisiae, Pichia pastoris, Yarrowia lipolytica).*

In an embodiment the host cell is a fungal cell, preferably a filamentous fungal cell, such as *Trichoderma* or *Trichoderma reesei.* In an embodiment the host cell is a bacterial cell, preferably a gram positive *Bacillus* cell, such as *B*. *subtilis, B. licheniformis, B. megaterium, B. amyloliquefaciens, B. pumilus.*

A "recombinant cell" or "recombinant host cell" refers to a cell or host cell, which has been genetically modified or altered to comprise a nucleic acid sequence which is not native to said cell or host cell. In an embodiment the genetic modification comprises integrating the polynucleotide in the genome of the host cell. In another embodiment the polynucleotide is exogenous in the host cell.

As used herein, "expression" includes any step involved in the production of a polypeptide in a host cell including, but not limited to, transcription, translation, post-translational modification, and secretion. Expression may be followed by harvesting, i.e. recovering, the host cells or the expressed product.

The term "expression vector" denotes a DNA molecule, linear or circular, that comprises a segment encoding a polypeptide of interest operably linked to additional segments that provide for its transcription. Such additional segments may include promoter and terminator sequences, and may optionally include one or more origins of replication, one or more selectable markers, an enhancer, a polyadenylation signal, carrier and the like. Expression vectors are generally derived from plasmid or viral DNA, or may contain elements of both. The expression vector may be any expression vector that is conveniently subjected to recombinant DNA procedures, and the choice of vector will often depend on the host cell into which the vector is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The term "recombinant produced" or "recombinantly produced" used herein in connection with production of a polypeptide or protein is defined according to the standard definition in the art.

The term "obtained from" and "obtainable" as used herein in connection with a specific microbial source means that the polynucleotide is expressed by the specific source (homologous expression), or by a cell in which a gene from the source has been inserted (heterologous expression).

The term "enzyme composition" means either a conventional enzymatic fermentation product, possibly isolated and purified, from a single species of a microorganism, such preparation usually comprising a number of different enzymatic activities; or a mixture of monocomponent enzymes, preferably enzymes derived from bacterial or fungal species by using conventional recombinant techniques, which enzymes have been fermented and possibly isolated and purified separately and which may originate from different species, preferably fungal or bacterial species or the fermentation product of a microorganism which acts as a host cell for production of a recombinant mannanase, but which microorganism simultaneously produces other enzymes.

The term "operably linked", when referring to DNA segments, denotes that the segments are arranged so that they function in concert for their intended purposes, e.g. transcription initiates in the promoter and proceeds through the coding segment to the terminator

The term "promoter" denotes a portion of a gene containing DNA sequences that provide for the binding of RNA polymerase and initiation of transcription. Promoter sequences are commonly, but not always, found in the 5' non-coding regions of genes.

The term "secretory signal sequence" denotes a DNA sequence that encodes a polypeptide (a "secretory peptide") that, as a component of a larger polypeptide, directs the larger polypeptide through a secretory pathway of a host cell in which it is produced. The secretory signal sequence can be native or it can be replaced with secretory signal sequence or carrier sequence from another source. Depending on the host cell, the larger peptide may be cleaved to remove the secretory peptide during transit through the secretory pathway.

The term "core region" denotes a domain of an enzyme, which may or may not have been modified or altered, but which has retained at least part of its original activity; the catalytic domain as known in the art has remained functional. The core region of a mannanase according to the invention corresponds to the amino acids aligned with the amino acids 27-331 of Man7, SEQ ID NO: 16. Core regions of other mannanases described herein are amino acids 35-324 of Man6, SEQ ID NO: 12, or amino acids 17-314 of Man14, SEQ ID NO: 20.

By the term "linker" or "spacer" is meant a polypeptide comprising at least two amino acids which may be present between the domains of a multidomain protein, for example an enzyme comprising an enzyme core and a binding domain such as a carbohydrate binding module (CBM) or any other enzyme hybrid, or between two proteins or polypeptides produced as a fusion polypeptide, for example a fusion protein comprising two core enzymes. For example, the fusion protein of an enzyme core with a CBM is provided by fusing a DNA sequence encoding the enzyme core, a DNA sequence encoding the linker and a DNA sequence encoding the CBM sequentially into one open reading frame and expressing this construct.

Efficient amount means an amount, which is sufficient to degrade mannose in the selected application.

The terms "detergent composition" and "detergent" include, unless otherwise indicated, solid, granular or powder-form all-purpose or heavy-duty washing agents, especially cleaning detergents; liquid, gel or paste-form all-purpose washing agents, especially the so- called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners; metal cleaners; as well as cleaning auxiliaries such as bleach additives and "stain-stick" or pre-treat types. The terms "detergent", "detergent composition" and "detergent formulation" are used in reference to mixtures, which are intended for use in a wash medium for the cleaning of soiled objects. In some embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents"). It is not intended that the present invention be limited to any particular detergent formulation or composition. It is intended that in addition to the mannanases according to the invention, the term encompasses detergents that may contain e.g., surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, hydrotropes, fabric hueing agents, dispersants, dye transfer inhibiting agents, fluorescent whitening agents, soil release polymers, anti-redepositions agents, anti-shrink agents, anti-wrinkling agents, bactericides, binders, carriers, dyes, enzyme stabilizers, fabric softeners, fillers, foam regulators, perfumes, pigments, sod suppressors, solvents, and structurants for liquid detergents, structure elasticizing agents, enzyme inhibitors or stabilizers, enzyme activators, transferase(s), hydrolytic enzymes, oxido reductases, bluing agents and fluorescent dyes, antioxidants, and solubilizers.

The term "textile" means any textile material including yarns, yarn intermediates, fibers, non-woven materials, natural materials, synthetic materials, and any other textile material, fabrics made of these materials and products made from fabrics (e.g., garments, linen and other articles). The textile or fabric may be in the form of knits, wovens, denims, non-wovens, felts, yarns, and towelling. The textile may be cellulose based, such as natural cellulosics including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained household laundry. When the term fabric or garment is used it is intended to include the broader term textiles as well.

The term "stability" includes storage stability and stability during use, e.g. during a wash process (in wash stability) and reflects the stability of the mannanase according to the invention as a function of time, e.g. how much activity is retained when the mannanase is kept in solution, in particular in a detergent solution. The stability is influenced by many factors, e.g. pH, temperature, detergent composition e.g. proteases, stabilizers, builders, surfactants etc. The mannanase stability may be measured using the 'activity assay' as described in examples.

"Mannanase activity" as used herein refers to the mannan degrading activity of a polypeptide. Degrading or modifying as used herein means that mannose units are hydrolyzed from the mannan polysaccharide by the mannanase. The mannan degrading activity of the polypeptides according to present invention can be tested according to standard test procedures known in the art. Example 7 provides an example of a standard method for determining mannanase activity.

In a further embodiment of the invention the at least one enzyme has mannanase activity. The mannanases comprised in the present enzyme composition of the invention are suitable for degrading and modifying mannan containing material in various chemical environments, preferably in detergent compositions.

In one embodiment of the present invention the enzyme composition further comprises one or more additional enzymes selected from the group consisting of protease, lipase, cutinase, amylase, carbohydrase, cellulase, pectinase, pectatelyase, pectinolytic enzyme, esterase, phytase, mannanase, arabinase, galactanase, xylanase, oxidase, xanthanase, xyloglucanase, DNAse, laccase, and/or peroxidase, preferably selected from the group consisting of proteases, amylases, cellulases and lipases.

The present enzyme composition comprising mannanase and an additional enzyme is advantageous in providing synergistic effect. Such additional enzymes are desired when the present enzyme composition comprising mannanase is used in detergents e.g. when washing stains. Particularly advantageous synergistic enzymes that work with mannanase are amylases, proteases and cellulases, or a combination thereof, such as a composition comprising mannanase, amylase and protease.

In general the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

A composition for use in solid laundry detergent, for example, may include 0.000001 % - 5%, such as 0.000005-2%, such as 0.00001%-1%, such as 0.00001 %-0.1% of enzyme protein by weight of the composition.

A composition for use in laundry liquid, for example, may include 0.000001 %-3%, such as 0.000005%-1%, such as 0.00001 %-0.1% of enzyme protein by weight of the composition.

A composition for use in automatic dishwash, for example, may include 0.000001 %-5%, such as 0.000005%-2%, such as 0.00001 %-1%, such as 0.00001 %-0.1% of enzyme protein by weight of the composition.

In a further embodiment of the present invention the detergent composition is in the form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition. There are a number of detergent formulation forms such as layers (same or different phases), pouches, as well as forms for machine dosing unit.

In an embodiment the present enzyme composition further comprises:
a. at least one preservative selected from benzoic acid, sodium benzoate, hydroxybenzoate, citric acid, ascorbic acid, or a combination thereof;
b. optionally at least one polyol selected from propylene glycol, glycerol, a sugar, sugar alcohol, lactic acid, boric acid, boric acid derivative, aromatic borate ester, phenyl boronic acid derivative, peptide, or a combination thereof;
c. optionally at least one enzyme selected from proteases, amylases, cellulases, lipases, xylanases, mannanases, cutinases, esterases, phytases, DNAses, pectinases, pectinolytic enzymes, pectate lyases, carbohydrases, arabinases, galactanases, xanthanases, xyloglucanase, laccases, peroxidases and oxidases with or without a mediator, or a combination thereof; and
d. optionally at least one filler selected from maltodextrin, flour, sodium chloride, sulfate, sodium sulfate, or a combination thereof.

The additional components a-d provide improved properties for the present enzyme composition. The enzyme composition is compatible with the additional components and improves applicability of the enzyme composition in various uses.

Salts, such as sodium chloride and sodium sulfate function as drying aids.

In an embodiment of the first aspect the present enzyme composition is in the form of a liquid composition or a solid composition such as solution, dispersion, paste, powder, granule, granulate, coated granulate, tablet, cake, crystal, crystal slurry, gel or pellet.

The present invention furthermore relates to different uses of the enzyme composition as herein disclosed, such as for degrading mannan and for use in a laundry process.

An enzyme composition can also be used in cleaning agents or boosters that are added on top of the detergent during or before the wash and that are for example in the form of liquid, gel, powder, granules or tablets. Enzyme composition and detergent components may also be soaked in a carrier like textiles.

In an embodiment the mannanase has relative activity of at least 50% in the pH range from 5.5 to 8.5. The relative activity may be determined by the method described in Example 7.

In an embodiment of the present invention the mannanase has a relative activity of at least 30% in the temperature range from 45° to 65°C.

Providing mannanases that retain activity in temperatures above ambient temperature is advantageous for applications wherein mannan degradation is required in such conditions. Further, the mannanases according to invention may have good stability and activity in alkaline conditions, which is advantageous in detergent use and in biomass processing.

In an embodiment the mannanase enzyme has an amino acid sequence with at least or about 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO:16.

In an embodiment the mannanase enzyme has an amino acid sequence which is not 100% identical to SEQ ID NO: 16 [Man7].

In an embodiment the present enzyme composition comprises the recombinant host cell of the second aspect.

In an embodiment of the second aspect the recombinant the recombinant polypeptide is a fusion protein which, in addition to having the amino acid sequence having mannanase activity, comprises at least one of:
an amino acid sequence providing a secretory signal sequence, such as *Bacillus amyloliquefaciens* xylanase signal sequence;
an amino acid sequence which facilitates purification, such as an affinity tag, His-tag;
an amino acid sequence which enhances production, such as an amino acid sequence which is a carrier, such as CBM;
an amino acid sequence having an enzyme activity; and
an amino acid sequence providing for the fusion protein with binding affinity, such as a carbohydrate binding moiety.

The CBM, carbohydrate binding moiety, as a carrier is advantageous e.g. in *Trichoderma* production.

In an embodiment the host cell is non-pathogenic. This is particularly advantageous for using the host cell in feed, and in detergent applications such as in home laundry detergents.

In an embodiment of the fifth aspect the mannan containing material is selected from plant based material, textile, waste water, sewage, oil or a combination thereof.

In another embodiment the mannan containing material is recycled waste paper; mechanical pulp, chemical pulp, semi chemical pulp, Kraft or other paper-making pulps; fibres subjected to a retting process; or guar gum or locust bean gum containing material.

In another embodiment degradation or modifying is carried out in an aqueous environment wherein mannanase shows activity.

In a preferred embodiment the mannan containing material, which is degraded or modified in the method, is on a textile or a fabric optionally with mannan stains. By degrading mannan attached to the textile or fabric, dirt or soil bound to mannan is released and not capable of binding again to the mannan or mannan stains. The textile or fabric can be of any material, for example cotton, flax/linen, jute, ramie, sisal or coir or manmade cellulosics (e.g. originating from wood pulp) including viscose/rayon, modal, cellulose acetate fibers (tricell), lyocell, cupro or blends thereof.

In an embodiment of the sixth aspect the animal is a monogastric animal or a ruminant. In another embodiment the animal is a broiler chicken, egg-laying chicken, swine, turkey, or an aquaculture organism such as fish. In another embodiment the animal is a ruminant.

In an embodiment the feed comprises or consists of maize and soybean meal.

In an embodiment the protein source of plant origin comprises or consist of soy, cereal such as barley, wheat, rye, oats, or maize.

In an embodiment the mannan containing product or by-product comprises or consists of palm kernel, guar meal or copra meal.

In an embodiment of the sixth or seventh aspect the animal feed or the feed supplement is formulated in the form of a wet composition or a dry composition.

In an embodiment or the ninth aspect the detergent is a liquid detergent or a solid detergent preferably in a form of a powder, bar, tablet, pouch, paste, gel, liquid, granule or granulate.

In an embodiment the composition comprising at least one mannanase enzyme is used in pulp and paper industry, biobleaching, fiber modification, drainage improvement and in the oil industry, i.e. in oil drilling or oil-servicing industry for hydro-fracturing or controlling the viscosity of drilling fluids.

In an embodiment the composition comprising at least one mannanase enzyme is used in textile and detergent industry, biomass processing and biomass hydrolysis, preferably in biofuel, starch, pulp and paper, food, baking, feed or beverage industries.

In an embodiment the mannanase hydrolyses endo-beta-1,4-mannosidic linkages randomly.

In an embodiment the mannanase is obtainable or derivable from a bacterial source.

In an embodiment the mannanase can be fused with at least one further polypeptide, thus forming a fusion polypeptide. The fusion polypeptide or the further polypeptide may have other catalytic or binding activities in addition to those of mannanase. In an embodiment the further polypeptide comprises or consists of carbohydrate binding module, which is optionally a fragment of another protein or enzyme derived from the same or different organism as the mannanase.

In an embodiment the mannanase is connected to the further polypeptide with a linker.

In an embodiment is provided a process for machine treatment of fabrics which process comprises treating fabric during a washing cycle of a machine washing process with a washing solution containing the enzyme composition of the first aspect, the enzyme obtainable from the recombinant host cell of the second aspect or the recombinant polypeptide of the third aspect.

In an embodiment is provided a use of the enzyme composition of the first aspect, the enzyme obtainable from the recombinant host cell of the second aspect, or the polypeptide of the third aspect together with an enzyme selected from protease, amylase, cellulase, lipase, xylanase, mannanase, cutinase, esterase, phytase, DNAse, pectinase, pectinolytic enzyme, pectate lyase, carbohydrase, arabinase, galactanase, xanthanase, xyloglucanase, laccase, peroxidase and oxidase with or without a mediator in a cleaning composition for fabric cleaning and/or fabric stain removal.

In an embodiment is provided a use of the enzyme composition of the first aspect, the enzyme obtainable from the recombinant host cell of the second aspect, or the polypeptide of the third aspect together with an enzyme selected from protease, amylase, cellulase, lipase, xylanase, mannanase, cutinase, esterase, phytase, DNAse, pectinase, pectinolytic enzyme, pectate lyase, carbohydrase, arabinase, galactanase, xanthanase, xyloglucanase, laccase, peroxidase and oxidase with or without a mediator in a cleaning composition for cleaning hard surfaces such as floors, walls, bathroom tile and the like.

In an embodiment is provided a use of the enzyme composition of the first aspect, the enzyme obtainable from the recombinant host cell of the second aspect, or the polypeptide of the third aspect together with an enzyme selected from protease, amylase, cellulase, lipase, xylanase, mannanase, cutinase, esterase, phytase, DNAse, pectinase, pectinolytic enzyme, pectate lyase, carbohydrase, arabinase, galactanase, xanthanase, xyloglucanase, laccase, peroxidase and oxidase with or without a mediator in a cleaning composition for hand and machine dishwashing.

### EXAMPLES

The following examples are provided to illustrate various aspects of the present invention. They are not intended to limit the invention, which is defined by the accompanying claims.

### Example 1. Screening

For identification of new beta-1,4-mannanases public databases (NCBI, EBI) and selected proprietary and public genomes were screened. All proprietary and public genomes used in this work are shown in Table 1. All hits were grouped and finally 15 genes of bacterial origin were selected for cloning in *Bacillus* based on the phylogenetic distance between each other (Table 2)

**Table 1. List of proprietary and public genomes used for screening of beta-1,4-mannanases**

| **Species** | **Strain** | **Source** |
|---|---|---|
| *Bacillus pumilus* | MS8 | ABE |
| *Amphibacillus xylanus* | NBRC 15112 | NCBI |
| *Bacillus hemicellulosilyticus* | JCM 9152 | NCBI |
| *Bacillus clausii* | KSM-K16 | NCBI |
| *Bacillus amyloliquefaciens* | RH1330 | ABE |
| *Virigibacillus soli* | PL205 | NCBI |

**Table 2. List of genes selected for cloning in Bacillus. Predicted PFAM domains and amino acid lengths of the proteins are shown**

| **Sequence ID** | **Species** | **GH family** | **Length** |
|---|---|---|---|
| orf2511 | *Bacillus amyloliquefaciens* | 26 | 360 aa |
| AXY_08250 | *Amphibacillus xylanus* | 5 | 497 aa |
| *man*7 | *Bacillus hemicellulosilyticus* | 5 | 490 aa |
| T1Z249.2 | *Bacillus nealsonii* | 5 | 369 aa |
| *man*6 | *Bacillus clausii* | 5 | 324 aa |
| Q9EYQ3 | *Clostridium cellulolyticum* | 5 | 424 aa |
| YdhT | *Bacillus cellulosilyticus* | 26 | 1183 aa |
| V5X1N9 | *Paenibacillus polymyxa* | 5 | 588 aa |
| Q9ZI87 | *Geobacillus stearothermophilus* | 5 | 694 aa |
| Q49HI4 | *Bacillus circulans* | 5 | 327 aa |
| orf0659 | *Bacillus pumilus* | 5 | 376 aa |
| JCM9152_1090 | *Bacillus hemicellulosilyticus* | 26 | 489 aa |
| D3HC62 | *Streptococcus gallolyticus* | 5 | 487 aa |
| A0LSH9 | *Acidothermus cellulolyticus* | 5 | 763 aa |
| *man*14 | *Virgibacillus soli* | 5 | 482 aa |

### Example 2. Cloning of bacterial mannanases in Bacillus

Unless otherwise stated, the molecular biological methods including DNA manipulations and transformations were performed as described in Sambrook and Russell (2001) and Harwood and Cutting (1990). The genes *man*6*, man*7 and *man*14 were amplified by PCR using Pfx Accu Prime Polymerase (Invitrogen). PCRs were performed according to manufacturer's instructions. Following PCR conditions were used for construction of the expression plasmids: 120 sec initial denaturation at 94 °C, followed by 35 cycles of 15 sec at 94 °C, 30 sec annealing at one of the following 50/55 °C, 110/290 sec extension at 68 °C and the final extension at 68 °C for 10 min. For amplification of *man*7 genomic DNA of *Bacillus hemicellulosilyticus* JCM 9152 was used. *man*6 and *man*14 were ordered as synthetic genes without codon optimization (Eurofins MWG, Germany). Sequences of primers used for cloning are shown in Table 3. Overhangs for hybridization are underlined.

**Table 3. List of primers used for amplification of man6, man7 and man14**

| **Template** | **Primer** | **bp** | **Sequence** | **Seq ID No** |
|---|---|---|---|---|
| syn. gene *man*6 | Man6_1 | 39 | | 1 |
| syn. gene *man*6 | Man6_2 | 39 | | 2 |
| gDNA *B. hemicellulosilyticus* | Man7_1 | 37 | | 3 |
| gDNA *B. hemicellulosilyticus* | Man7_2 | 36 | | 4 |
| syn. Gene *man14* | Man14_1 | 40 | | 5 |
| syn. Gene *man14* | Man14_2 | 39 | | 6 |
| pUB110 derivate | Vec_1 | 17 | AGCTGCAGAGGCGGTTG | 7 |
| pUB110 derivate | Vec_2 | 21 | GACAGAGATATACCGACAGTG | 8 |

Genes were cloned in a standard vector pEV1 pEV1 (Fig. 1), a pUB110 derivate including promoter PaprE from *Bacillus licheniformis* and xylanase signal peptide from *Bacillus amyloliquefaciens,* by using NEBuilder®Hifi DNA Assembly Master Mix (NEB, Frankfurt). A vector:insert ration of 1:3 was applied for cloning. The total amount of fragments was at 0.2 pmol in a total volume of 20 µl. Samples were incubated for 40 min at 50°C. For construction purposes, expression plasmids were transformed by induced competence in *Bacillus subtilis* SCK6 as described in Zhang & Zhang 2011. The transformed cells were plated onto LB (Luria-Bertani) plates supplemented with 10 mg/l Kanamycin. Plates were incubated for 20h at 37°C. Arising colonies were picked and plasmid was isolated using QiaPrep MiniPrep Kit (Qiagen, Hilden). Isolation procedure was carried out according to the manufacturers recommendations for Gram positives plasmid preparations. Inserts were sequenced via Sanger sequencing (GATC, Germany) and revealed the DNA sequences corresponding to the mature parts of the mannanases Man6, Man7 and Man14. Sequence comparisons were done using ClustalW sequence alignment (Thompson et al 1994). Finally, expression plasmids were transformed in an appropriate *Bacillus* production strain via electroporation. *Bacillus* production strain was grown in electroporation medium containing 20 g/l Trypton, 10 g/l yeast extract, 10 g NaCl and 2 M saccharose and 10 ml were harvested at an OD (600nm) of 0.4. Cells were washed with electroporation buffer containing 0,272 M saccharose, 1 mM MgCl₂ and 7 mM KH₂PO4 and finally resuspended in 250 µl electroporation buffer. Electroporation was performed using following conditions: 1,2 kV, 150 Ω, 50 µF. 1ml electroporation medium was added afterwards and cells were incubated for 3h at 37°C. Cells were plated on LB plates supplemented with 20 mg/l kanamycin and incubated for 18h at 37°C. Clones were verified as described above and used for generation of material for analytic tests. Therefore, strains were inoculated in a standard expression under protein inducing conditions and incubated for 30h at 37°C. Supernatants were harvested and used for analytical and application tests. Genes and enzyme characteristics are shown in Table 4 and 5.

**Table 4. The summary on the GH5 family mannanase encoding genes from Bacillus clausii KSM-K16, Bacillus hemicellulosilyticus JCM 9152 and Virgibacillus soli PL205.**

| **Gene** | **Length including SP (bp)** | **SEQ ID NO** |
|---|---|---|
| *man*6 | 975 | 9 |
| *man*7 | 1473 | 13 |
| *man*14 | 1449 | 17 |

**Table 5. The summary of the amino acid sequences deduced from the GH5 mannanase encoding gene sequences from Bacillus clausii KSM-K16, Bacillus hemicellulosilyticus JCM 9152 and Virgibacillus soli PL205.**

| **Man protein** | **No of AAs** | **Length of SS** | **CBM** | **Predicted MW (Da), ss not included** | **Predicted pi, ss not included** | **SEQ ID NO** |
|---|---|---|---|---|---|---|
| Man6 | 324 | 35 | | 31.84 | 4.56 | 11 |
| Man7 | 490 | 21 | Yes | 51.36 | 4.81 | 15 |
| Man14 | 482 | 16 | Yes | 50.68 | 4.35 | 19 |

### Example 3. PCR-cloning of bacterial mannanases man6 and man7 in Trichoderma reesei

Standard molecular biology methods were used in the isolation and enzyme treatments of DNA (e.g. isolation of plasmid DNA, digestion of DNA to produce DNA fragments), in *E. coli* transformations, sequencing etc. The basic methods used were either as described by the enzyme, reagent or kit manufacturer or as described in the standard molecular biology handbook, e.g. Sambrook and Russell (2001). Isolation of genomic DNA was performed as described in detail by Raeder and Broda (1985).

*Man*6 and *man*7 from *Bacillus clausii* and *Bacillus hemicellulosilyticus,* respectively, were also cloned for expression in *Trichoderma reesei.* The genes were PCR-cloned using synthetic genes with codon optimization for *Trichoderma reesei.* DNA sequences encoding the signal peptides of *man*6 and *man*7 were removed by using PCR and new cloning sites created. The sequences of the primers are shown in Table 6 (SEQ ID NOs: 21-24).

**Table 6. The oligonucleotides used as PCR primers to amplify Bacillus hemicellulosilyticus and Bacillus clausii mannanase genes.**

| **Template, (synthetic) DNA from** | **Oligonucleotides** | **Length (bp)** | **Sequence^{(a}** | **SEQ ID NO:** |
|---|---|---|---|---|
| *Bacillus hemicellulosilytic* us | BMAN1 | 60 | | 21 |
| *Bacillus hemicellulosilytic* us | BMAN2 | 46 | | 22 |
| *Bacillus clausii* | BMAN3 | 60 | | 23 |
| *Bacillus clausii* | BMAN4 | 50 | | 24 |

| | | | | |
|---|---|---|---|---|
| (^{a}"s" in the parenthesis = sense strand, "as" = antisense strand. | | | | |

The genes were amplified by PCR with primers described in Table 6 and using synthetic DNAs as templates in the reactions. The PCR mixtures of *Bacillus clausii man6* and *Bacillus hemicellulosilyticus man*7 contained each 1× HF buffer for Phusion HF Polymerase (NEB/BioNordika, Finland), 0.2 mM dNTP mix (Thermo Fisher Scientific, Finland), 1 µM each primer, 3% DMSO (Thermo Fisher Scientific), 1 unit of Phusion High-Fidelity Polymerase (NEB/BioNordika, Finland) and 50 ng of the corresponding plasmid DNA. The conditions for the PCR reactions were the following: 30 sec initial denaturation at 98°C, followed by 28 cycles of 10 sec at 98°C, 30 sec annealing at one of the following 45/50/55/60 °C, 45 sec extension at 72°C and the final extension at 72 °C for 7min.

Primer combination described in Table 6 produced specific DNA products having the expected sizes. The PCR products were isolated from agarose gel with GenJet Gel Extraction Kit (Thermo Fisher Scientific) according to manufacturer's instructions, digested with *Nru*l and *Bam*HI restriction enzymes (Thermo Fisher Scientific) and cloned into an expression vector cleaved with *Nru*l and *Bam*Hl. Ligation mixtures were transformed into *Escherichia coli* XL1-Blue (AH Diagnostics) and plated on LB (Luria-Bertani) plates containing 50-100 µg/ml ampicillin. Several *E. coli* colonies were collected from the plates and DNA was isolated with GenJet Plasmid Miniprep Kit (Thermo Fisher Scientific). Positive clones were screened using restriction digestions. The genes encoding the *Bacillus clausii man*6 and *Bacillus hemicellulosilyticus man*7 GH5 mannanases without their own signal peptide encoding sequences were sequenced and the plasmids were named pALK4274 and pALK4273, respectively (For details see Example 6).

### Example 4. Cloning of synthetic bacterial mannanase man14

Standard molecular biology methods were used in the isolation and enzyme treatments of DNA (e.g. isolation of plasmid DNA, digestion of DNA to produce DNA fragments), in *E. coli* transformations, sequencing etc. The basic methods used were either as described by the enzyme, reagent or kit manufacturer or as described in the standard molecular biology handbook, e.g. Sambrook and Russell (2001). Isolation of genomic DNA was performed as described in detail by Raeder and Broda (1985).

Mannanase gene *man14* from *Virgibacillus soli was* also cloned for *Trichoderma* expression. The gene encoding GH5 family mannanase Man14 from *Virgibacillus soli was* ordered from GenScript as a synthetic construct with codon optimization for *Trichoderma reesei.*

Plasmid DNA obtained from GenScript including the *man14* gene was resuspended in sterile water, digested with *Nru*l and *Bam*HI restriction enzymes (Thermo Fisher Scientific) according to manufacturer's instructions and cloned into an expression vector cleaved with *Nru*l and *Bam*Hl. Ligation mixture was transformed into *Escherichia coli* XL1-Blue (AH Diagnostics) and plated on LB (Luria-Bertani) plates containing 50-100 µg/ml ampicillin. Several *E. coli* colonies were collected from the plates and DNA was isolated with GenJet Plasmid Miniprep Kit (Thermo Fisher Scientific). Positive clones were screened using restriction digestions and they were shown to contain inserts of expected sizes. Fusion sites of *Virgibacillus soli man14* to the expression plasmid were sequenced and the plasmid was named pALK4414 (For details see Example 6).

### Example 5. Production of recombinant bacterial GH5 mannanase proteins in Bacillus

Expression plasmids were constructed for production of recombinant GH5 mannanase (Man6, Man7 and Man14) proteins from *Bacillus clausii, Bacillus hemicellulosilyticus* and *Virgibacillus soli.* The expression plasmids constructed are listed in Table 7. The recombinant GH5 genes *(man*6*, man*7 and *man*14*),* without their own signal sequences, were fused to the *Bacillus licheniformis PaprE promoter* and *B*. *amyloliquefaciens* xylanase signal peptide. The transcription termination was ensured by a strong terminator and a kanamycin resistance marker was used for selection of the transformants. The transformations were performed as described in Example 2.

**Table 7. The expression plasmids constructed to produce Man6, Man7 and Man14 recombinant proteins from Bacillus clausii, Bacillus hemicellulosilyticus and Virgibacillus soli in an appropriate Bacillus expression strain.**

| **Mannanase (GH5) protein** | **Expression plasmid** |
|---|---|
| Man6 | pEV1 Man6 |
| Man7 | pEV1 Man7 |
| Man14 | pEV1 Man14 |

The GH5 production of the transformants was analyzed from the culture supernatants of the shake flask cultivations. The transformants were inoculated from the LB plates to shake flasks containing 2 % glucose, 6 % corn steep powder, 1,3 % (NH4)2HPO4, 0,05 % MgSO4 × 7H2O and 0,5 % CaCl2. pH was adjusted to pH 7.5. The GH5 protein production of the transformants was analyzed from culture supernatants after growing them for 30 hours at 37 °C, 180 rpm. Heterologous production of recombinant proteins was analyzed by SDS-PAGE with subsequent Coomassie staining.

The best producing transformants were chosen to be cultivated in laboratory scale bioreactors. The transformants were cultivated in bioreactors at 37 °C under protein inducing conditions and additional feeding until a suitable yield was reached. The supernatants were recovered for application tests by centrifugation or filtration.

### Example 6. Production of recombinant bacterial GH5 mannanase proteins in Trichoderma reesei

Expression plasmids were constructed for production of recombinant GH5 mannanase (Man6, Man7 and Man14) proteins from *Bacillus clausii, Bacillus hemicellulosilyticus* and *Virgibacillus soli* (See Examples 3 and 4) in *Trichoderma reesei.* The expression plasmids constructed are listed in Table 8. The recombinant GH5 genes *(man*6*, man*7 and *man*14*),* without their own signal sequences, were fused to the *T. reesei cel7A*/*cbh1* promoter with *T. reesei cel6Ncbh2* CBM carrier and linker followed by Kex2 protease recognition site. The transcription termination was ensured by the *T. reesei cel7A*/*cbh1* terminator and the *A. nidulans amdS* marker gene was used for selection of the transformants as described in Paloheimo *et al.* (2003). The linear expression cassettes (Fig. 2) were isolated from the vector backbones after *Not*l digestions and were transformed into *T. reesei* protoplasts. The host strains used does not produce any of the four major *T. reesei* cellulases (CBHI, CBHII, EGI, EGII). The transformations were performed as in Penttilä *et al.* (1987) with the modifications described in Karhunen *et al.* (1993), selecting acetamidase as a sole nitrogen source *(amd*S marker gene). The transformants were purified on selection plates through single conidia prior to sporulating them on PD.

**Table 8. The expression cassettes constructed to produce Man6, Man7 and Man14 recombinant proteins from Bacillus clausii, Bacillus hemicellulosilyticus and Virgibacillus soli in Trichoderma reesei. The overall structure of the expression cassettes was as described in Fig. 2.**

| **Mannanase (GH5) protein** | **Expression plasmid** | **Expression cassette ^{(a}** |
|---|---|---|
| Man6 | pALK4274 | 7.0 kb *Not*l |
| Man7 | pALK4273 | 7.5 kb *Not*l |
| Man14 | pALK4414 | 7.6 kb *Not*l |

| | | |
|---|---|---|
| ^{(a} The expression cassette for *T. reesei* transformation was isolated from vector backbone by using *Not*l digestion. | | |

The mannanase production of the transformants was analyzed from the culture supernatants of the shake flask cultivations. The transformants were inoculated from the PD slants to shake flasks containing 50 ml of complex lactose-based cellulase inducing medium (Joutsjoki *at al.* 1993) buffered with 5 % KH₂PO₄. The GH5 protein production of the transformants was analyzed from culture supernatants after growing them for 7 days at 30 °C, 250 rpm. Heterologous production of recombinant proteins was analyzed by SDS-PAGE with subsequent Coomassie staining.

The best producing transformants were chosen to be cultivated in laboratory scale bioreactors. The transformants were cultivated in bioreactors either on batch or by additional feeding type of process under protein inducing conditions at a typical mesophilic fungal cultivation temperature and slightly acidic conditions. The cultivation was continued until depletion of the medium sugars or until suitable yield was reached. The supernatants were recovered for application tests by centrifugation or by filtration.

### Example 7. Assay of galactomannanase activity by DNS -method

Mannanase activity (MNU) was measured as the release of reducing sugars from galactomannan (0.3 w/w-%) at 50°C and pH 7.0 in 5 min. The amount of released reducing carbohydrates was determined spectrophotometrically using dinitrosalicylic acid.

Substrate (0,3 w/w-%) used in the assay was prepared as follows: 0.6 g of locust bean gum (Sigma G-0753) was in 50 mM sodium citrate buffer pH 7 (or citrate phosphate buffer pH 7) at about 80 °C using a heating magnetic stirrer and heated up to boiling point. The solution was cooled and let to dissolve overnight in a cold room (2 - 8 °C) with continuous stirring and insoluble residues were removed by centrifugation. After that solution was filled up to 200 ml by buffer. Substrate was stored as frozen and melted by heating in a boiling water bath to about 80 °C, cooled to room temperature and mixed carefully before use.

DNS reagent used in the assay was prepared by dissolving 50 g of 3.5-dinitrosalisylic acid (Sigma D-550) in about 4 liter of water. With continuous magnetic stirring 80.0 g of NaOH was gradually added and let to dissolve. An amount of 1500 g of Rochelle Salt (K-Na-tartrate, Merck 8087) was added in small portions with continuous stirring. The solution that was cautiously warmed to a maximum temperature of 45 °C., was cooled to room temperature and filled up to 5000 ml. After that it was filtered through Whatman 1 filter paper and stored in a dark bottle at room temperature.

The reaction was first started by adding 1.8 ml of substrate solution to each of the two test tubes and let to equilibrate at 50 °C for 5 minutes, after which 200 µl of suitably diluted enzyme solution was added to one of the tubes, mixed well with vortex mixer and incubated exactly for 5 min at 50 °C. Enzyme blanks didn't need to be equilibrated or incubated. The reaction was stopped by adding 3.0 ml of DNS reagent into both tubes and mixed. 200 µl of sample solution was added to the enzyme blank tubes. Both tubes were placed in a boiling water bath. After boiling for exactly 5 minutes, the tubes were placed in a cooling water bath and allow them to cool to room temperature. The absorbance of sample was measured against the enzyme blank at 540 nm and activity was read from the calibration curve and multiplied by the dilution factor. A suitable diluted sample yielded an absorbance difference of 0.15 - 0.4.

Standard curve was prepared 20 mM from mannose stock solution by dissolving 360 mg of mannose (SigmaM-6020, stored in a desiccator) in assay buffer and diluted to solutions containing 3, 6, 10 and 14 µmol/ml of mannose. Standards were handled like the samples except for incubating at 50°C. The absorbances were measured against the reagent blank (containing buffer instead of standard dilution of mannose) at 540 nm. Calibration curve was constructed for every series of assays.

One mannanase unit (MNU) was defined as the amount of enzyme that produces reductive carbohydrates having a reductive power corresponding to one nmol of mannose from galactomannan in one second under the assay conditions (1 MNU = 1nkat).

### Example 8. Purification of Man6 mannanase

Cells and solids were removed from the fermentation culture medium by centrifugation for 10 min, 4000 g at 4°C. The supernatant of 10 ml was used for protein purification. The sample was filtered through 0.44 µm PVDF membrane (Millex-HV, Merck Millipore Ltd,Carrigtwohill, IRL). The filtrate was loaded onto a HiPrep 26/10 Desalting column (GE Healthcare, Uppsala, Sweden) equilibrated in 20 mM HEPES pH 7. The desalted sample was then loaded onto a 5 ml HiTrap Q HP column (GE Healthcare, Uppsala, Sweden) pre-equilibrated with 20 mM HEPES pH 7. After sample loading, the column was washed with the same buffer for 20 ml. Proteins were eluted with linear salt gradient 20 mM HEPES, 500 mM NaCl pH 7 in 15 CVs. Fractions of 5 ml were collected and analyzed on SDS-PAGE. The fractions containing target protein were combined and concentrated to 2 ml using Vivaspin 20, 10 kDa MWCO ultrafiltration devices (GE Healthcare). The concentrated sample was further fractionated using Superdex 75 26/60 gel-filtration column equilibrated with 20 mM MES, 200 mM NaCl pH 6,5. Fractions of 2 ml were collected and analyzed by SDS-PAGE. Fractions containing pure mannanase were combined. Other mannanases were purified using the same protocol but changing the buffer composition in desalting and ion exchange steps. Buffer compositions are shown in Table 9.

**Table 9. Buffers used in ion exchange chromatography**

| **Mannanase** | **Buffers used in ion exchange chromatography** |
|---|---|
| Man6 | 20 mM HEPES pH 7 |
| Man7 | 20 mM HEPES pH 7 |
| Man14 | 20 mM MES pH 6 |

Purified samples were above 95% pure.

Enzyme content of the purified sample was determined using UV absorbance 280 nm measurements. Excitation coefficients for each mannanases were calculated on the bases of amino acid sequence of the enzyme by using ExPASy (Server http://web.expasy.org/protparam/). (Gasteiger et al. 2005).

The enzyme activity (MNU) of purified samples was measured as release of reducing sugars as described in Example 7.

The specific activity (MNU/mg) of mannanases was calculated by dividing MNU activity of purified sample with the amount of purified enzyme. Obtained values were used for calculating enzyme dosages used in Examples 10 and 11.

### pH profiles of mannanases

The pH profiles of purified mannanases were determined using the beta-mannazyme tablet assay Azurine-crosslinked carob galactomannan (T-MNZ 11/14) from Megazyme with minor modifications to the suggested protocol. The linearity of the assay has been checked with each purified enzymes. The assay was performed in 40 mM Britton-Robinson buffer adjusted to pH values between 4 and 11. The enzyme solution was diluted into the assay buffer and 500 µl of enzyme solution was equilibrated at 50°C water bath for 5 min before adding one substrate tablet. After 10 minutes, the reaction was stopped by adding 10ml 2% Tris pH 12. The reaction tubes were left at room temperature for 5 min, stirred and the liquid filtered through a Whatman No.1 paper filter. Release of blue dye from the substrate was quantified by measuring the absorbance at 595 nm.

Enzyme activity at each pH was reported as relative activity where the activity at the pH optimum was set to 100%. The pH profiles were shown in Figure 3. Relative activity (%) of mannanase is calculated by dividing mannanase activity of a sample by the mannanase activity of a reference sample. In the case of pH profile, the reference sample is a sample at the optimal pH. In the case of temperature profile the reference sample is a sample at the optimal temperature.

### Temperature profiles of mannanases

The temperature optimum of purified mannanases was determined using the beta-mannazyme tablet assay Azurine-crosslinked carob galactomannan (T-MNZ 11/14) from Megazyme with minor modifications to suggested protocol. The assay was performed at temperatures varying between 30-90°C for 10 minutes in 40 mM Britton-Robinson buffer pH7. Enzyme activity was reported as relative activity where the activity at temperature optimum was set to 100%. The temperature profiles were shown in Figure 4.

### Temperature and pH characteristics of mannanases

Man6 has a molecular mass between 30-35 kDa. The optimal temperature of the enzyme at pH 7 is from 50°C to 70°C. Said enzyme has pH optimum at the pH range of at least pH 6 to pH 9 at 50°C. The optimal temperature and pH optimum were determined using 10 min reaction time and Azurine-crosslinked carob galactomannan as a substrate.

Man7 has a molecular mass between 50-55 kDa. The optimal temperature of the enzyme at pH 7 is from 40°C to 60°C. Said enzyme has pH optimum at the pH range of at least pH 7 to pH 10 at 50°C. The optimal temperature and pH optimum were determined using 10 min reaction time and Azurine-crosslinked carob galactomannan as a substrate.

Man14 has a molecular mass between 30-40 kDa. The optimal temperature of the enzyme at pH 7 is from 50°C to 60°C. Said enzyme has pH optimum at the pH range of at least pH 7 to pH 8 at 50°C. The optimal temperature and pH optimum were determined using 10 min reaction time and Azurine-crosslinked carob galactomannan as a substrate.

### Example 9. Stain removal performance of Man6 and Man7 mannanases with commercial detergents without bleaching agents

Man6 and Man7 mannanases produced in *Bacillus* (as described in Example 5) and in *Trichoderma* (as described in Example 6), were tested for their ability to remove mannanase sensitive standard stains at 40 °C and water hardness of 16°dH with commercial detergents without bleaching agents and compared to commercial mannanase preparation Mannaway® 4,0 L (Novozymes). The following artificially soiled test cloths from Center for test material B.V. (the Netherlands) were used: Chocolate pudding mannanase sensitive on cotton (E-165), Locust bean gum, with pigment on cotton (C-S-73) and on polyester/cotton (PC-S-73) and Guar gum with carbon black on cotton (C-S-43). The fabric was cut in 6 cm x 6 cm swatches and 2 pieces of each were used in test.

Commercial heavyduty liquid detergent A containing all other enzymes except mannanase was used at concentration of 4.4 g per liter of wash liquor and Commercial Color detergent powder without enzymes was used at 3.8 g/l. Detergent containing wash liquors we prepared in synthetic tap water with hardness of 16 °dH. Protease Savinase® 16 L (0.5 w/w %) and amylase Stainzyme® 12 L (0.4 w/w %) was added into hard water used with commercial Color detergent powder, the liquid detergent already contained amylase and protease. pH of the wash liquor of Color detergent powder was approximately 10 and with the liquid detergent approximately 8.3.

Mannanase dosages were in range 0 - 0.2/0.25% of detergent weight but for the evaluation the dosages were calculated as enzyme activity units (MNU) per ml wash liquor or as mg of active enzyme protein (AEP) per I of wash liquor. Activity was measured as described in Example 7. AEP content of each preparation was calculated by dividing the enzyme activity with specific activity, defined in Example 8. Control sample contained the detergent solution but no mannanase.

For synthetic tap water with hardness of 16 °dH the following stock solutions were prepared in deionized water (Milli-Q or equivalent):
Stock solution with 1000 °d Calcium-hardness: CaCl2 x 2 H2O (1.02382.1000, Merck KGaA, Germany) 26.22 g/l
Stock solution with 200 °d Magnesium-hardness: MgSO4 × 7 H2O (1.05886.1000, Merck KGaA, Germany) 8.79g/l H2O
NaHCO3 stock solution: NaHCO3 (1.06329.1000 Merck KGaA, Germany) 29.6g/l 13.3 ml CaCl2 solution, 13.3 ml MgSO4 solution and 10.0 ml of freshly made NaHCO3 solution were added in volumetric flask in the given order, made up to 1 liter with deionized water and mixed. The hardness of water was determined by complexometric titration and found correct.

Stain removal treatments were performed in Atlas LP-2 Launder-Ometer as follows. Launder-Ometer was first preheated to 40°C. Then detergent, 250 ml synthetic tap water with hardness of 16 °dH and diluted enzyme (<1.0 ml) were added into 1.2 liter containers. Stains were added and the Launder-Ometer was run at 40°C for 60 min with a rotation speed of 42 rpm. After that the swatches were carefully rinsed under running water and dried overnight at indoor air, on a grid protected against daylight.

The stain removal effect was evaluated by measuring the colour as reflectance values with Konica Minolta CM-3610A spectrophotometer using L*a*b* color space coordinates (illuminant D65/10°, 420 nm cut). Fading of the stains, indicating mannanase performance (stain removal efficiency) was calculated as ΔL* (delta L*), which means lightness value L* of enzyme treated fabric minus lightness value L* of fabric treated with washing liquor without mannanase (control). Final results (total stain removal effect) were shown as sum of ΔL* of each stains. Color values of each stains were average of 2 swatches.

The results obtained with commercial liquid detergent are shown in Figs. 6-7. The mannanases according to the invention have similar (Man6) or considerably better (Man7) stain removal performance with liquid detergent when dosed as activity units or as active enzyme protein compared to commercial mannanase preparation Mannaway® 4,0 L. Similar performance was obtained with Man6 and Man7 regardless of the expression host, *Bacillus* or *Trichoderma* (Fig 6).

The results obtained with commercial color detergent powder (Figs. 8-9) show that the mannanases according to the invention have better stain removal performance with color detergent powder when dosed as activity units or as active enzyme protein compared to commercial mannanase preparation Mannaway® 4,0 L.

### Example 10. Stain removal performance Man6 and Man7 mannanases with bleach containing detergent

Man6 and Man7 mannanases produced in *Bacillus* (as described in Example 5) were tested for their ability to remove mannanase sensitive standard stains at 40°C and water hardness of 16°dH with commercial bleach detergent powder and compared to commercial mannanase preparation Mannaway® 4,0 L (Novozymes). Test system was similar to described in Example 9, except three different artificially soiled test cloths from Center for testmaterial B.V. (the Netherlands) were used: Chocolate pudding mannanase sensitive on cotton (E-165), Locust bean gum, with pigment on cotton (C-S-73) and Guar gum with carbon black on cotton (C-S-43). Commercial Color detergent powder was used at concentration of 4.2g per liter of wash liquor and pH of the wash liquor was approx. 9.5. Protease Savinase® 16 L (0.5 w/w %) and amylase Stainzyme® 12 L (0.4 w/w %) were added into hard water used in test, since the detergent didn't contain any enzymes.

The color of the swatches after treatment was measured and results calculated as sum of ΔL* of each 3 stains as described in Example 9.

The results (Fig. 10) obtained with commercial bleach containing detergent indicate that the mannanase according to the invention (Man7) has considerably better stain removal performance compared to commercial mannanase Mannaway® 4,0 L when dosed as active enzyme protein. With Man6 at least similar performance compared to a commercial bacterial mannanase is obtained.

### Example 11. Stain removal performance Man14 mannanase with commercial liquid detergent

Man14 mannanase produced in *Bacillus* (as described in Example 5) was tested for their ability to remove mannanase sensitive standard stains at 40 °C and water hardness of 16°dH with commercial heavy duty liquid detergent B and compared to commercial mannanase preparation Mannaway® 4,0 L (Novozymes). Test system was similar to that described in Example 9, except two different artificially soiled test cloths from Center for testmaterial B.V. (the Netherlands) were used: Chocolate pudding mannanase sensitive on cotton (E-165) and Locust bean gum, with pigment on cotton (C-S-73). Commercial heavy duty liquid detergent B was used at concentration of 5 g per liter of wash liquor and pH of the wash liquor was approximately 8.3. Protease Savinase® 16 L (0.5w/w %) and amylase Stainzyme® 12 L (0,4 w/w %) were added into hard water used in test, since the detergent didn't contain any enzymes.

The color of the swatches after treatment was measured and results calculated as sum of ΔL* of each 2 stains as described in Example 9.

The results (Figs. 11-12) obtained with commercial liquid containing detergent indicate Man14 had good performance in a liquid detergent, comparable to commercial product, when dosed either as activity units or as active enzyme protein.

### Example 12. Stability of Man6 and Man7 mannanases in commercial liquid detergents

The stability of Man6 and Man7 mannanase preparations produced in *Bacillus* were tested in OMO Color liquid obtained from local super market and compared to commercial mannanase preparation Mannaway® 4,0 L. Mannanase preparations were added 0.5% w/w-% in detergents and samples were incubated in plastic tubes with caps at 37°C for 5 weeks. The activity was measured at certain intervals by activity assay described in Example 7 except using 30 min incubation time. Results were calculated as residual activity (%), which was obtained by dividing the activity of a sample taken at certain time point by initial activity of the sample.

The stability of Man7 produced both in *Bacillus* and *Trichoderma* and Man6 produced in *Trichoderma* were tested against Mannaway® 4,0 L also in commercial liquid heavyduty detergent A containing protease but no mannanase. In this test 1 % -(w/w) of mannanases were used and samples incubated for 37°C for 12 weeks.

The results in Omo Color (Fig. 13) show that Man6 had considerably better and Man7 similar stability compared to Mannaway® 4,0 L. Both Man7 and especially Man6 were more stable than Mannaway® 4,0 L with another commercial liquid detergent A, as shown in Fig 14. Results obtained in another test at same conditions showed that Man6 had similar stability regardless of the expression host, *Bacillus* or *Trichoderma* (data not shown).

The results of the stability experiments show that the mannanase according to the invention is stabile in detergents for several weeks even when stored at high temperature like 37°C. The stability of the mannanases according to the invention (Man6 and Man7) is improved compared to a commercial bacterial mannanase in liquid detergent.

### Example 13. Efficiency study with mannanase alone and in combination with a non-starch polysaccharide (NSP) degrading enzyme in broilers

Effects of recombinant mannanases of the invention are studied on growth in broilers. Ultrafiltrate of the fermentation broth including the recombinant mannanase is dried and target levels applied to a pelleted broiler diet alone or in combination with a commercial available xylanase based product.

A control diet based on corn and dehulled solvent extracted soybean meal is fed without enzyme or added by different levels of the recombinant mannanase of the invention alone or in combination with a standard dose of a commercial xylanase.

Initial weight of the broilers is between 30g and 50g. The trial lasts between three and five weeks. Each treatment consists at minimum of six replicates with 10 broilers each. In each case the diet is analysed for moisture, crude protein, crude fibre, fat, ash, and enzyme protein.

Five diets are prepared:
1) unsupplemented control (BD)
2) BD + mannanase 1 - 500 mg/kg
3) BD + mannanase 1 - 1000 mg/kg
4) BD + mannanase 1 - 500 mg/kg + xylanase 1 ― 10 mg/kg
5) BD + xylanase 1 ― 10 mg/kg

Health status and mortality of the animals is checked daily by visual inspection. At days 0, 14, and 35 body weight gain (BW), feed intake (FI), and feedconversion ratio (FCR) are measured. FCR is calculated as the total feed consumed divided by the weight gain during the same period. Determination of the effect of the recombinant mannanases is based on the comparison to those animals fed the same diet or the same diet but added by xylanase.

### Example 14. Instant coffee production

Pure mannan is the main storage polysaccharide component of coffee endosperms and is responsible for their high viscosity, which negatively affects the technological processing of instant coffee and increases energy consumption during drying. Those effects are attributed to mannan forming hard, insoluble crystalline structures. β-mannanase, often together with other enzymes such as pectinase and cellulase, is added during the concentration step of instant coffee production to reduce viscosity in coffee extracts. Mannanase is also be employed for hydrolyzing galactomannans present in a liquid coffee extract in order to inhibit gel formation during freeze drying of instant coffee. Furthermore, due to the use of enzymatic treatment the coffee bean extracts can be concentrated by a low cost procedure such as evaporation.

The test is performed according the following flow-chart of Fig. 15 at temperatures of 10°C and a enzyme dosage of 0.15% d.s.

Mannanases of the invention are tested in mixture composed of different enzymes, such as pectinases and cellulases.

The viscosity of the coffee extract increases significantly over time under standard process conditions. However, the viscosity is significantly reduced using the enzyme mixture containing the mannanases of the invention resulting an improved downstream processing such as spray- or freeze drying.

### Example 15. Pineapple processing

In particular, mannanase is useful for pineapple mill juice extraction and clarification, as pineapple contains a large fraction of mannans, including glucomannans and galactomannans.

Mannanase helps to improve extraction of valuable fruit components, lower the viscosity of fruit juice prior to concentration, and increase filtration rate and stability of the final product.

The pineapples are crushed in a meat grinder and fill 500 g mash in a 1000 ml beaker. The enzyme is applied at 21 °C with a reaction time of 60 minutes. The mash is then pressed with a small Hafico press according to the press protocol: 0 bar 2 min - 50 bar 2 min - 100 bar 2 min - 150 bar 2 min - 200 bar 1 min - 300 bar 1 min - 400 bar 1min. The obtained juice is then centrifuged at 4500 rpm for 5 minutes and analyzed for turbidity and viscosity.

Mannanases of the invention are tested in enzyme mixtures A, B and C (Table 10).

The enzymes are first diluted with tab water before being added to the pineapple mash

**Table 10. Enzyme mixtures**

| | **Enzyme activity** | **Dosage [ppm]** | **5 ml of [% enzyme solution]** |
|---|---|---|---|
| blank | | | 5 ml H2O |
| Mixture A | Pectinase | 50 | 0.50% |
| Mixture B | Pectinase + Arabanase | 50 | 0.50% |
| Mixture C | Pectinase + Mannanase | 50 | 0.50% |

Applying mannanases of the invention leads to increased yield and lower turbidity of juice in pineapple processing.

### Example 16. Mannanase treatment of soya beans for soya milk production

For the enzymatic treatment of soya beans to get soya milk the "hot process" is commonly used. For the hot soya milk process the dried soya beans were mixed and crushed in a mixer with boiling tap water in a ratio of 1:7 (soaked beans: water). The whole soya slurry is cooled down to 50-55 °C before enzyme addition. The pH level for the soya slurry should be around pH 6.5 and can be adjusted with NaHCO₃. The mannanase enzyme is dosed at 1 kg/t of dried soya beans into the slurry and stirred for 30 min. After completion of the reaction time, the slurry is pressed using a laboratory press to obtain the final product: soya milk. In order to ensure the same pressing profile, the pressure as well as the corresponding pressing time is specified, as shown in table 11. Besides the sample for enzymatic reaction, a control sample without any enzyme is prepared, in which the enzyme solution was replaced with water.

**Table 11. Press scheme**

| | | | | |
|---|---|---|---|---|
| **pressure [bar]** | 0 | 50 | 100 | 300 |
| **time [min]** | 2 | 2 | 2 | 1 |

After pressing the soya milk is heated in a microwave until boiling to stop the enzyme reaction. Analysis of the soya milk:
- Yield in gram/time
- °Brix, which gives a direct correlation of the amount of sugar in the soy milk, is determined with a refractometer
- The turbidity of the juice is measured with a NTU-photometer, which measures the nephelometric turbidity.
- The brightness will be measured with a LAB-measurement
- Protein content is determined with a CN-Analyser (combustion method)
- Flavour

Soya milk treated with the mannanases of the invention shows a increased yield, brighter colour, increased °Brix, a lower turbidity, a higher protein content and a better taste (off flavour removal).

### Example 17. Wash performance of liquid detergent compositions according to the invention

The wash performance of liquid detergent compositions according to present invention was determined by using standardized stains obtainable from CFT (Center for Testmaterials) B.V., Vlaardingen, Netherlands ("CFT"), Eidgenössische Material- und Prüfanstalt Testmaterialien AG [Federal materials and testing agency, Testmaterials], St. Gallen, Switzerland ("EMPA") and Warwick Equest Ltd Unit 55, Consett Business Park, Consett, County Durham ("Equest").

A liquid washing agent with the following composition was used as base formulation (all values in weight percent):

**Table 12.**

| **Chemical name** | **Active substance raw material [%]** | **Active substance detergent formulation [%]** |
|---|---|---|
| Water demin. | 100 | Rest |
| Alkyl benzene sulfonic acid | 96 | 2-7 |
| Anionic surfactants | 70 | 6-10 |
| C12-C18 Fatty acid sodium salt | 30 | 1-4 |
| Nonionic surfactants | 100 | 4-7 |
| Phosphonates | 40 | 0.1-2 |
| Citric acid | 100 | 1-3 |
| NaOH | 50 | 1-4 |
| Boronic acid | 100 | 0.1-2 |
| Antifoaming agent | 100 | 0.01-1 |
| Glycerol | 100 | 1-3 |
| Enzymes | 100 | 0.1-2 |
| Preserving agent | 100 | 0.05-1 |
| Ethanol | 93 | 0.5-2 |
| Optical brightener | 90 | 0.01-1 |
| Perfume | 100 | 0.1-1 |
| Dye | 100 | 0.001-0.1 |

The pH of the detergent composition was between 8.2-8.6.

Based on this base formulation, liquid detergent compositions 1 and 2 were prepared by adding respective enzymes as indicated below:
Composition 1: Enzyme according to SEQ ID NO:12 (Man6)
Composition 2: Enzyme according to SEQ ID NO:16 (Man7)

The wash was performed as follows according to the AISE Method: 3.5 kg Clean ballast cloth, 4 SBL Cloths, Miele washing machine, 20°C and 40°C Short program.

All mannanases were added in the same amounts based on total protein content.

The dosing ratio of the liquid washing agent was 4.0 grams per liter of washing liquor. The washing procedure was performed for 60 minutes at a temperature of 20°C and 40°C, the water having a water hardness between 15.5 and 16.5° (German degrees of hardness).

The results obtained are the difference values between the remission units obtained with the detergents and the remission units obtained with the detergent containing the commercially available reference mannanase (Mannaway 4.0L, obtained from Novozymes). A positive value therefore indicates an improved wash performance of the detergent compositions comprising the mannanases of present invention compared to the same detergent composition comprising the reference mannanase. Within the washing test a large range of stains were tested.

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the enzyme combinations. A positive value therefore indicates an improved wash performance due to the enzyme combinations present in the detergent. Mannanases of the invention in detergent compositions show improved performance on a variety of mannan comprising stains.

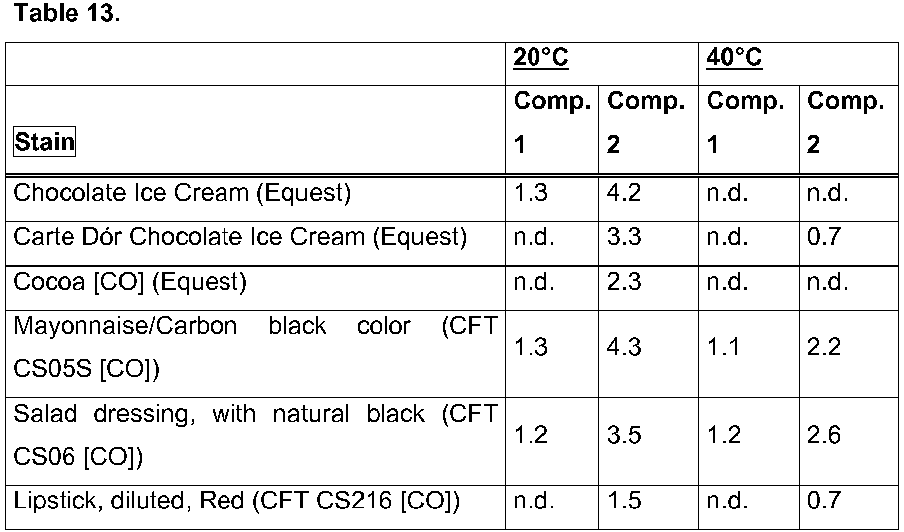

### Example 18. Wash performance of powder detergent compositions according to the invention

The wash performance of powder detergent compositions according to present invention was determined by using standardized stains obtainable from CFT (Center for Testmaterials) B.V., Vlaardingen, Netherlands ("CFT"), Eidgenössische Material- und Prüfanstalt Testmaterialien AG [Federal materials and testing agency, Testmaterials], St. Gallen, Switzerland ("EMPA") and Warwick Equest Ltd Unit 55, Consett Business Park, Consett, County Durham ("Equest").

A solid washing agent with the following composition was used as base formulation (all values in weight percent):

**Table 14.**

| Chemical name | Active substance raw material [%] | Active substance detergent formulation [%] |
|---|---|---|
| Water demin. | 100 | 1-4 |
| Alkyl benzene sulfonic acid | 97 | 9-13 |
| Nonionic surfactants | 100 | 4-7 |
| Percarbonates | 88 | 9-13 |
| TAED | 92 | 1-5 |
| Phosphonates | 60 | 0.1-3 |
| Polyacrylates | 45 | 1-4 |
| Sodium silicate | 40 | 5-10 |
| Sodium carbonate | 100 | 18-22 |
| Carboxymethylcellulose | 69 | 1-4 |
| Soil release polymer | 100 | 0.1-1 |
| Optical brightener | 70 | 0.1-1 |
| Antifoaming agent | t.q. | 0.01-1 |
| Sodium sulfate | 100 | 22-30 |
| Enzymes | 100 | 0.1-1 |
| Perfume | 100 | 0.1-1 |
| NaOH | 100 | 0.1-1 |
| Rest | - | 1-4 |

Based on this base formulation, solid detergent compositions 3 and 4 were prepared by adding respective enzymes as indicated below:
Composition 3: Enzyme according to SEQ ID NO:12 (Man6)
Composition 4: Enzyme according to SEQ ID NO:16 (Man7)

The wash was performed as follows according to the AISE Method: 3.5 kg Clean ballast cloth, 4 SBL Cloths, Miele washing machine, 20°C and 40°C Short program. All mannanases were added in the same amounts based on total protein content.

The dosing ratio of the powder washing agent was 3.8 grams per liter of washing liquor. The composition of the detergent is listed in Table 14. The washing procedure was performed for 60 minutes at a temperature of 20°C and 40°C, the water having a water hardness between 15.5 and 16.5° (German degrees of hardness).

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the reference mannanase (Mannaway 4.0L, obtained from Novozymes). A positive value therefore indicates an improved wash performance of the mannanases in the detergent. Mannanases of the invention show improved performance on several stains in Table 15. Therefore, it is evident that mannanases according to the invention show improved wash performance compared to Mannaway.

**Table 15. 20/40°C Powder**

| | **20°C** | | **40°C** | |
|---|---|---|---|---|
| **Stain** | **Comp. 3** | **Comp. 4** | **Comp. 3** | **Comp. 4** |
| Carte Dór Chocolate Ice Cream (Equest) | 1.4 | 2.8 | 2.1 | 0.5 |
| Vienetta (Equest) | 0.5 | 0.8 | 0.5 | n.d. |
| Chocolate Icecream L [CO] (Equest) | 0.9 | 0.9 | 1.1 | n.d. |
| Porridge (EMPA 163 [CO]) | n.d. | n.d. | 1.3 | 5.1 |
| Cocoa (CFT CS02 [CO]) | 1.8 | 3.1 | n.d. | n.d. |
| Mayonnaise/Carbon black color (CFT CS05S [CO]) | n.d. | 1.0 | n.d. | 2.7 |
| Salad dressing, with natural black (CFT CS06 [CO]) | 2.0 | 4.8 | 1.3 | 5.1 |
| Sebum BEY with carbon black (CFT CS32 [CO]) | 0.7 | 1.4 | 0.5 | 0.7 |
| Chocolate drink, pure (CFT CS44 [CO]) | n.d. | 1.4 | n.d. | 0.8 |

Without limiting the scope and interpretation of the patent claims, certain technical effects of one or more of the aspects or embodiments disclosed herein are listed in the following: A technical effect is degradation or modification of mannan. Another technical effect is provision of mannanase which has good storage stability.

The foregoing description has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented above, but that it can be implemented in other embodiments using equivalent means without deviating from the characteristics of the invention.

Furthermore, some of the features of the above-disclosed aspects and embodiments of this invention may be used to advantage without the corresponding use of other features. As such, the foregoing description should be considered as merely illustrative of the principles of the present invention, and not in limitation thereof. Hence, the scope of the invention is only restricted by the appended patent claims.

In an embodiment at least one component of the compositions of the invention has a different chemical, structural or physical characteristic compared to the corresponding natural component from which the at least one component is derived from. In an embodiment said characteristic is at least one of uniform size, homogeneous dispersion, different isoform, different codon degeneracy, different post-translational modification, different methylation, different tertiary or quaternary structure, different enzyme activity, different affinity, different binding activity, and different immunogenicity.

### References:

Gasteiger E., Hoogland C., Gattiker A., Duvaud S., Wilkins M.R., Appel R.D., Bairoch A.;Protein Identification and Analysis Tools on the ExPASy Server; (In) John M. Walker (ed): The Proteomics Protocols Handbook, Humana Press (2005). pp. 571-607.

Harwood, C.R (Ed.) (1990): Molecular biological methods for bacillus. Chichester: Wiley & Sons (Modern microbiological methods).

Joutsjoki VV, Torkkeli TK, and Nevalainen KMH. (1993) Transformation of Trichoderma reesei with the Hormoconis resinae glucoamylase P (gamP) gene: production of a heterologous glucoamylase by Trichoderma reesei. Curr. Genet. 24: 223-228.

Karhunen T, Mäntylä M, Nevalainen KMH, and Suominen PL. (1993) High frequency one-step gene replacement in Trichoderma reesei. I. Endoglucanase I overproduction. Mol. Gen. Genet. 241: 515-522.

Paloheimo M, Mäntylä A, Kallio J, and Suominen P. (2003) Highyield production of a bacterial xylanase in the filamentous fungus Trichoderma reesei requires a carrier polypeptide with an intact domain structure. Appl. Env. Microbiol. 69: 7073-7082.

Penttilä M, Nevalainen H, Rättö M, Salminen E, and Knowles J. (1987) A versatile transformation system for the cellulolytic filamentous fungus Trichoderma reesei. Gene 61: 155-164.

Raeder U, and Broda P. (1985) Rapid preparation of DNA from filamentous fungi. Lett. Appl. Microbiol. 1: 17-20.

Sambrook J, and Russell DW. (2001) Molecular cloning, a laboratory manual. Cold Spring Harbor Laboratory, New York, US.

Thompson JD, Higgins DG, Gibson TJ (1994) CLUSTAL W. Improving the sensitivity of progressive multiple sequence alignment through sequence weighting, position-specific gap penalties and weight matrix choice. Nucleic Acids Research 22 (22): 4673-4680. DOI: 10.1093/nar/22.22.4673.

Zhang XZ and Zhang Y-HP (2011) Simple, fast and high-efficiency transformation system or directed evolution of cellulose in Bacillus subtilis. Microbial Biotechnology 4(1): 98-105

## Claims

1. An enzyme composition comprising at least one mannanase enzyme having an amino acid sequence which has at least 80% sequence identity with SEQ ID NO: 16 (Man7) and which mannanase enzyme has a relative mannanase activity of at least 30% in the temperature range from 45°C to 65°C.

2. The enzyme composition of claim 1 further comprising:
a. at least one preservative selected from benzoic acid, sodium benzoate, hydroxybenzoate, citric acid, ascorbic acid, or a combination thereof;
b. optionally at least one polyol selected from propylene glycol, glycerol, a sugar, sugar alcohol, lactic acid, boric acid, boric acid derivative, aromatic borate ester, phenyl boronic acid derivative, peptide, or a combination thereof;
c. optionally at least one enzyme selected from proteases, amylases, cellulases, lipases, xylanases, mannanases, cutinases, esterases, phytases, DNAses, pectinases, pectinolytic enzymes, pectate lyases, carbohydrases, arabinases, galactanases, xanthanases, xyloglucanases, laccases, peroxidases and oxidases with or without a mediator, or a combination thereof; and
d. optionally at least one filler selected from maltodextrin, flour, sodium chloride, sulfate, sodium sulfate, or a combination thereof.

3. The enzyme composition of claims 1-2 in the form of a liquid composition or a solid composition such as solution, dispersion, paste, powder, granule, granulate, coated granulate, tablet, cake, crystal, crystal slurry, gel, or pellet.

4. A recombinant host cell comprising genetic elements that allow producing at least one recombinant polypeptide having mannanase activity, and a relative mannanase activity of at least 30% in the temperature range from 45°C to 65°C and
at least 80% sequence identity with the amino acid sequence of SEQ ID NO: 16, and wherein the host cell is selected from the group consisting of:
fungal cells,
filamentous fungal cells from Division *Ascomycota,* Subdivision *Pezizomycotina;* preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola;*
more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium;*
more preferably from the group consisting of *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G.* zeaea, *Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,*
bacterial cells, preferably gram positive Bacilli such as *B*. *subtilis, B. licheniformis, B. megaterium, B. amyloliquefaciens, B. pumilus,* gram negative bacteria such as *Escherichia coli,* actinomycetales such as *Streptomyces* sp., and
yeasts, such as *Saccharomyces cerevisiae, Pichia pastoris, Yarrowia lipolytica,* most preferably *Trichoderma reesei* or *Bacillus.*

5. The recombinant host cell of claim 4, wherein the recombinant polypeptide is a fusion protein which, in addition to having the amino acid sequence having mannanase activity, comprises at least one of:
an amino acid sequence providing a secretory signal sequence, such as *Bacillus amyloliquefaciens* xylanase signal peptide;
an amino acid sequence which facilitates purification, such as an affinity tag, His-tag;
an amino acid sequence which enhances production, such as an amino acid sequence which is a carrier, such as CBM;
an amino acid sequence having an enzyme activity; and
an amino acid sequence providing for the fusion protein with binding affinity,
such as a carbohydrate binding moiety.

6. A recombinant polypeptide having mannanase activity and obtainable by using the host cell of claims 4-5 and having a relative mannanase activity of at least 30% in the temperature range from 45°C to 65°C.

7. A method for producing mannanase having a relative mannanase activity of at least 30% in the temperature range from 45°C to 65°C comprising:
a. cultivating a recombinant host cell of claims 4-5, wherein
i. the genetic elements comprise at least one control sequence which controls the production of the recombinant polypeptide in the recombinant host cell under conditions that allow production of the polypeptide;
ii. the genetic elements optionally comprise at least one sequence encoding a signal sequence for transporting the polypeptide outside the host cell; and
iii. cultivating is carried out in conditions allowing production of the polypeptide; and
b. recovering the polypeptide.

8. A method for degrading or modifying mannan containing material comprising treating said mannan containing material with an effective amount of the enzyme composition of claims 1-3 or the recombinant polypeptide of claim 6.

9. The method of claim 8 wherein the mannan containing material is plant based material, textile, waste water, sewage, oil, or a combination thereof.

10. An animal feed comprising the enzyme composition of claims 1-3 or the enzyme obtainable from the recombinant host cell of claims 4-5, and at least one protein source of plant origin or a mannan containing product or by-product, and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

11. A feed supplement comprising the enzyme composition of claims 1-3 or the enzyme obtainable from the host cell of claims 4-5; and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate or a combination thereof.

12. Use of the animal feed of claim 10 or the feed supplement of claim 11 in:
a. feeding animals, preferably monogastric animals or ruminants; and/or
b. improving weight gain of animals.

13. A use of the enzyme composition of claims 1-3 or the enzyme obtainable from the recombinant host cell of claims 4-5 in a detergent.

14. The use of claim 13 wherein the detergent is a liquid detergent or a dry detergent preferably in a form of a powder, bar, tablet, pouch, paste, gel, liquid, granule or granulate.

15. A use of the enzyme composition of claims 1-3 or the enzyme obtainable from the recombinant host cell of claims 4-5 in oil drilling.

16. A use of the enzyme composition of claims 1-3 or the enzyme obtainable from the recombinant host cell of claims 4-5 in processing coffee extract, fruit juice, pineapple juice, or soya milk.

## Patentansprüche

1. Enzymzusammensetzung, umfassend wenigstens ein Mannanaseenzym mit einer Aminosäuresequenz, die wenigstens 80 % Sequenzidentität mit SEQ ID NO: 16 (Man7) aufweist und das Mannanaseenzym eine relative Mannanaseaktivität von wenigstens 30 % in dem Temperaturbereich von 45 °C bis 65 °C aufweist.

2. Enzymzusammensetzung nach Anspruch 1, ferner umfassend:
a. wenigstens ein Konservierungsmittel, ausgewählt aus Benzoesäure, Natriumbenzoat, Hydroxybenzoat, Zitronensäure, Ascorbinsäure oder einer Kombination davon;
b. gegebenenfalls wenigstens ein Polyol, ausgewählt aus Propylenglykol, Glycerin, einem Zucker, Zuckeralkohol, Milchsäure, Borsäure, Borsäurederivat, aromatischem Boratester, Phenylboronsäurederivat, Peptid oder einer Kombination davon;
c. gegebenenfalls wenigstens ein Enzym ausgewählt aus Proteasen, Amylasen, Cellulasen, Lipasen, Xylanasen, Mannanasen, Cutinasen, Esterasen, Phytasen, DNasen, Pektinasen, pektinolytischen Enzymen, Pektatlyasen, Kohlenhydraten, Arabinasen, Galactanasen, Xanthanasen, Xyloglucanasen, Laccasen, Peroxidasen und Oxidasen mit oder ohne Mediator oder einer Kombination davon; und
d. gegebenenfalls wenigstens einen Füllstoff, ausgewählt aus Maltodextrin, Mehl, Natriumchlorid, Sulfat, Natriumsulfat oder einer Kombination davon.

3. Enzymzusammensetzung nach Ansprüchen 1-2 in der Form einer liquiden Zusammensetzung oder einer festen Zusammensetzung wie z.B. Lösung, Dispersion, Paste, Pulver, Körnchen, Granulat, beschichtetes Granulat, Tablette, Kuchen, Kristall, Kristallaufschlämmung, Gel oder Pellet.

4. Rekombinante Wirtszelle, umfassend genetische Elemente, die Herstellen wenigstens eines rekombinanten Polypeptids mit Mannanaseaktivität und einer relativen Mannanaseaktivität von wenigstens 30 % in dem Temperaturbereich von 45 °C bis 65 °C und wenigstens 80 % Sequenzidentität mit der Aminosäuresequenz von SEQ ID NO: 16 ermöglichen,
und wobei die Wirtszelle ausgewählt ist aus der Gruppe bestehend aus:
Pilzzellen,
filamentösen Pilzzellen aus Abteilung Ascomycota, Unterabteilung Pezizomycotina; bevorzugt aus der Gruppe bestehend aus Mitgliedern der Klasse Sordariomycetes, Unterklasse Hypocreomycetidae, Ordnungen Hypocreales und Microascales und Aspergillus, Chrysosporium, Myceliophthora und Humicola;
besonders bevorzugt aus der Gruppe bestehend aus Familien Hypocreacea, Nectriaceae, Clavicipitaceae, Microascaceae und Gattungen Trichoderma (Anamorph von Hypocrea), Fusarium, Gibberella, Nectria, Stachybotrys, Claviceps, Metarhizium, Villosiclava, Ophiocordyceps, Cephalosporium und Scedosporium;
besonders bevorzugt aus der Gruppe bestehend aus Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum und Scedosporium apiospermum und Aspergillus niger, Aspergillus awamori, Aspergillus oryzae, Chrysosporium lucknowense, Myceliophthora thermophila, Humicola insolens und Humicola grisea.
Bakterienzellen, bevorzugt grampositive Bazillen wie z.B. B. subtilis, B. licheniformis, B. megaterium, B. amyloliquefaciens, B. pumilus, gramnegative Bakterien wie z.B. Escherichia coli, Actinomycetales wie z.B. Streptomyces sp. und
Hefen wie z.B. Saccharomyces cerevisiae, Pichia pastoris, Yarrowia lipolytica, am bevorzugtesten Trichoderma reesei oder Bacillus.

5. Rekombinante Wirtszelle nach Anspruch 4, wobei das rekombinante Polypeptid ein Fusionsprotein ist, das zusätzlich zum Aufweisen der Aminosäuresequenz mit Mannanaseaktivität umfasst wenigstens eines von:
einer Aminosäuresequenz, die eine sekretorische Signalsequenz bereitstellt, wie z.B. Bacillus amyloliquefaciens Xylanase-Signalpeptid;
einer Aminosäuresequenz, die Reinigung erleichtert, wie z.B. ein Affinitäts-Tag, His-Tag;
einer Aminosäuresequenz, die die Produktion steigert, wie z.B. eine Aminosäuresequenz, die ein Träger ist, wie z.B. CBM;
einer Aminosäuresequenz mit einer Enzymaktivität; und
einer Aminosäuresequenz, bereitstellend für das Fusionsprotein mit Bindungsaffinität, wie z.B. ein Kohlenhydratbindungsteil.

6. Rekombinantes Polypeptid mit Mannanaseaktivität und erhältlich durch Verwenden der Wirtszelle nach Ansprüchen 4-5 und mit einer relativen Mannanaseaktivität von wenigstens 30 % in dem Temperaturbereich von 45 °C bis 65 °C.

7. Verfahren zum Herstellen von Mannanase mit einer relativen Mannanaseaktivität von wenigstens 30 % in dem Temperaturbereich von 45 °C bis 65 °C, umfassend:
a. Kultivieren einer rekombinanten Wirtszelle nach Ansprüchen 4-5, wobei
i. die genetischen Elemente wenigstens eine Steuersequenz umfassen, die die Herstellung des rekombinanten Polypeptids in der rekombinanten Wirtszelle unter Bedingungen steuert, die Herstellung des Polypeptids ermöglichen;
ii. die genetischen Elemente gegebenenfalls wenigstens eine Sequenz umfassen, die eine Signalsequenz zum Transportieren des Polypeptids außerhalb der Wirtszelle codiert; und
iii. Kultivieren unter Bedingungen durchgeführt wird, die Herstellung des Polypeptids ermöglichen; und
b. Gewinnen des Polypeptids.

8. Verfahren zum Abbauen oder Modifizieren von Mannan enthaltendem Material, umfassend Behandeln des Mannan enthaltenden Materials mit einer wirksamen Menge der Enzymzusammensetzung nach Ansprüchen 1-3 oder des rekombinanten Polypeptids nach Anspruch 6.

9. Verfahren nach Anspruch 8, wobei das Mannan enthaltende Material auf Pflanzen basierendes Material, Textil, Abwasser, Schmutzwasser, Öl oder eine Kombination davon ist.

10. Tierfutter, umfassend die Enzymzusammensetzung nach Ansprüchen 1-3 oder das Enzym, das von der rekombinanten Wirtszelle nach Ansprüchen 4-5 erhältlich ist, und wenigstens eine Proteinquelle pflanzlichen Ursprungs oder ein Mannan enthaltendes Produkt oder Nebenprodukt, und
a. optional wenigstens ein Enzym ausgewählt aus Protease, Amylase, Phytase, Xylanase, Endoglucanase, Beta-Glucanase oder einer Kombination davon; und
b. optional wenigstens einen Füllstoff ausgewählt aus Maltodextrin, Mehl, Salz, Natriumchlorid, Sulfat, Natriumsulfat oder einer Kombination davon.

11. Futterzusatz, umfassend die Enzymzusammensetzung nach Ansprüchen 1-3 oder das Enzym, das von der Wirtszelle nach Ansprüchen 4-5 erhältlich ist; und
a. optional wenigstens ein Enzym ausgewählt aus Protease, Amylase, Phytase, Xylanase, Endoglucanase, Beta-Glucanase oder einer Kombination davon; und
b. optional wenigstens einen Füllstoff ausgewählt aus Maltodextrin, Mehl, Salz, Natriumchlorid, Sulfat, Natriumsulfat oder einer Kombination davon.

12. Verwendung des Tierfutters nach Anspruch 10 oder des Futterzusatzes nach Anspruch 11 zum:
a. Füttern von Tieren, bevorzugt monogastrischen Tieren oder Wiederkäuern; und / oder
b. Verbesserung von Gewichtszunahme von Tieren.

13. Verwendung der Enzymzusammensetzung nach Ansprüchen 1-3 oder des Enzyms, das von der rekombinanten Wirtszelle nach Ansprüchen 4-5 in einem Detergens erhältlich ist.

14. Verwendung nach Anspruch 13, wobei das Detergens ein liquides Detergens oder ein trockenes Detergens ist, bevorzugt in einer Form eines Pulvers, eines Riegels, einer Tablette, einer Tasche, einer Paste, eines Gels, eines Liquids, eines Körnchens oder eines Granulats.

15. Verwendung der Enzymzusammensetzung nach Ansprüchen 1-3 oder des Enzyms, das von der rekombinanten Wirtszelle nach Ansprüchen 4-5 beim Ölbohren erhältlich ist.

16. Verwendung der Enzymzusammensetzung nach Ansprüchen 1-3 oder des Enzyms, das von der rekombinanten Wirtszelle nach Ansprüchen 4-5 bei der Verarbeitung von Kaffeeextrakt, Fruchtsaft, Ananassaft oder Sojamilch erhältlich ist.

## Revendications

1. Une composition enzymatique comprenant au moins une enzyme mannanase ayant une séquence d'acides aminés qui a au moins 80% d'identité de séquence avec SEQ ID NO : 16 (Man7) et laquelle enzyme mannanase a une activité mannanase relative d'au moins 30% dans la gamme de température allant de 45°C à 65°C.

2. La composition enzymatique selon la revendication 1, comprenant en outre :
a. au moins un conservateur choisi parmi l'acide benzoïque, le benzoate de sodium, l'hydroxybenzoate, l'acide citrique, l'acide ascorbique ou une combinaison de ceux-ci ;
b. optionnellement au moins un polyol choisi parmi le propylène glycol, le glycérol, un sucre, un sucre-alcool, l'acide lactique, l'acide borique, un dérivé d'acide borique, un ester borate aromatique, un dérivé d'acide phényl boronique, un peptide ou une combinaison de ceux-ci ;
c. optionnellement, au moins une enzyme choisie parmi les protéases, les amylases, les cellulases, les lipases, les xylanases, les mannanases, les cutinases, les estérases, les phytases, les DNAses, les pectinases, les enzymes pectinolytiques, les pectates lyases, les carbohydrases, les arabinases, les galactanases, les xanthanases, les xyloglucanases, les laccanases, les peroxydases et les oxydases avec ou sans médiateur, ou une combinaison de ceux-ci ; et
d. optionnellement, au moins une charge choisie parmi la maltodextrine, la farine, le chlorure de sodium, le sulfate, le sulfate de sodium ou une combinaison de ceux-ci.

3. La composition enzymatique selon les revendications 1 à 2 sous la forme d'une composition liquide ou d'une composition solide telle qu'une solution, une dispersion, une pâte, une poudre, un granule, un granulé, un granulé enrobé, un comprimé, un gâteau, un cristal, une suspension cristalline, un gel ou une pastille.

4. Une cellule hôte recombinante comprenant des éléments génétiques qui permettent de produire au moins un polypeptide recombinant ayant une activité mannanase, et une activité mannanase relative d'au moins 30% dans la gamme de températures allant de 45°C à 65°C, et
au moins 80% d'identité de séquence avec la séquence d'acides aminés de SEQ ID NO : 16,
la cellule hôte étant choisie dans le groupe constitué par :
des cellules fongiques,
des cellules fongiques filamenteuses de la division *Ascomycota,* subdivision *Pezizomycotina* ; de préférence dans le groupe constitué des membres de la classe *Sordariomycetes,* de la sous-classe *Hypocreomycetidae,* des ordres *Hypocreales* et *Microascales* et *Aspergillus, Chrysosporium,*
*Myceliophthora* et *Humicola ;*
de façon encore préférée, dans le groupe constitué des familles *Hypocreacea, Nectriaceae, Clavicipitaceae, Microascaceae* et du genre *Trichoderma* (anamorphe de *Hypocrea), Fusarium, Gibberella, Nectria, Stachybotrys, Claviceps, Metarhizium, Villosiclava, Ophiocordyceps, Cephalosporium* et *Scedosporium ;*
de façon encore préférée, dans le groupe constitué de *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis,* G *zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* et *Scedosporium apiospermum,* et *Aspergillus niger, Aspergillus awamori, Aspergillus oryzae, Chrysosporium lucknowense, Myceliophthora thermophila, Humicola insolens,* et *Humicola grisea,*
des cellules bactériennes, de préférence des bacilles à Gram positif tels que *B. subtilis, B. licheniformis, B. megaterium, B. amyloliquefaciens, B. pumilus,* des bactéries à Gram négatif telles que *Escherichia coli,* des actinomycétales telles que *Streptomyces* sp., et
des levures, telles que *Saccharomyces cerevisiae, Pichia pastoris, Yarrowia lipolytica,*
de façon encore préférée *Trichoderma reesei* ou *Bacillus.*

5. La cellule hôte recombinante selon la revendication 4, dans laquelle le polypeptide recombinant est une protéine de fusion qui, en plus d'avoir la séquence d'acides aminés ayant une activité mannanase, comprend au moins l'un parmi :
une séquence d'acides aminés fournissant une séquence signal de sécrétion, telle que le peptide signal de xylanase de *Bacillus amyloliquefaciens* ;
une séquence d'acides aminés qui facilite la purification, telle qu'un marqueur d'affinité, un marqueur His ;
une séquence d'acides aminés qui améliore la production, telle qu'une séquence d'acides aminés qui est un support, telle que CBM ;
une séquence d'acides aminés ayant une activité enzymatique ; et
une séquence d'acides aminés fournissant la protéine de fusion avec une affinité de liaison, telle qu'une fraction de liaison aux glucides.

6. Un polypeptide recombinant ayant une activité mannanase et apte à être obtenu en utilisant la cellule hôte selon les revendications 4 à 5 et ayant une activité mannanase relative d'au moins 30% dans la gamme de températures allant de 45°C à 65°C.

7. Un procédé de production de mannanase ayant une activité mannanase relative d'au moins 30% dans la gamme de températures allant de 45°C à 65°C, comprenant :
a. le fait de cultiver une cellule hôte recombinante selon les revendications 4 à 5,
i. les éléments génétiques comprennent au moins une séquence de commande qui commande la production du polypeptide recombinant dans la cellule hôte recombinante dans des conditions permettant la production du polypeptide ;
ii. les éléments génétiques comprennent optionnellement au moins une séquence codant pour une séquence signal afin de transporter le polypeptide hors de la cellule hôte ; et
iii. la culture est effectuée dans des conditions permettant la production du polypeptide ; et
b. le fait de récupérer le polypeptide.

8. Un procédé pour dégrader ou modifier un matériau contenant du mannane comprenant le fait de traiter ledit matériau contenant du mannane avec une quantité efficace de la composition enzymatique selon les revendications 1 à 3 ou du polypeptide recombinant selon la revendication 6.

9. Le procédé selon la revendication 8, dans lequel le matériau contenant du mannane est un matériau à base de plante, du textile, des eaux usées, des eaux d'égout, de l'huile ou une combinaison de ceux-ci.

10. Aliment pour animaux comprenant la composition enzymatique selon les revendications 1 à 3 ou l'enzyme apte à être obtenue à partir de la cellule hôte recombinante selon les revendications 4 à 5, et au moins une source de protéines d'origine végétale ou un produit ou sous-produit contenant du mannane, et
a. optionnellement, au moins une enzyme choisie parmi la protéase, l'amylase, la phytase, la xylanase, l'endoglucanase, la bêta-glucanase ou une combinaison de celles-ci ; et
b. optionnellement, au moins une charge choisie parmi la maltodextrine, la farine, le sel, le chlorure de sodium, le sulfate, le sulfate de sodium ou une combinaison de ceux-ci.

11. Un complément alimentaire comprenant la composition enzymatique selon les revendications 1 à 3 ou l'enzyme apte à être obtenue à partir de la cellule hôte selon les revendications 4 à 5 ; et
a. optionnellement, au moins une enzyme choisie parmi la protéase, l'amylase, la phytase, la xylanase, l'endoglucanase, la bêta-glucanase, ou une combinaison de celles-ci ; et
b. optionnellement, au moins une charge choisie parmi la maltodextrine, la farine, le sel, le chlorure de sodium, le sulfate, le sulfate de sodium ou une combinaison de ceux-ci.

12. Utilisation de l'aliment pour animaux selon la revendication 10 ou du complément alimentaire selon la revendication 11 pour :
a. nourrir des animaux, de préférence des animaux monogastriques ou des ruminants ; et / ou
b. améliorer la prise de poids des animaux.

13. Une utilisation de la composition enzymatique selon les revendications 1 à 3 ou de l'enzyme apte à être obtenue à partir de la cellule hôte recombinante selon les revendications 4 à 5 dans un détergent.

14. L'utilisation selon la revendication 13, dans laquelle le détergent est un détergent liquide ou un détergent sec de préférence sous la forme d'une poudre, d'un pain, d'un comprimé, d'un sachet, d'une pâte, d'un gel, d'un liquide, d'un granule ou d'un granulé.

15. Une utilisation de la composition enzymatique selon les revendications 1 à 3 ou de l'enzyme apte à être obtenue à partir de la cellule hôte recombinante selon les revendications 4 à 5 dans le forage pétrolier.

16. Une utilisation de la composition enzymatique selon les revendications 1 à 3 ou de l'enzyme apte à être obtenue à partir de la cellule hôte recombinante selon les revendications 4 à 5 dans le traitement d'extrait de café, de jus de fruit, de jus d'ananas ou de lait de soja.
